# EUROPEAN PATENT APPLICATION

(11) **EP 4 722 202 A1**
(43) Date of publication of application: **08.04.2026**
(21) Application number: 25203700.7
(22) Date of filing: 22.09.2025
(51) Int. Cl.: C07D 307/91, C07C 211/54, C07C 211/58, C07C 211/60, C07D 307/92, C07D 333/74, C07D 333/76, H10K 50/15, H10K 85/60

(54) **LIGHT-EMITTING ELEMENT, MONOAMINE COMPOUND FOR THE LIGHT-EMITTING ELEMENT, AND ELECTRONIC APPARATUS INCLUDING THE LIGHT-EMITTING ELEMENT**

(30) Priority: 03.10.2024 JP 2024174032
(71) Applicant: Samsung Display Co., Ltd., Yongin-si, Gyeonggi-Do (KR)
(72) Inventor: SAKAMOTO, Naoya, Kanagawa, 220-0011 (JP)
(74) Representative: Marks & Clerk LLP

(57) **Abstract**

The light-emitting element includes a first electrode, a hole transport region on the first electrode, an emission layer on the hole transport region, an electron transport region on the emission layer, and a second electrode on the electron transport region. The hole transport region includes a monoamine compound represented by Formula 1.

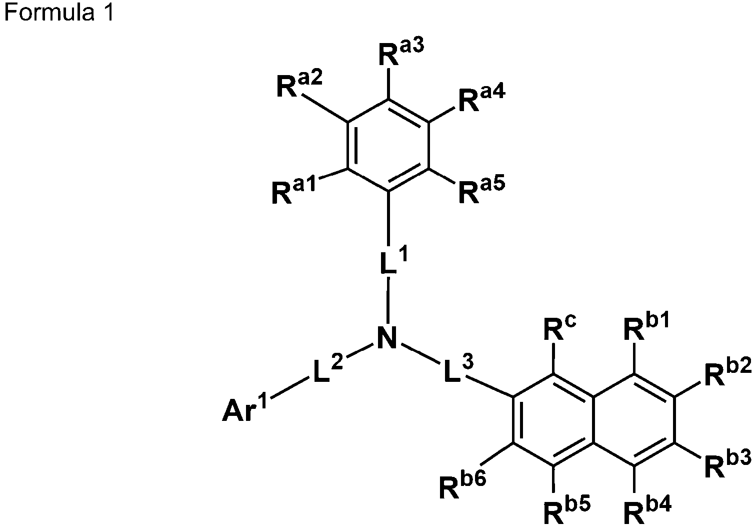

## Description

### BACKGROUND

### 1. Field

One or more embodiments of the present disclosure relate to a light-emitting element, a monoamine compound used in a light-emitting element, and an electronic apparatus including a light-emitting element.

### 2. Description of the Related Art

Various electronic apparatuses include image display devices. Among such devices, organic electroluminescence (EL) display devices have been widely adopted and are under active development. Organic electroluminescence display devices are self-luminous and include light-emitting elements in which holes and electrons, injected separately from a first electrode and a second electrode, recombine in an emission layer to emit light (e.g., to display images).

For application of light-emitting elements to display devices, there is a continuous demand or desire for higher luminous efficiency and/or longer operational lifetime. Accordingly, the development of materials for light-emitting elements that may stably achieve these desired characteristics remains an active area of research and development.

### SUMMARY

One or more aspects of embodiments of the present disclosure are directed toward a light-emitting element that has improved light-emitting efficiency and lifespan, and an electronic apparatus including the same.

One or more aspects of embodiments of the present disclosure are directed toward a monoamine compound that is a material for a light-emitting element, which improves light-emitting efficiency and lifespan of the light-emitting element.

Additional aspects will be set forth in part in the description which follows and, in part, will be apparent from the description, or may be learned by practice of the presented embodiments.

According to one or more embodiments of the present disclosure, a light-emitting element includes a first electrode, a hole transport region on (e.g., arranged on) the first electrode and including a monoamine compound represented by Formula 1, an emission layer on (e.g., arranged on) the hole transport region, an electron transport region on (e.g., arranged on) the emission layer; and a second electrode on (e.g., arranged on) the electron transport region.

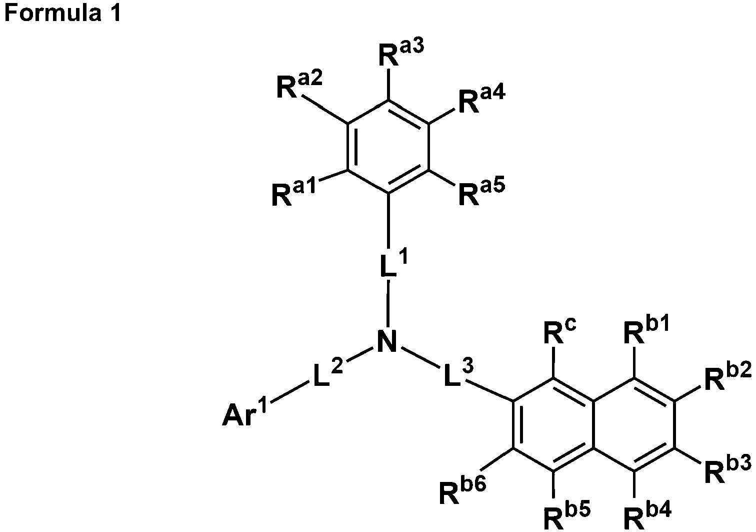

In Formula 1, L¹ to L³ are each independently a direct linkage, a substituted or unsubstituted arylene group having 6 to 30 ring-forming carbon atoms, or a substituted or unsubstituted heteroarylene group having 5 to 30 ring-forming carbon atoms, a hydrogen or a deuterium is bonded to each atom at an ortho position with respect to N in L¹ to L³; Ar¹ is a substituted or unsubstituted aryl group having 10 to 30 ring-forming carbon atoms or a substituted or unsubstituted heteroaryl group having 5 to 30 ring-forming carbon atoms, a case where Ar¹ is an aryl group in which a plurality of phenyl groups is connected by a direct linkage is excluded; at least two selected from among R^{a1} to R^{a5} are each a substituted or unsubstituted phenyl group, and the rest are each independently hydrogen, deuterium, a halogen, a substituted or unsubstituted alkyl group having 1 to 15 carbon atoms, a substituted or unsubstituted aryl group having 6 to 30 ring-forming carbon atoms, or a substituted or unsubstituted heteroaryl group having 5 to 30 ring-forming carbon atoms, a case where R^{a1} and R^{a5} are each a phenyl group at the same time is excluded; R^{c} is hydrogen, deuterium, or fluorine; at least one selected from among R^{b1} to R^{b6} is a substituted or unsubstituted phenyl group, and the rest are each independently hydrogen, deuterium, a halogen, a substituted or unsubstituted alkyl group having 1 to 15 carbon atoms, a substituted or unsubstituted aryl group having 6 to 30 ring-forming carbon atoms, or a substituted or unsubstituted heteroaryl group having 5 to 30 ring-forming carbon atoms; if (e.g., when) R^{a2} and R^{a3} are phenyl groups or R^{a3} and R^{a4} are phenyl groups, then L¹ is a direct linkage; the monoamine compound includes no nitrogen-containing heterocyclic group (e.g., excludes a nitrogen-containing heterocyclic group); the monoamine compound includes no cyclic group including a carbon atom which includes *sp³* hybrid orbitals (e.g., excludes a cyclic group including a carbon atom which includes *sp³* hybrid orbitals); and the monoamine compound may include a chemical structure of Formula 1 in which any hydrogen in the chemical structure is substituted with deuterium, in other words, any hydrogen of the monoamine compound is optionally substituted with deuterium.

In one or more embodiments, the monoamine represented by Formula 1 may be represented by any one selected from among Formula 1-A1 to Formula 1-A4.

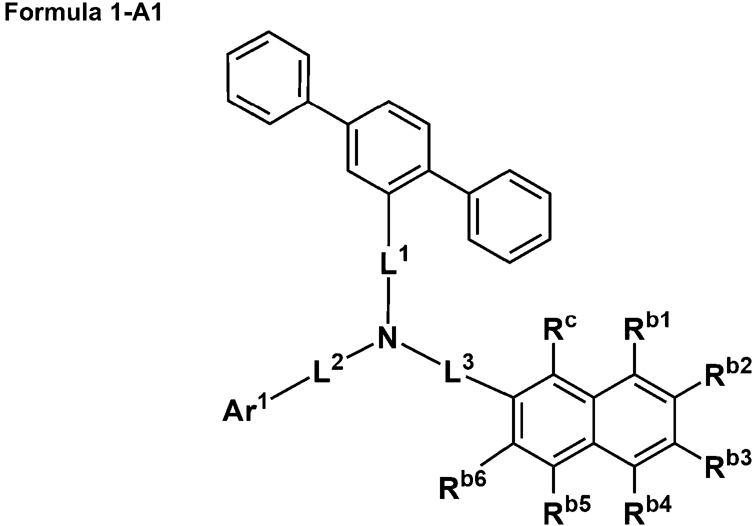

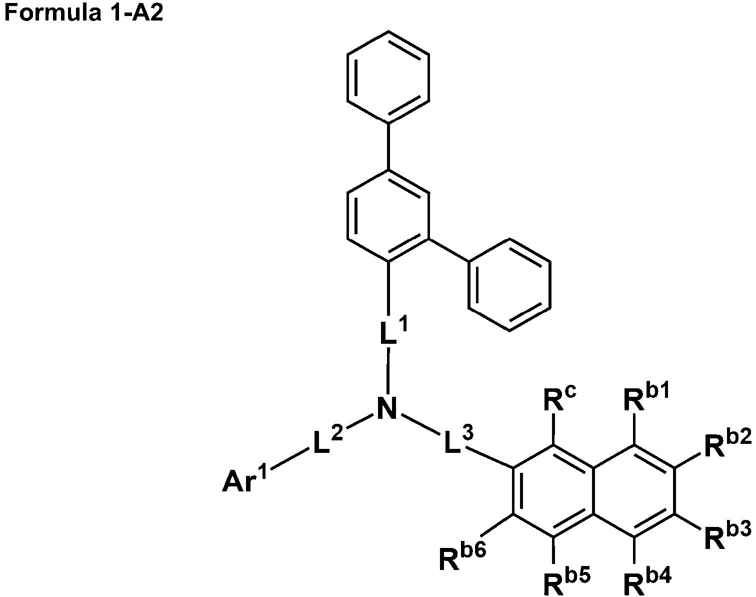

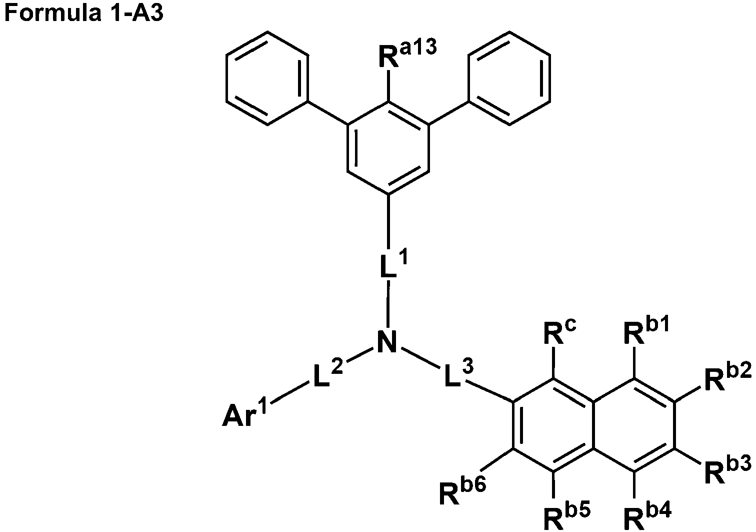

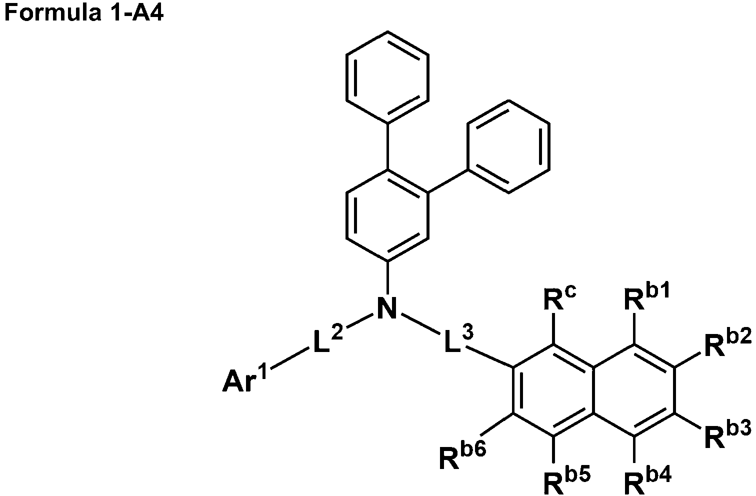

In Formula 1-A3, R^{a13} is hydrogen or fluorine, and in Formula 1-A1 to Formula 1-A4, L¹ to L³, Ar¹, R^{c}, and R^{b1} to R^{b6} are each independently the same as defined in Formula 1.

In one or more embodiments, in Formula 1-A1 to Formula 1-A4, at least one selected from among L¹ to L³ may be a substituted or unsubstituted phenylene group, and the rest may each be a direct linkage.

In one or more embodiments, the monoamine compound represented by Formula 1 may be represented by any one selected from among Formula 1-B1 to Formula 1-B6.

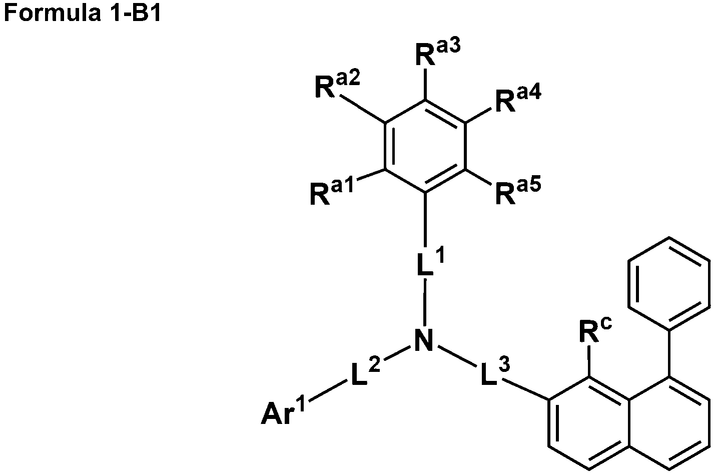

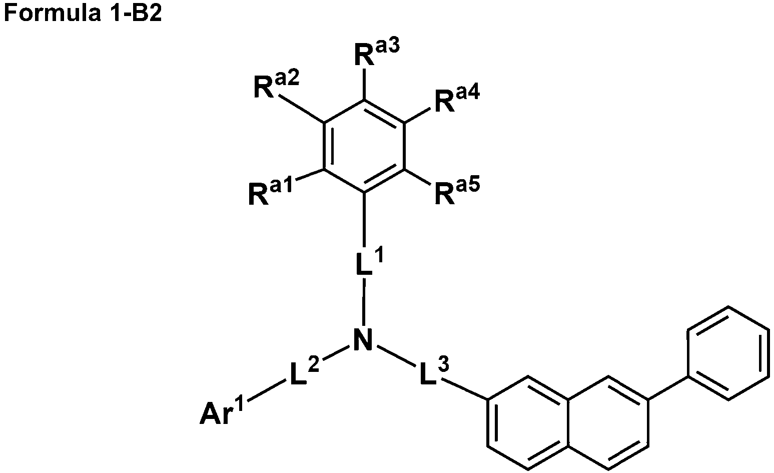

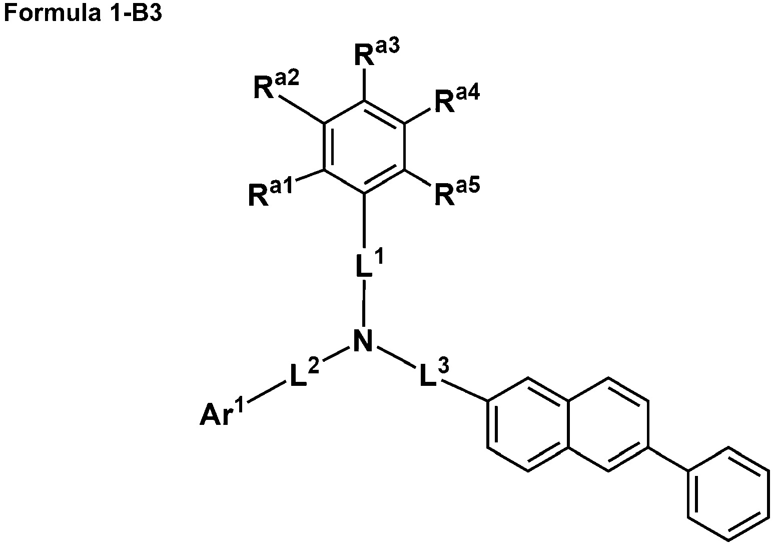

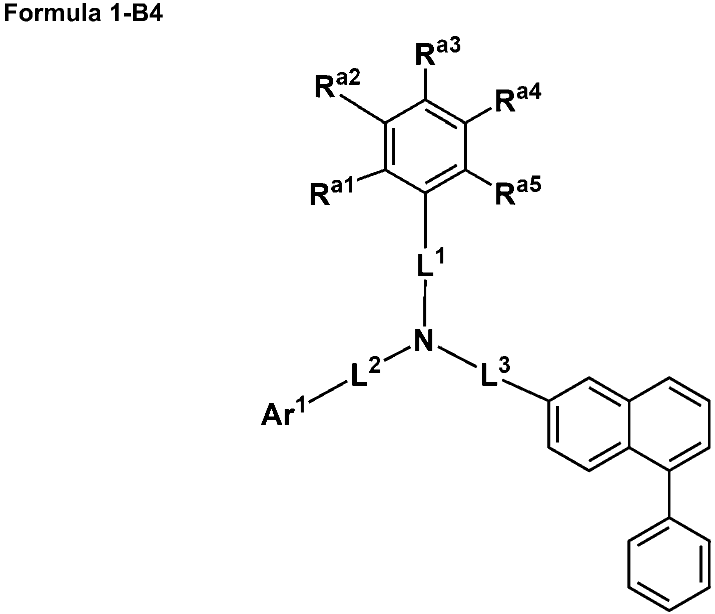

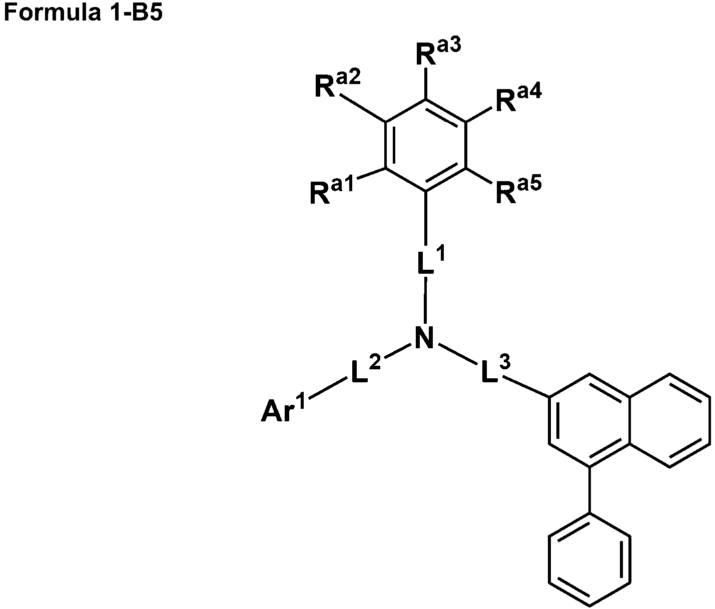

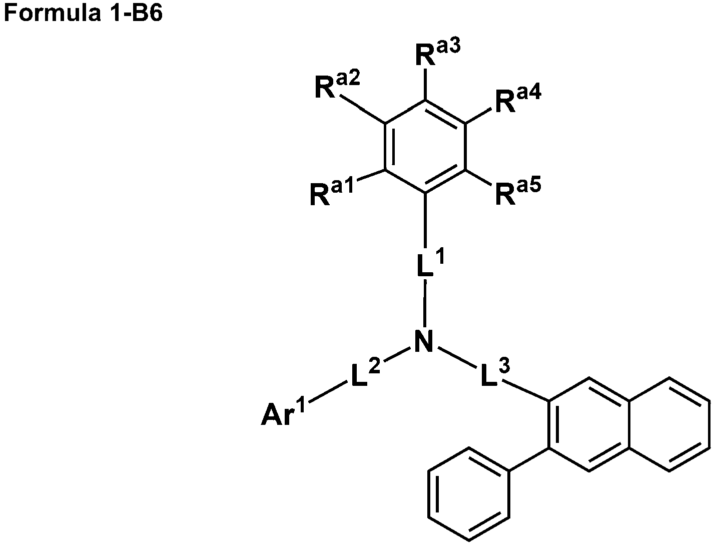

In Formula 1-B1 to Formula 1-B6, L¹ to L³, Ar¹, and R^{a1} to R^{a5}, R^{c} are each independently the same as defined in Formula 1.

In one or more embodiments, in Formula 1, Ar¹ may be represented by any one selected from among AR-1 to AR-20.

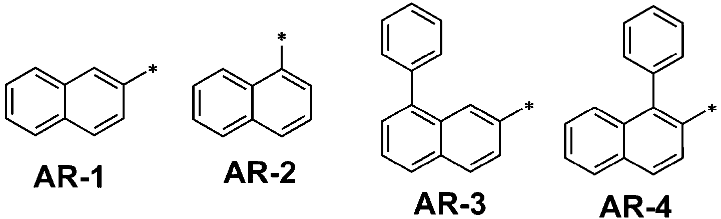

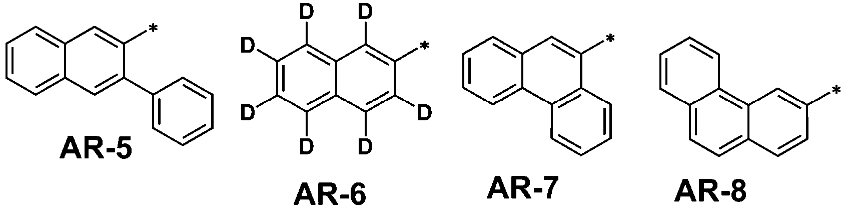

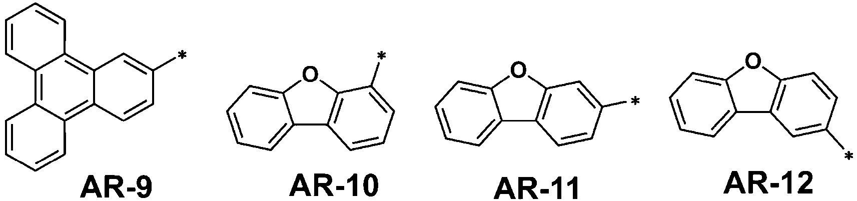

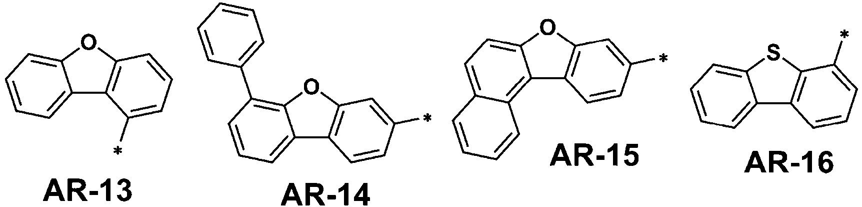

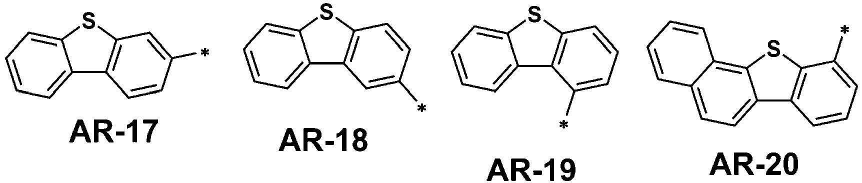

In AR-6, D is deuterium.

In one or more embodiments, in Formula 1, Ar¹ may be a substituted or unsubstituted naphthalene group, a substituted or unsubstituted phenanthrene group, a substituted or unsubstituted dibenzofuran group, a substituted or unsubstituted dibenzothiophene group, a substituted or unsubstituted benzonaphthofuran group, or a substituted or unsubstituted benzonaphthothiophene group.

In one or more embodiments, in Formula 1, L¹ to L³ may each independently be a direct linkage or represented by L-1 or L-2.

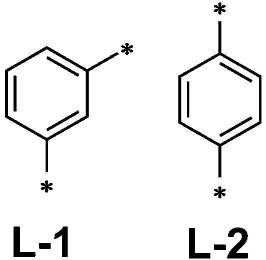

In one or more embodiments, the hole transport region may include a hole injection layer, a hole transport layer on (e.g., arranged on) the hole injection layer, and/or an electron blocking layer on (e.g., arranged on) the hole transport layer, and at least one of the hole injection layer, the hole transport layer, or the electron blocking layer may include the monoamine compound.

In one or more embodiments, the emission layer may include a compound represented by Formula E-1:

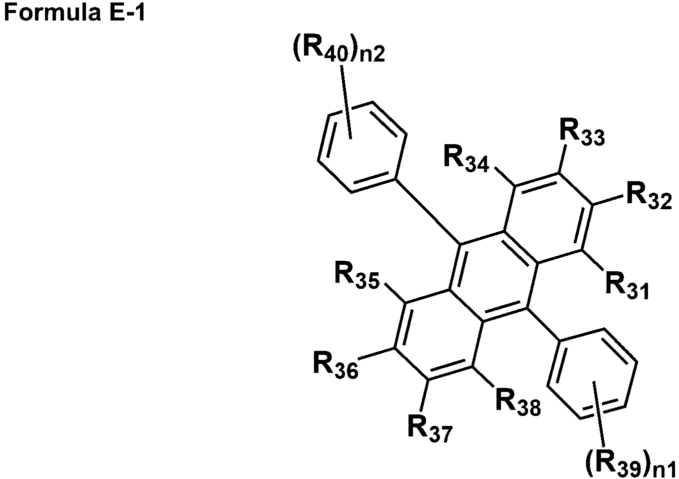

In Formula E-1, n1 and n2 are each independently an integer of 0 to 5, and R₃₁ to R₄₀ are each independently hydrogen, deuterium, a halogen, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms, and/or bonded to an adjacent group to form a ring.

In one or more embodiments of the present disclosure, provided is a monoamine compound represented by Formula 1.

In one or more embodiments of the present disclosure, an electronic apparatus includes a display device including a base layer, a circuit layer on (e.g., arranged on) the base layer, and a display element layer on (e.g., arranged on) the circuit layer and including a light-emitting element, wherein the light-emitting element includes a first electrode, a hole transport region on (e.g., arranged on) the first electrode and including a monoamine compound represented by Formula 1, an emission layer on (e.g., arranged on) the hole transport region, an electron transport region on (e.g., arranged on) the emission layer, and a second electrode on (e.g., arranged on) the electron transport region.

In one or more embodiments, the display device included in the electronic apparatus may further include at least one of an light control layer and a color filter layer, wherein the light control layer may include a quantum dot, and the color filter layer may include a pigment or a dye.

In one or more embodiments, the electronic apparatus may include any one selected from among a television, a monitor, an outdoor billboard, a personal computer, a laptop computer, a personal digital assistant, a vehicle display device, a game console, a portable electronic device, and a camera.

At least some of the above and other features of the invention are set out in the claims.

For example, the present disclosure, through various embodiments, provides a monoamine compound and its application in light-emitting elements, which exhibit enhanced luminous efficiency and operational lifespan. These light-emitting elements are suitable for utilization in organic electroluminescence display devices. The monoamine compound, represented by Formula 1, is incorporated into the hole transport region of the light-emitting element, contributing to improved charge transport, material stability, and emission characteristics. Furthermore, the disclosure extends to electronic apparatuses incorporating such display devices, which may include televisions, monitors, portable electronic devices, and/or vehicular displays, among others. The disclosed technology thus offers advancements in the performance and reliability of next-generation display technologies.

### BRIEF DESCRIPTION OF DRAWINGS

The accompanying drawings are included to provide a further understanding of the present disclosure, and are incorporated in and constitute a part of this disclosure. The drawings illustrate embodiments of the present disclosure and, together with the description, serve to explain principles of the disclosure. Above and/or other aspects of the disclosure should become apparent and appreciated from the following description of embodiments taken in conjunction with the accompanying drawings. In the drawings:
FIG. 1 is a plan view illustrating a display device according to one or more embodiments of the present disclosure;
FIG. 2 is a cross-sectional view illustrating a portion taken along the line I-I' of FIG. 1 according to one or more embodiments of the present disclosure;
FIG. 3 is a cross-sectional view schematically illustrating a light-emitting element according to one or more embodiments of the present disclosure;
FIG. 4 is a cross-sectional view schematically illustrating a light-emitting element according to one or more embodiments of the present disclosure;
FIG. 5 is a cross-sectional view schematically illustrating a light-emitting element according to one or more embodiments of the present disclosure;
FIG. 6 is a cross-sectional view schematically illustrating a light-emitting element according to one or more embodiments of the present disclosure;
FIG. 7 is a cross-sectional view illustrating a display device according to one or more embodiments of the present disclosure;
FIG. 8 is a cross-sectional view illustrating a display device according to one or more embodiments of the present disclosure;
FIG. 9 is a cross-sectional view illustrating a display device according to one or more embodiments of the present disclosure;
FIG. 10 is a cross-sectional view illustrating a display device according to one or more embodiments of the present disclosure;
FIG. 11 is a view illustrating an interior of a vehicle in which a display device according to one or more embodiments is arranged;
FIG. 12 is a perspective view illustrating an electronic apparatus according to one or more embodiments of the present disclosure;
FIG. 13 is an exploded perspective view illustrating an electronic apparatus according to one or more embodiments of the present disclosure;
FIG. 14 is a block diagram of an electronic apparatus according to one or more embodiments of the present disclosure; and
FIG. 15 shows schematic views of electronic apparatuses according to one or more embodiments of the present disclosure.

### DETAILED DESCRIPTION

The present disclosure may be modified in many alternate forms, and thus specific/example embodiments will be exemplified in the drawings and described in more detail. It should be understood, however, that it is not intended to limit the present disclosure to the particular forms disclosed, but rather, is intended to cover all modifications, equivalents, and alternatives falling within the scope of the present disclosure.

In this disclosure, it will be understood that if (e.g., when) an element (or a region, a layer, a portion, and/or the like) is referred to as being "on", "connected to" or "coupled to" another element, it may be directly arranged on, connected to, or coupled to the other element, or one or more other elements may be arranged therebetween. In the present disclosure, "directly on" may refer to that there are no additional layers, films, regions, plates, and/or like, between a layer, a film, a region, a plate, etc. and the other part. For example, "directly on" may refer to two layers or two members are arranged without utilizing an additional member such as an adhesive member therebetween.

Like reference numerals or symbols refer to like elements throughout the disclosure, and duplicative descriptions thereof may not be provided for conciseness. In the drawings, the thickness, ratio, and size of the elements may be exaggerated for effectively describing the technical contents. The terms "and/or" and/or "/" and/or "or" may include any and all combinations of one or more of the associated listed elements. Expressions such as "at least one of," "one of," and "selected from," when preceding a list of elements, modify the entire list of elements and do not modify the individual elements of the list. For example, "at least one of a, b, or c", "at least one selected from a, b, and c", "at least one selected from among a to c", etc., may indicate only a, only b, only c, both (e.g., simultaneously) a and b, both (e.g., simultaneously) a and c, both (e.g., simultaneously) b and c, all of a, b, and c, or variations thereof. The "/" utilized herein may be interpreted as "and" or as "or" depending on the situation.

It will be understood that, although the terms "first", "second", and/or the like, may be used herein to describe one or more suitable elements, the elements are not to be limited by these terms. These terms are only used to distinguish one element from another element. For instance, a first element could be termed a second element without departing from the scope of the disclosure. Similarly, a second element could be termed a first element. The singular expressions "a", "an", "one", and "the" are intended to include the plural forms as well, unless the context clearly indicates otherwise. Further, the utilization of "may" when describing embodiments of the present disclosure refers to "one or more embodiments of the present disclosure."

In addition, the terms "below", "under", "on the lower side", "above", "over", "on the upper side", and/or the like may be used to describe the relationships between the elements illustrated in the drawings. These terms are relative concepts and are described on the basis of the directions indicated in the drawings.

It will be further understood that the terms "comprise(s)/comprising," "include(s)/including," and/or "has(have)/having," if (e.g., when) used in this disclosure, specify the presence of stated features, numbers, steps, operations, elements, components, or combinations thereof, but do not preclude the possibility of the presence or addition of one or more other features, numbers, steps, operations, elements, components, and/or combinations thereof. Additionally, the terms "comprise(s)/comprising," "include(s)/including," "has(have)/having," or other similar terms include or support the terms "consisting of" and "consisting essentially of," indicating the presence of stated features, numbers, steps, operations, elements, and/or components, without or essentially without the presence of other features, numbers, steps, operations, elements, components, and/or groups thereof.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

In the specification, an integer selected from 0 to 3 refers to an integer selected from 0, 1, 2, and 3; an integer selected from 0 to 4 refers to an integer selected from 0, 1, 2, 3, and 4; an integer selected from 0 to 5 refers to an integer selected from 0, 1, 2, 3, 4, and 5; an integer selected from 0 to 7 refers to an integer selected from 0, 1, 2, 3, 4, 5, 6, and 7; an integer selected from 0 to 8 refers to an integer selected from 0, 1, 2, 3, 4, 5, 6, 7, and 8; an integer selected from 0 to 9 refers to an integer selected from 0, 1, 2, 3, 4, 5, 6, 7, 8, and 9; and an integer selected from 0 to 10 refers to an integer selected from 0, 1, 2, 3, 4, 5, 6, 7, 8, 9, and 10.

In the disclosure, the term "substituted or unsubstituted" may refer to be substituted or unsubstituted with at least one substituent selected from the group consisting of deuterium, a halogen, a cyano group, a nitro group, an amine group, an amino group, a silyl group, an oxy group, a thio group, a sulfinyl group, a sulfonyl group, a carbonyl group, a boron group, a phosphine oxide group, a phosphine sulfide group, an alkyl group, an alkenyl group, an alkynyl group, a hydrocarbon ring group, an aryl group, and a heterocyclic group. In addition, each of the substituents exemplified above may be substituted or unsubstituted. For example, a biphenyl group may be interpreted as an aryl group or a phenyl group substituted with a phenyl group.

In the disclosure, the phrase "bonded to an adjacent group to form a ring" may refer to that a group is bonded to an adjacent group to form a substituted or unsubstituted hydrocarbon ring or a substituted or unsubstituted heterocycle. The hydrocarbon ring may include an aliphatic hydrocarbon ring and/or an aromatic hydrocarbon ring. The heterocycle may include an aliphatic heterocycle and/or an aromatic heterocycle. The hydrocarbon ring and the heterocycle may each be monocyclic or polycyclic. In addition, the rings formed by adjacent groups being bonded to each other may be connected to another ring to form a spiro structure.

In the disclosure, the term "adjacent group" may refer to a substituent substituted for an atom which is directly linked to an atom substituted with a corresponding substituent, another substituent substituted for an atom which is substituted with a corresponding substituent, or a substituent sterically positioned at the nearest position to a corresponding substituent. For example, two methyl groups in 1,2-dimethylbenzene may be interpreted as "adjacent groups" to each other, and two ethyl groups in 1,1-diethylcyclopentane may be interpreted as "adjacent groups" to each other. In addition, two methyl groups in 4,5-dimethylphenanthrene may be interpreted as "adjacent groups" to each other.

In the disclosure, examples of a halogen may include fluorine, chlorine, bromine, or iodine.

In the disclosure, an alkyl group may be linear or branched. The number of carbons in the alkyl group is 1 to 60, 1 to 50, 1 to 30, 1 to 20, 1 to 10, or 1 to 6. Examples of the alkyl group may include a methyl group, an ethyl group, an n-propyl group, an isopropyl group, an n-butyl group, an s-butyl group, a t-butyl group, an i-butyl group, a 2-ethylbutyl group, a 3,3-dimethylbutyl group, an n-pentyl group, an i-pentyl group, a neopentyl group, a t-pentyl group, a 1-methylpentyl group, a 3-methylpentyl group, a 2-ethylpentyl group, a 4-methyl-2-pentyl group, an n-hexyl group, a 1-methylhexyl group, a 2-ethylhexyl group, a 2-butylhexyl group, an n-heptyl group, a 1-methylheptyl group, a 2,2-dimethylheptyl group, a 2-ethylheptyl group, a 2-butylheptyl group, an n-octyl group, a t-octyl group, a 2-ethyloctyl group, a 2-butyloctyl group, a 2-hexyloctyl group, a 3,7-dimethyloctyl group, an n-nonyl group, an n-decyl group, a 2-ethyldecyl group, a 2-butyldecyl group, a 2-hexyldecyl group, a 2-octyldecyl group, an n-undecyl group, an n-dodecyl group, a 2-ethyldodecyl group, a 2-butyldodecyl group, a 2-hexyldocecyl group, a 2-octyldodecyl group, an n-tridecyl group, an n-tetradecyl group, an n-pentadecyl group, an n-hexadecyl group, a 2-ethylhexadecyl group, a 2-butylhexadecyl group, a 2-hexylhexadecyl group, a 2-octylhexadecyl group, an n-heptadecyl group, an n-octadecyl group, an n-nonadecyl group, an n-eicosyl group, a 2-ethyleicosyl group, a 2-butyleicosyl group, a 2-hexyleicosyl group, a 2-octyleicosyl group, an n-henicosyl group, an n-docosyl group, an n-tricosyl group, an n-tetracosyl group, an n-pentacosyl group, an n-hexacosyl group, an n-heptacosyl group, an n-octacosyl group, an n-nonacosyl group, an n-triacontyl group, and/or the like, but embodiments of the present disclosure are not limited thereto.

In the disclosure, a cycloalkyl group may refer to a cyclic alkyl group. The number of carbons in the cycloalkyl group is 3 to 60, 3 to 50, 3 to 30, 3 to 20, or 3 to 10. Examples of the cycloalkyl group may include a cyclopropyl group, a cyclobutyl group, a cyclopentyl group, a cyclohexyl group, a 4-methylcyclohexyl group, a 4-t-butylcyclohexyl group, a cycloheptyl group, a cyclooctyl group, a cyclononyl group, a cyclodecyl group, a norbornyl group, a 1-adamantyl group, a 2-adamantyl group, an isobornyl group, a bicycloheptyl group, and/or the like, but embodiments of the present disclosure are not limited thereto.

In the disclosure, an alkenyl group refers to a hydrocarbon group including at least one carbon-carbon double bond in the middle or terminal of an alkyl group having 2 or more carbon atoms. The alkenyl group may be linear or branched. The number of carbon atoms in the alkenyl group is not specifically limited, for example, may be 2 to 60, 2 to 30, 2 to 20, or 2 to 10. Examples of the alkenyl group include a vinyl group, a 1-butenyl group, a 1-pentenyl group, a 1,3-butadienyl group, a styrenyl group, a styryl vinyl group, and/or the like, but embodiments of the present disclosure are not limited thereto.

In the disclosure, an alkynyl group refers to a hydrocarbon group including at least one carbon-carbon triple bond in the middle or terminal of an alkyl group having 2 or more carbon atoms. The alkynyl group may be linear or branched. Although the number of carbon atoms is not specifically limited, it may be 2 to 30, 2 to 20, or 2 to 10. Examples of the alkynyl group may include an ethynyl group, a propynyl group, and/or the like, but embodiments of the present disclosure are not limited thereto.

In the disclosure, a hydrocarbon ring group may refer to any functional group or substituent derived from an aliphatic hydrocarbon ring or an aromatic hydrocarbon ring. The hydrocarbon ring group may be a saturated hydrocarbon ring group having 6 to 60, 6 to 30, 5 to 30, or 5 to 20 ring-forming carbon atoms.

In the disclosure, an aryl group refers to any functional group or substituent derived from an aromatic hydrocarbon ring. The aryl group may be a monocyclic aryl group or a polycyclic aryl group. The number of ring-forming carbon atoms in the aryl group may be 6 to 60, 6 to 30, 6 to 20, or 6 to 15. Examples of the aryl group may include a phenyl group, a naphthyl group, a fluorenyl group, an anthracenyl group, a phenanthryl group, a biphenyl group, a terphenyl group, a quaterphenyl group, a quinquephenyl group, a sexiphenyl group, a triphenylenyl group, a pyrenyl group, a benzofluoranthenyl group, a chrysenyl group, and/or the like, but embodiments of the present disclosure are not limited thereto.

In the disclosure, a fluorenyl group may be substituted, and two substituents may be bonded to each other to form a spiro structure. Examples of the substituted fluorenyl group are as follows. However, embodiments of the present disclosure are not limited thereto.

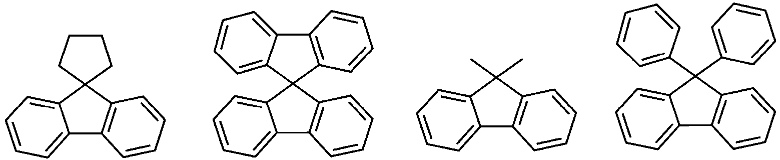

A heterocyclic group as used herein refers to any functional group or substituent derived from a ring containing at least one of B, O, N, P, Si, S, or Se as a heteroatom. The heterocyclic group includes an aliphatic heterocyclic group and/or an aromatic heterocyclic group. The aromatic heterocyclic group may be a heteroaryl group. The aliphatic heterocycle and the aromatic heterocycle may each be monocyclic or polycyclic.

In the disclosure, the heterocyclic group may contain at least one of B, O, N, P, Si, S or Se as a heteroatom. If (e.g., when) the heterocyclic group contains two or more heteroatoms, the two or more heteroatoms may be the same as or different from each other. The heterocyclic group may be a monocyclic heterocyclic group or a polycyclic heterocyclic group, and includes a heteroaryl group. The number of ring-forming carbon atoms in the heterocyclic group may be 2 to 60, 2 to 30, 5 to 30, 2 to 20, or 2 to 10.

In the disclosure, the aliphatic heterocyclic group may include at least one of B, O, N, P, Si, S or Se as a heteroatom. The number of ring-forming carbon atoms in the aliphatic heterocyclic group may be 2 to 60, 2 to 30, 2 to 20, or 2 to 10. Examples of the aliphatic heterocyclic group may include an oxirane group, a thiirane group, a pyrrolidine group, a piperidine group, a tetrahydrofuran group, a tetrahydrothiophene group, a thiane group, a tetrahydropyran group, a 1,4-dioxane group, and/or the like, but embodiments of the present disclosure are not limited thereto.

In the disclosure, the heteroaryl group may contain at least one of B, O, N, P, Si, S or Se as a heteroatom. If (e.g., when) the heteroaryl group contains two or more heteroatoms, the two or more heteroatoms may be the same as or different from each other. The heteroaryl group may be a monocyclic heterocyclic group or a polycyclic heterocyclic group. The number of ring-forming carbon atoms in the heteroaryl group may be 2 to 60, 2 to 30, 2 to 20, or 2 to 10. Examples of the heteroaryl group may include a thiophene group, a furan group, a pyrrole group, an imidazole group, a pyridine group, a bipyridine group, a pyrimidine group, a triazine group, a triazole group, an acridyl group, a pyridazine group, a pyrazinyl group, a quinoline group, a quinazoline group, a quinoxaline group, a phenoxazine group, a phthalazine group, a pyrido pyrimidine group, a pyrido pyrazine group, a pyrazino pyrazine group, an isoquinoline group, an indole group, a carbazole group, an N-arylcarbazole group, an N-heteroarylcarbazole group, an N-alkylcarbazole group, a benzoxazole group, a benzimidazole group, a benzothiazole group, a benzocarbazole group, a benzothiophene group, a dibenzothiophene group, a thienothiophene group, a benzofuran group, a phenanthroline group, a thiazole group, an isoxazole group, an oxazole group, an oxadiazole group, a thiadiazole group, a phenothiazine group, a dibenzosilole group, a dibenzofuran group, and/or the like, but embodiments of the present disclosure are not limited thereto.

In the disclosure, the above description of the aryl group may be applied to an arylene group except that the arylene group is a divalent group. The above description of the heteroaryl group may be applied to a heteroarylene group except that the heteroarylene group is a divalent group.

In the disclosure, a silyl group includes an alkylsilyl group and/or an arylsilyl group. Examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and/or the like, but embodiments of the present disclosure are not limited thereto.

In the disclosure, the number of carbon atoms in a carbonyl group is not specifically limited, but may be 1 to 40, 1 to 30, or 1 to 20. For example, the carbonyl group may have the following structures, but embodiments of the present disclosure are not limited thereto.

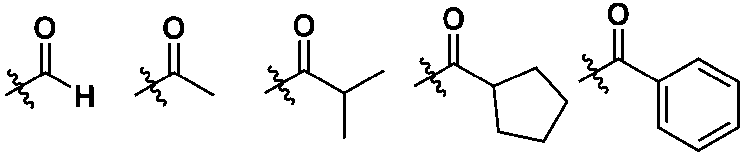

In the disclosure, the number of carbon atoms in a sulfinyl group or a sulfonyl group is not particularly limited, for example, may be 1 to 30. The sulfinyl group may include an alkyl sulfinyl group and/or an aryl sulfinyl group. The sulfonyl group may include an alkyl sulfonyl group and/or an aryl sulfonyl group.

In the disclosure, a thio group may include an alkylthio group and/or an arylthio group. The thio group may refer to that a sulfur atom is bonded to the alkyl group or the aryl group defined above. Examples of the thio group may include a methylthio group, an ethylthio group, a propylthio group, a pentylthio group, a hexylthio group, an octylthio group, a dodecylthio group, a cyclopentylthio group, a cyclohexylthio group, a phenylthio group, a naphthylthio group, but embodiments of the present disclosures are not limited thereto.

In the disclosure, an oxy group may refer to that an oxygen atom is bonded to the alkyl group or the aryl group defined above. The oxy group may include an alkoxy group and/or an aryl oxy group. The alkoxy group may be a linear chain, a branched chain, or a ring. The number of carbon atoms in the alkoxy group is not specifically limited, but may be, for example, 1 to 20 or 1 to 10. Examples of the oxy group may include methoxy, ethoxy, n-propoxy, isopropoxy, butoxy, pentyloxy, hexyloxy, octyloxy, nonyloxy, decyloxy, benzyloxy, and/or the like, but embodiments of the present disclosure are not limited thereto.

A boron group as used herein may refer to that a boron atom is bonded to the alkyl group or the aryl group defined above. The boron group includes an alkyl boron group and/or an aryl boron group. Examples of the boron group may include a dimethylboron group, a diethylboron group, a t-butylmethylboron group, a diphenylboron group, a phenylboron group, and/or the like, but embodiments of the present disclosure are not limited thereto.

In the disclosure, the number of carbon atoms in an amine group is not specifically limited, for example, may be 1 to 50, 1 to 30, or 1 to 20. The amine group may include an alkyl amine group and/or an aryl amine group. Examples of the amine group may include a methylamine group, a dimethylamine group, a phenylamine group, a diphenylamine group, a naphthylamine group, a 9-methyl-anthracenylamine group, and/or the like, but embodiments of the present disclosure are not limited thereto.

In the disclosure, the alkyl group among an alkylthio group, an alkylsulfoxy group, an alkylaryl group, an alkylamino group, an alkyl boron group, an alkyl silyl group, and an alkyl amine group is the same as the examples of the alkyl group described above.

In the disclosure, the aryl group among an aryloxy group, an arylthio group, an arylsulfoxy group, an arylamino group, an arylboron group, an arylsilyl group, an arylamine group is the same as the examples of the aryl group described above.

In the disclosure, the phosphine oxide group may be substituted with, for example, at least one of the alkyl groups or aryl groups described above.

In the disclosure, the phosphine sulfide group may be substituted with, for example, at least one of the alkyl groups or aryl groups described above.

In the disclosure, a direct linkage may refer to a single bond. In the disclosure, " 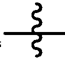 " and "-*"each refer to a position to be connected.

Hereinafter, embodiments of the disclosure will be described with reference to the accompanying drawings.

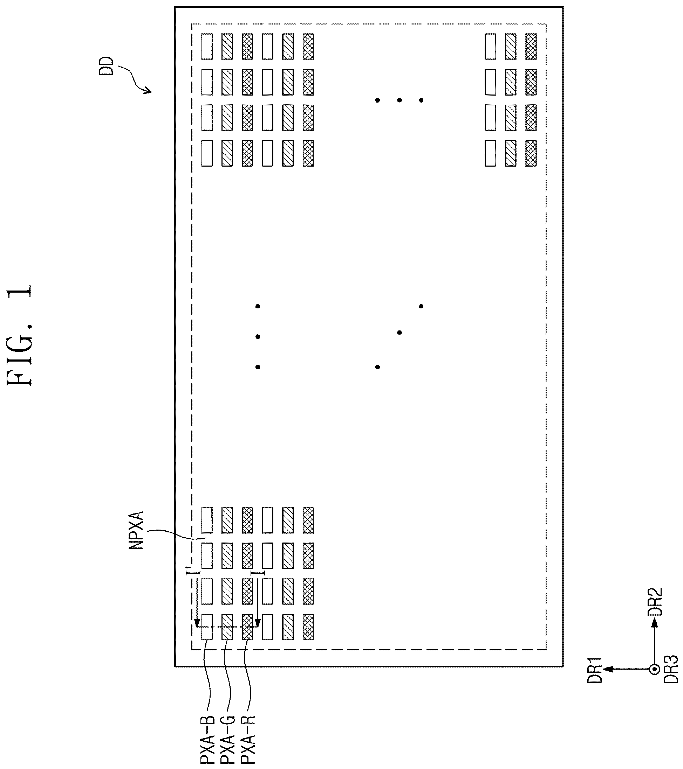

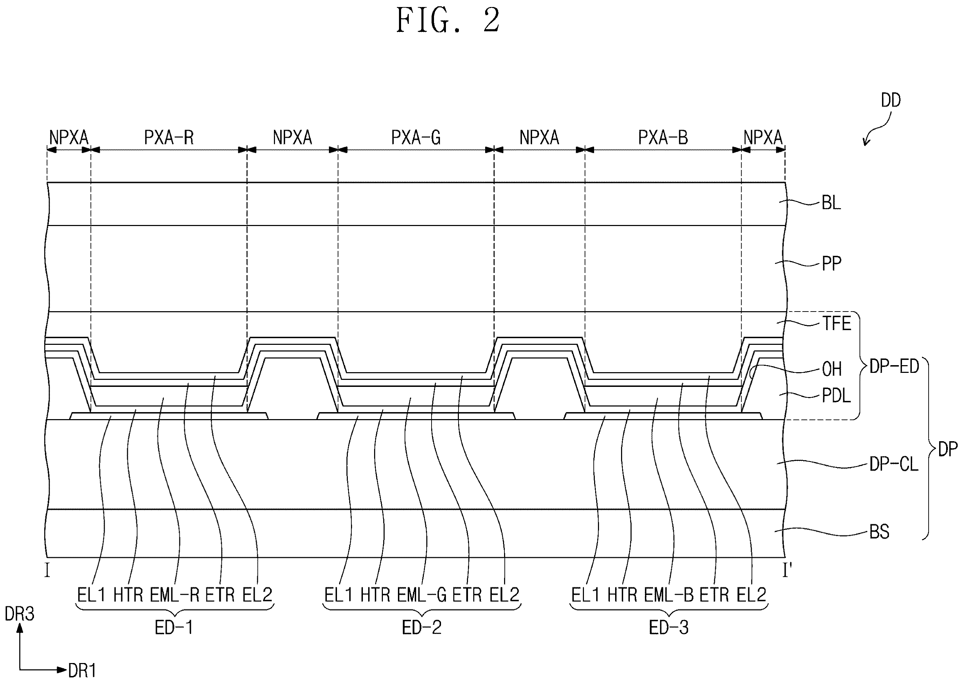

FIG. 1 is a plan view illustrating a display device DD according to one or more embodiments of the present disclosure. FIG. 2 is a cross-sectional view of the display device DD according to one or more embodiments of the present disclosure. FIG. 2 is a cross-sectional view illustrating a part taken along the line I-I' of the display device DD of FIG. 1 according to one or more embodiments.

The display device DD may include a display panel DP and an optical layer PP arranged on the display panel DP. The display panel DP may include light-emitting elements ED-1, ED-2, and ED-3. The display device DD may include a plurality of light-emitting elements ED-1, ED-2, and ED-3. The optical layer PP may be arranged on the display panel DP to control reflected light in the display panel DP due to external light. The optical layer PP may include, for example, a polarization layer and/or a color filter layer. In one or more embodiments, the optical layer PP may not be provided in the display device DD.

A base substrate BL may be arranged on the optical layer PP. The base substrate BL may be a member which provides a base surface on which the optical layer PP is arranged. The base substrate BL may be a glass substrate, a metal substrate, a plastic substrate, and/or the like. However, embodiments of the present disclosure are not limited thereto, and the base substrate BL may be an inorganic layer, an organic layer, or a composite material layer. In one or more embodiments, the base substrate BL may not be provided.

The display device DD according to one or more embodiments may further include a filling layer. The filling layer may be arranged between a display element layer DP-ED and the base substrate BL. The filling layer may be an organic material layer. The filling layer may include at least one of an acrylic-based resin, a silicone-based resin, or an epoxy-based resin.

The display panel DP may include a base layer BS, a circuit layer DP-CL provided on the base layer BS, and the display element layer DP-ED. The display element layer DP-ED may include a pixel defining film PDL, the light-emitting elements ED-1, ED-2, and ED-3 respectively arranged between portions of the pixel defining film PDL, and an encapsulation layer TFE arranged on the light-emitting elements ED-1, ED-2, and ED-3.

The base layer BS may be a member which provides a base surface on which the display element layer DP-ED is arranged. The base layer BS may be a glass substrate, a metal substrate, a plastic substrate, and/or the like. However, embodiments of the present disclosure are not limited thereto, and the base layer BS may be an inorganic layer, an organic layer, or a composite material layer.

In one or more embodiments, the circuit layer DP-CL is arranged on the base layer BS, and the circuit layer DP-CL may include a plurality of transistors. Each of the transistors may include a control electrode, an input electrode, and an output electrode. For example, in one or more embodiments, the circuit layer DP-CL may include switching transistor(s) and driving transistor(s) for driving the light-emitting elements ED-1, ED-2, and ED-3 of the display element layer DP-ED.

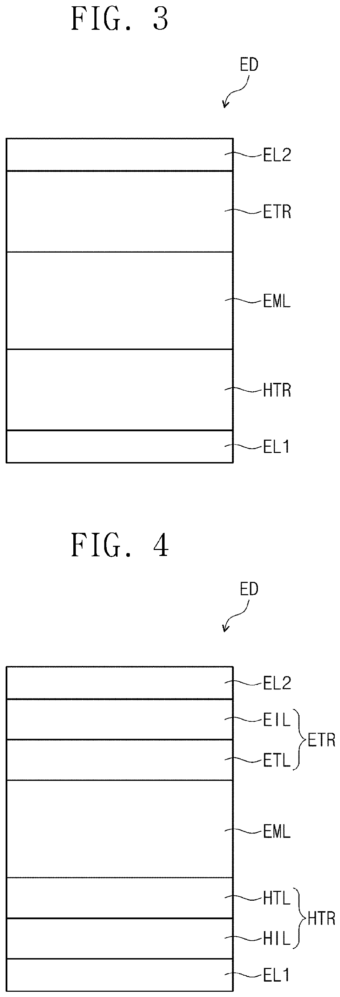

Each of the light-emitting elements ED-1, ED-2, and ED-3 may have a structure of one of the light-emitting elements ED of embodiments according to FIGS. 3 to 6, which will be described later. Each of the light-emitting elements ED-1, ED-2, and ED-3 may include a first electrode EL1, a hole transport region HTR, respective emission layers EML-R, EML-G, and EML-B, an electron transport region ETR, and a second electrode EL2.

FIG. 2 illustrates one or more embodiments in which the respective emission layers EML-R, EML-G, and EML-B of the light-emitting elements ED-1, ED-2, and ED-3 are arranged in openings OH defined in the pixel defining film PDL, and the hole transport region HTR, the electron transport region ETR, and the second electrode EL2 are each provided as a common layer across the entire light-emitting elements ED-1, ED-2, and ED-3. However, embodiments of the present disclosure are not limited thereto, for example, in one or more embodiments, the hole transport region HTR and the electron transport region ETR may each be provided by being patterned inside the openings OH defined in the pixel defining film PDL. For example, in one or more embodiments, the hole transport region HTR, the respective emission layers EML-R, EML-G, and EML-B, and the electron transport region ETR of the light-emitting elements ED-1, ED-2, and ED-3 may be provided by being patterned in an inkjet printing method. For example, in one or more embodiments, the hole transport region HTR and the electron transport region ETR may be individually patterned within the openings of the pixel defining film PDL, rather than being formed as continuous layers. In such cases, the hole transport region HTR, the emission layer EML-R/G/B, and the electron transport region ETR may be deposited utilizing a patterned method such as inkjet printing, allowing for precise material placement and improved pixel definition.

The encapsulation layer TFE may cover the light-emitting elements ED-1, ED-2, and ED-3. The encapsulation layer TFE may seal the display element layer DP-ED. The encapsulation layer TFE may be a thin film encapsulation layer. The encapsulation layer TFE may be formed by laminating one layer or a plurality of layers. The encapsulation layer TFE may include at least one insulation layer. The encapsulation layer TFE according to one or more embodiments may include at least one inorganic film (hereinafter, an encapsulation-inorganic film). The encapsulation layer TFE according to one or more embodiments may include at least one organic film (hereinafter, an encapsulation-organic film) and at least one encapsulation-inorganic film.

The encapsulation-inorganic film protects the display element layer DP-ED from moisture/oxygen, and the encapsulation-organic film protects the display element layer DP-ED from foreign substances such as dust particles. The encapsulation-inorganic film may include silicon nitride, silicon oxynitride, silicon oxide, titanium oxide, aluminium oxide, and/or the like, but embodiments of the present disclosure are not particularly limited thereto. The encapsulation-organic film may include an acrylic-based compound, an epoxy-based compound, and/or the like. In one or more embodiments, the encapsulation-organic film may include a photopolymerizable organic material, but embodiments of the present disclosure are not particularly limited thereto.

The encapsulation layer TFE may be arranged on the second electrode EL2 and may be arranged to fill the opening OH.

Referring to FIG. 1 and FIG. 2, the display device DD may include a non-light emitting region NPXA and light emitting regions PXA-R, PXA-G, and PXA-B. The light emitting regions PXA-R, PXA-G, and PXA-B may be regions in which light generated by the respective light-emitting elements ED-1, ED-2, and ED-3 is emitted. The light emitting regions PXA-R, PXA-G, and PXA-B may be spaced apart (e.g., spaced and/or apart) from one another on a plane (e.g., in plan view).

Each of the light emitting regions PXA-R, PXA-G, and PXA-B may be a region divided by the pixel defining film PDL. The non-light emitting region NPXA may be regions between neighboring light emitting regions PXA-R, PXA-G, and PXA-B, which corresponds to the pixel defining film PDL. In the disclosure, the light emitting regions PXA-R, PXA-G, and PXA-B may respectively correspond to pixels. The pixel defining film PDL may divide the light-emitting elements ED-1, ED-2, and ED-3. The respective emission layers EML-R, EML-G, and EML-B of the light-emitting elements ED-1, ED-2, and ED-3 may be arranged in openings OH defined in the pixel defining film PDL and separated from one another.

The light emitting regions PXA-R, PXA-G, and PXA-B may be divided into a plurality of groups according to the color of light generated from the light-emitting elements ED-1, ED-2, and ED-3. In the display device DD of one or more embodiments illustrated in FIG. 1 and FIG. 2, three light emitting regions PXA-R, PXA-G, and PXA-B, which emit red light, green light, and blue light, respectively, are exemplarily illustrated. For example, in one or more embodiments, the display device DD may include a red light emitting region PXA-R, a green light emitting region PXA-G, and a blue light emitting region PXA-B that are separated from one another.

In the display device DD according to one or more embodiments, the plurality of light-emitting elements ED-1, ED-2, and ED-3 may be to emit light beams having wavelengths different from one another. For example, in one or more embodiments, the display device DD may include a first light-emitting element ED-1 that emits red light, a second light-emitting element ED-2 that emits green light, and a third light-emitting element ED-3 that emits blue light. For example, the red light emitting region PXA-R, the green light emitting region PXA-G, and the blue light emitting region PXA-B of the display device DD may correspond to the first light-emitting element ED-1, the second light-emitting element ED-2, and the third light-emitting element ED-3, respectively.

However, embodiments of the present disclosure are not limited thereto, and the first to third light-emitting elements ED-1, ED-2, and ED-3 may be to emit light beams in substantially the same wavelength range, or at least one light-emitting element may be to emit a light beam in a wavelength range different from the others. For example, in one or more embodiments, the first to third light-emitting elements ED-1, ED-2, and ED-3 may all emit blue light.

The light emitting regions PXA-R, PXA-G, and PXA-B in the display device DD according to one or more embodiments may be arranged in a stripe form. Referring to FIG. 1, the plurality of red light emitting regions PXA-R may be arranged with each other along a second direction axis DR2, the plurality of green light emitting regions PXA-G may be arranged with each other along the second direction axis DR2, and the plurality of blue light emitting regions PXA-B may be arranged with each other along the second direction axis DR2. In addition, the red light emitting region PXA-R, the green light emitting region PXA-G, and the blue light emitting region PXA-B may be alternately arranged in this order along a first direction axis DR1.

FIG. 1 and FIG. 2 illustrate that all the light emitting regions PXA-R, PXA-G, and PXA-B have similar area, but embodiments of the present disclosure are not limited thereto. For example, in one or more embodiments, the light emitting regions PXA-R, PXA-G, and PXA-B may have different areas from one another according to the wavelength range of the emitted light. In this regard, the areas of the light emitting regions PXA-R, PXA-G, and PXA-B may refer to areas if (e.g., when) viewed on a plane defined by the first direction axis DR1 and the second direction axis DR2.

An arrangement form of the light emitting regions PXA-R, PXA-G, and PXA-B is not limited to the configuration illustrated in FIG. 1, and the order in which the red light emitting region PXA-R, the green light emitting region PXA-G, and the blue light emitting region PXA-B are arranged may be provided in one or more suitable combinations according to the characteristics of display quality desired or required in the display device DD. For example, in one or more embodiments, the arrangement form of the light emitting regions PXA-R, PXA-G, and PXA-B may be a pentile (PENTILE^{®}) arrangement form (for example, an RGBG matrix, an RGBG structure, or an RGBG matrix structure) or a diamond (Diamond Pixel^{™}) arrangement form (e.g., red, blue, and green (RGB) light-emitting regions arranged in the shape of diamonds). PENTILE^{®} is a duly registered trademark of Samsung Display Co., Ltd. Diamond Pixel^{™} is a trademark of Samsung Display Co., Ltd.

In one or more embodiments, the areas of the light emitting regions PXA-R, PXA-G, and PXA-B may be different from one another. For example, in one or more embodiments, the area of the green light emitting region PXA-G may be smaller than that of the blue light emitting region PXA-B, but embodiments of the present disclosure are not limited thereto.

Hereinafter, FIG. 3 to FIG. 6 are each a cross-sectional view schematically showing a light-emitting element according to one or more embodiments of the present disclosure. A light-emitting element ED of one or more embodiments includes a first electrode EL1, a hole transport region HTR, an emission layer EML, an electron transport region ETR, and a second electrode EL2, which are stacked in the stated order.

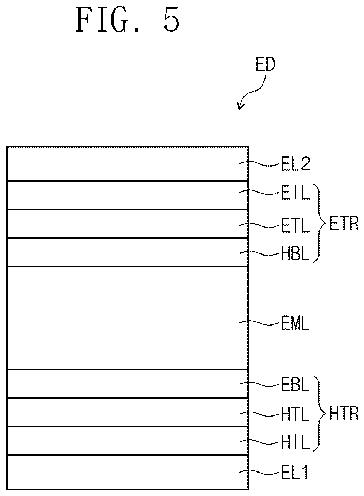

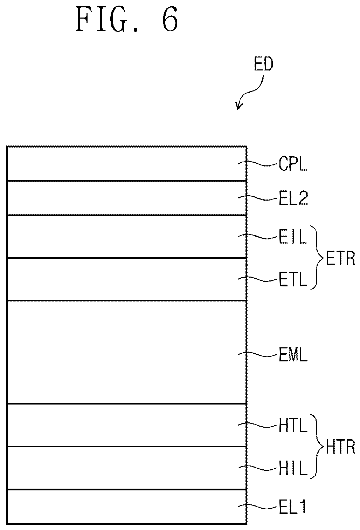

Compared with FIG. 3, FIG. 4 illustrates a cross-sectional view of a light-emitting element ED of one or more embodiments, in which a hole transport region HTR includes a hole injection layer HIL and a hole transport layer HTL, and an electron transport region ETR includes an electron injection layer EIL and an electron transport layer ETL. In addition, compared with FIG. 3, FIG. 5 illustrates a cross-sectional view of a light-emitting element ED of one or more embodiments, in which a hole transport region HTR includes a hole injection layer HIL, a hole transport layer HTL, and an electron blocking layer EBL, and an electron transport region ETR includes an electron injection layer EIL, an electron transport layer ETL, and a hole blocking layer HBL. Compared with FIG. 4, FIG. 6 illustrates a cross-sectional view of a light-emitting element ED of one or more embodiments, in which a capping layer CPL is arranged on the second electrode EL2.

The first electrode EL1 has conductivity (e.g., is an electron conductor). The first electrode EL1 may be formed of a metal material, a metal alloy, or a conductive compound. The first electrode EL1 may be an anode or a cathode. However, embodiments of the present disclosure are not limited thereto. In one or more embodiments, the first electrode EL1 may be a pixel electrode. The first electrode EL1 may be a transmissive electrode, a transflective electrode, or a reflective electrode. The first electrode EL1 may include at least one selected from among silver (Ag), magnesium (Mg), copper (Cu), aluminium (Al), platinum (Pt), palladium (Pd), gold (Au), nickel (Ni), neodymium (Nd), iridium (Ir), chromium (Cr), lithium (Li), calcium (Ca), lithium fluoride (LiF), molybdenum (Mo), titanium (Ti), tungsten (W), indium (In), tin (Sn), and zinc (Zn), a compound of two or more selected therefrom, a mixture of two or more selected therefrom, or an oxide thereof.

If (e.g., when) the first electrode EL1 is a transmissive electrode, the first electrode EL1 may include a transparent metal oxide such as indium tin oxide (ITO), indium zinc oxide (IZO), zinc oxide (ZnO), or indium tin zinc oxide (ITZO). If (e.g., when) the first electrode EL1 is a transflective electrode or a reflective electrode, the first electrode EL1 may include Ag, Mg, Cu, Al, Pt, Pd, Au, Ni, Nd, Ir, Cr, Li, Ca, LiF/Ca (a stacked structure of LiF and Ca), LiF/Al (a stacked structure of LiF and Al), Mo, Ti, W, or a compound or a mixture thereof (e.g., a mixture of Ag and Mg). In one or more embodiments, the first electrode EL1 may have a multilayer structure including a reflective film or a transflective film formed of one or more of the above-described materials, and a transparent conductive film formed of ITO, IZO, ZnO, ITZO, and/or the like. For example, in one or more embodiments, the first electrode EL1 may have a three-layer structure of ITO/Ag/ITO, but embodiments of the present disclosure are not limited thereto. In one or more embodiments, the first electrode EL1 may include one of the above-described metal materials, a combination of at least two metal materials of the above-described metal materials, an oxide of the above-described metal materials, and/or the like. A thickness of the first electrode EL1 may be from about 700 angstroms (Å) to about 10,000 Å. For example, in one or more embodiments, the thickness of the first electrode EL1 may be from about 1,000 Å to about 3,000 Å.

The light-emitting element ED according to one or more embodiments includes a monoamine compound of one or more embodiments of the present disclosure. In one or more embodiments, the hole transport region HTR includes a monoamine compound of one or more embodiments. In one or more embodiments, at least one of the hole injection layer HIL, the hole transport layer HTL, or the electron blocking layer EBL may include a monoamine compound of one or more embodiments. For example, in one or more embodiments, the hole transport layer HTL may include a monoamine compound of one or more embodiments.

The monoamine compound according to one or more embodiments may include only one amine group. In the present disclosure, a group in which substituents (for example, an aryl group) bonded to an amine group are bonded to each other to form a ring (for example, a carbazole group) is not included in an amine group, and such a group (for example, a carbazole group) is included in a heteroaryl group. A cyclic group including a nitrogen atom as a ring-forming atom (for example, a pyridine group, a pyrimidine group, a triazine group, a carbazole group, and/or the like) is not included in an amine group, and is included in a heterocyclic group.

The monoamine compound according to one or more embodiments may include first to third substituents which are directly/indirectly bonded to the amine group of the monoamine compound. The first substituent may be a diphenylphenyl group (e.g., a terphenyl group), and the diphenylphenyl group may be a phenyl group substituted with two phenyl groups. The second substituent may be a phenylnaphthalenyl group, and may be a naphthalenyl group substituted with a phenyl group. The third substituent may be an aryl group or heteroaryl group. As a result, the monoamine compound according to one or more embodiments may exhibit excellent or suitable hole transporting property, and the light-emitting element ED according to one or more embodiments including the monoamine compound according to one or more embodiments may exhibit high light-emitting efficiency and long lifespan characteristics.

According to one or more embodiments of the present disclosure, the light-emitting element ED includes a monoamine compound of one or more embodiments. The monoamine compound of one or more embodiments is represented by Formula 1.

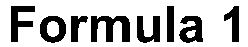

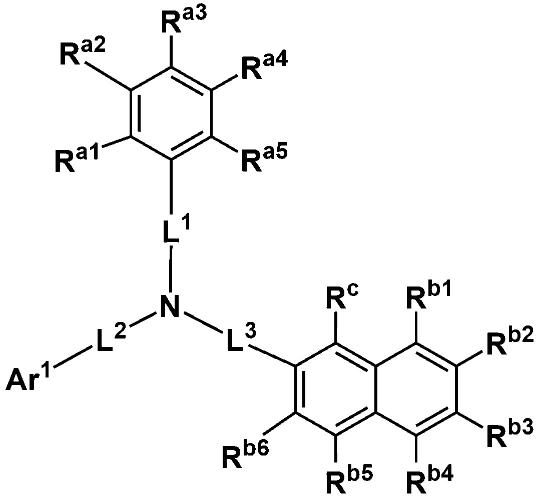

In Formula 1, a phenyl group including (e.g., substituted with) R^{a1} to R^{a5} may correspond to the above-described first substituent. A naphthalenyl group including (e.g., substituted with) R^{b1} to R^{b6} may correspond to the above-described second substituent. Ar¹ may correspond to the above-described third substituent.

In Formula 1, L¹ to L³ are each independently a direct linkage, a substituted or unsubstituted arylene group having 6 to 30 ring-forming carbon atoms, or a substituted or unsubstituted heteroarylene group having 5 to 30 ring-forming carbon atoms. For example, L¹ to L³ may each independently be a direct linkage, a substituted or unsubstituted arylene group having 6 to 20 ring-forming carbon atoms, or a substituted or unsubstituted heteroarylene group having 5 to 20 ring-forming carbon atoms. Atoms at an ortho position with respect to N in L¹ to L³ are each independently bonded to hydrogen or deuterium. If (e.g., when) L¹ is an arylene group or a heteroarylene group, an atom at an ortho position with respect to N to which L¹ is bonded (that is, a ring-forming atom in the arylene group, or a ring-forming atom in the heteroarylene group) may be bonded to hydrogen or deuterium. If (e.g., when) L² is an arylene group or a heteroarylene group, an atom at an ortho position with respect to N to which L² is bonded (that is, a ring-forming atom in the arylene group, or a ring-forming atom in the heteroarylene group) may be bonded to hydrogen or deuterium. If (e.g., when) L³ is an arylene group or a heteroarylene group, an atom at an ortho position with respect to N to which L³ is bonded (that is, a ring-forming atom in the arylene group, or a ring-forming atom in the heteroarylene group) may be bonded to hydrogen or deuterium.

For example, in one or more embodiments, L¹ to L³ may each independently be a direct linkage or a substituted or unsubstituted phenylene group. In one or more embodiments, L¹ to L³ may each independently be a direct linkage or be represented by L-1 or L-2. L-1 and L-2 each represent an unsubstituted phenylene group.

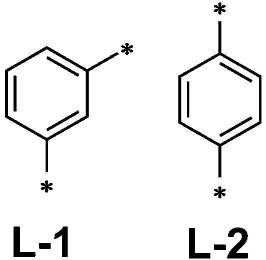

In Formula 1, Ar¹ is a substituted or unsubstituted aryl group having 10 to 30 ring-forming carbon atoms or a substituted or unsubstituted heteroaryl group having 5 to 30 ring-forming carbon atoms. For example, Ar1 may be a substituted or unsubstituted aryl group having 10 to 20 ring-forming carbon atoms or a substituted or unsubstituted heteroaryl group having 5 to 20 ring-forming carbon atoms. However, a case where Ar¹ is an aryl group, in which a plurality of phenyl groups is connected by a direct linkage, is excluded. For example, in one or more embodiments, Ar¹ may be a fused polycyclic aryl group or a fused polycyclic heteroaryl group, in each of which a plurality of cyclic groups is fused. If (e.g., when) Ar¹ is a heteroaryl group, O or S may be included as a ring-forming atom of the heteroaryl group.

In one or more embodiments, Ar¹ may be a substituted or unsubstituted naphthalene group, a substituted or unsubstituted phenanthrene group, a substituted or unsubstituted dibenzofuran group, a substituted or unsubstituted dibenzothiophene group, a substituted or unsubstituted benzonaphthofuran group, or a substituted or unsubstituted benzonaphthothiophene group. Ar¹ may be represented by any one selected from among AR-1 to AR-20. In one or more embodiments, in Formula 1, L² in Ar¹-L²-* may be a direct linkage or represented by the above-described L-2, and Ar¹ may be represented by any one selected from among AR-1 to AR-20. In AR-6, D is deuterium.

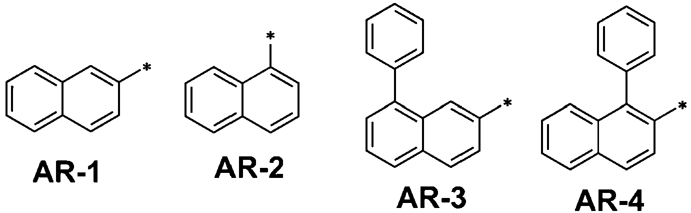

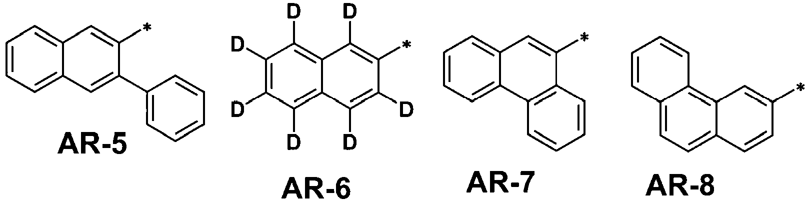

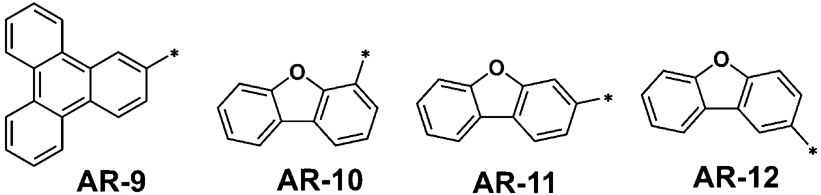

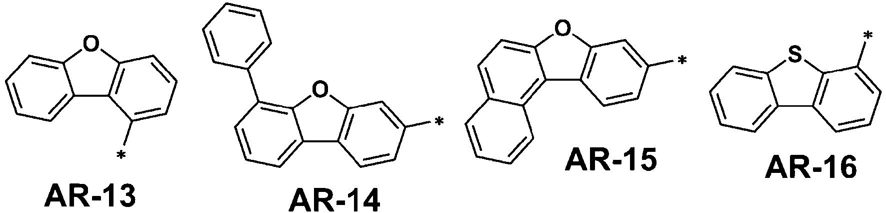

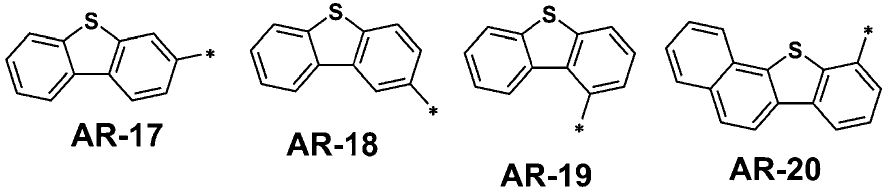

In Formula 1, at least two selected from among R^{a1} to R^{a5} are each a substituted or unsubstituted phenyl group, and the rest are each independently hydrogen, deuterium, a halogen, a substituted or unsubstituted alkyl group having 1 to 15 (e.g. 1 to 10) carbon atoms, a substituted or unsubstituted aryl group having 6 to 30 (e.g. 6 to 20) ring-forming carbon atoms, or a substituted or unsubstituted heteroaryl group having 5 to 30 (e.g. 5 to 20) ring-forming carbon atoms. However, a case where R^{a1} and R^{a5} are each a phenyl group at the same time is excluded.

If (e.g., when) R^{a2} and R^{a3} are phenyl groups or R^{a3} and R^{a4} are phenyl groups, L¹ is a direct linkage. For example, in one or more embodiments, if (e.g., when) R^{a2} and R^{a3} are each a phenyl group, L¹ is a direct linkage. In one or more embodiments, if (e.g., when) R^{a3} and R^{a4} are each a phenyl group, L¹ is a direct linkage.

For example, in one or more embodiments, two selected from among R^{a1} to R^{a5} may each be a substituted or unsubstituted phenyl group, and the rest may each independently be hydrogen or fluorine. If (e.g., when) two selected from among R^{a1} to R^{a5} are each a substituted or unsubstituted phenyl group, R^{a2} and R^{a5} may each be a phenyl group, R^{a2} and R^{a4} may each be a phenyl group, or R^{a3} and R^{a5} may each be a phenyl group.

In Formula 1, R^{c} is hydrogen, deuterium, or fluorine.

In Formula 1, at least one selected from among R^{b1} to R^{b6} is a substituted or unsubstituted phenyl group, and the rest are each independently hydrogen, deuterium, a halogen, a substituted or unsubstituted alkyl group having 1 to 15 (e.g. 1 to 10) carbon atoms, a substituted or unsubstituted aryl group having 6 to 30 (e.g. 6 to 20) ring-forming carbon atoms, or a substituted or unsubstituted heteroaryl group having 5 to 30 (e.g. 5 to 20) ring-forming carbon atoms. For example, in one or more embodiments, any one selected from among R^{b1} to R^{b6} may be a substituted or unsubstituted phenyl group, and the rest may each independently be hydrogen, deuterium, or fluorine.

The monoamine compound represented by Formula 1 may include a chemical structure of Formula 1 in which any hydrogen in the chemical structure is substituted with deuterium. The monoamine compound according to one or more embodiments may include a deuterium as a substituent or include a substituent that is substituted with deuterium. For example, in one or more embodiments, at least one selected from among the above-described first to third substituents may include deuterium, or at least one selected from among L¹ to L³ in Formula 1 may include deuterium. However, this is an example, and embodiments of the present disclosure are not limited thereto.

If (e.g., when) a substituent other than hydrogen or deuterium is bonded to an atom at an ortho position with respect to N in L¹ to L³ in Formula 1, the material stability of the amine compound deteriorates due to steric hindrance in a molecule. In addition, if (e.g., when) a functional layer such as a hole transport layer is formed by depositing an amine compound in which a substituent other than hydrogen or deuterium is bonded to the atom at an ortho position with respect to N in L¹ to L³, the stability of the functional layer deteriorates, thereby causing a decrease in lifespan of the light-emitting element.

In Formula 1, if (e.g., when) Ar¹ is an aryl group that has less than 10 ring-forming carbon atoms (for example, a phenyl group), a compound including such Ar¹ causes a decrease in stability during the driving of the light-emitting element. In addition, if (e.g., when) Ar¹ is an aryl group in which a plurality of phenyl groups is connected by a direct linkage, the hole transporting property of the compound including such Ar¹ deteriorates, and the efficiency of the light-emitting element decreases. The aryl group in which a plurality of phenyl groups is connected by a direct linkage may be represented by Formula AY-1. A case where Ar¹ is an aryl group represented by Formula AY-1 is excluded.

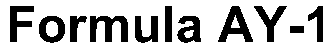

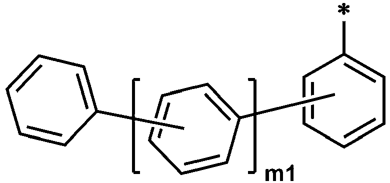

In Formula AY-1, m1 is an integer of 0 or greater. In Formula AY-1, a plurality of phenyl groups and a plurality of phenylene groups may each independently be substituted or unsubstituted. For example, if (e.g., when) m1 is 0 (zero), the aryl group represented by Formula AY-1 may be a substituted or unsubstituted biphenyl group. If (e.g., when) m1 is 1, the aryl group represented by Formula AY-1 may be a substituted or unsubstituted terphenyl group.

In Formula 1, a case where R^{a1} and R^{a5} are each a phenyl group at the same time is excluded. If (e.g., when) R^{a1} and R^{a5} are each a phenyl group at the same time, the material stability of the compound deteriorates due to steric hindrance.

In Formula 1, if (e.g., when) R^{c} is a substituent other than hydrogen, deuterium, or fluorine, the material stability of the compound deteriorates due to steric hindrance.

A compound, in which R^{a2} and R^{a3} are each a phenyl group or R^{a3} and R^{a4} are each a phenyl group, and L¹ is not a direct linkage, causes a deposition temperature to increase during the manufacture of a light-emitting element, thereby lowering the lifespan of the light-emitting element. For example, the compound, in which R^{a2} and R^{a3} are each a phenyl group or R^{a3} and R^{a4} are each a phenyl group, and L¹ is a phenylene group, includes a para-terphenyl moiety, and thus a deposition temperature increases.

In Formula 1, a compound in which at least one selected from among R^{b1} to R^{b6} is not a phenyl group has poor hole transporting property. In a compound in which all R^{b1} to R^{b6} are hydrogens, intermolecular stacking is facilitated, thereby deteriorating hole transport and reducing the lifespan of the light-emitting element.

The monoamine compound, according to one or more embodiments represented by Formula 1, includes no nitrogen-containing heterocyclic group. In other words, the monoamine compound represented by Formula 1, according to one or more embodiments, excludes any nitrogen-containing heterocyclic group. The nitrogen-containing heterocyclic group is a heterocyclic group including a nitrogen atom as a ring-forming atom. For example, the nitrogen-containing heterocyclic group is a pyridine group, a pyrimidine group, a triazine group, a carbazole group, and/or the like. A compound including the nitrogen-containing heterocyclic group loses a charge transporting balance and has low thermal stability and high deposition temperature. When a functional layer such as a hole transport layer is formed by depositing the compound including the nitrogen-containing heterocyclic group, the stability of the functional layer deteriorates, which causes a decrease in lifespan of the light-emitting element. For example, the monoamine compound represented by Formula 1 explicitly excludes heterocyclic groups in which a nitrogen atom is a ring-forming atom. A nitrogen-containing heterocyclic group may include, for example, a pyridine group, pyrimidine group, triazine group, or carbazole group. Compounds containing such nitrogen-based heterocycles may exhibit imbalanced charge transport properties, reduced thermal stability, and elevated deposition temperatures. When such compounds are utilized to form functional layers-such as a hole transport layer-the resulting layer may suffer from poor stability, ultimately leading to a shortened operational lifespan of the light-emitting element.

The monoamine compound represented by Formula 1 according to one or more embodiments includes no cyclic group which includes a carbon atom including *sp³* hybrid orbitals. In other words, the monoamine compound represented by Formula 1 according to one or more embodiments excludes any cyclic group which includes a carbon atom including *sp³* hybrid orbitals. Hereinafter, the 'carbon atom including *sp³* hybrid orbitals' will be referred to as a '*sp³* carbon atom'. The *sp³* carbon atom may be a quaternary carbon atom. The cyclic group including the *sp³* carbon atom has low thermal stability, and if (e.g., when) a functional layer such as a hole transport layer is formed by depositing a compound which includes a cyclic group including the *sp³* carbon atom, the stability of the functional layer deteriorates, thereby causing a decrease in lifespan of the light-emitting element. For example, the monoamine compound represented by Formula 1 excludes any cyclic structure that incorporates an *sp³* carbon atom, such as a quaternary carbon. Cyclic groups (e.g., a fluorene) containing *sp³* carbon atoms tend to exhibit lower thermal stability. When such compounds are utilized to form functional layers-such as a hole transport layer-the resulting layer may degrade more easily under thermal stress, leading to reduced stability and a shorter operational lifespan of the light-emitting element.

In one or more embodiments, the monoamine compound represented by Formula 1 may be represented by any one selected from among Formula 1-A1 to Formula 1-A4. Formula 1-A1 to Formula 1-A4 may represent embodiments in which two selected from among R^{a1} to R^{a5} are each a phenyl group in Formula 1. Formula 1-A4 may represent embodiments in which R^{a2} and R^{a3} (or R^{a3} and R^{a4}) are each a phenyl group, and L¹ is a direct linkage in Formula 1.

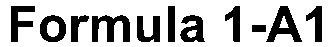

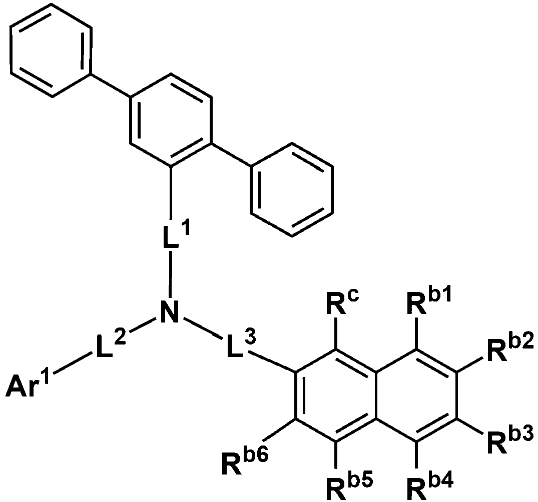

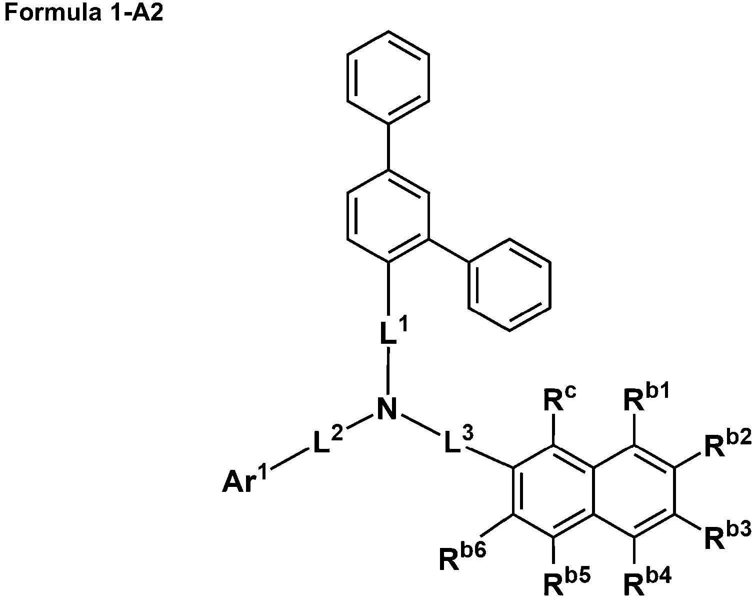

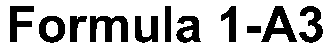

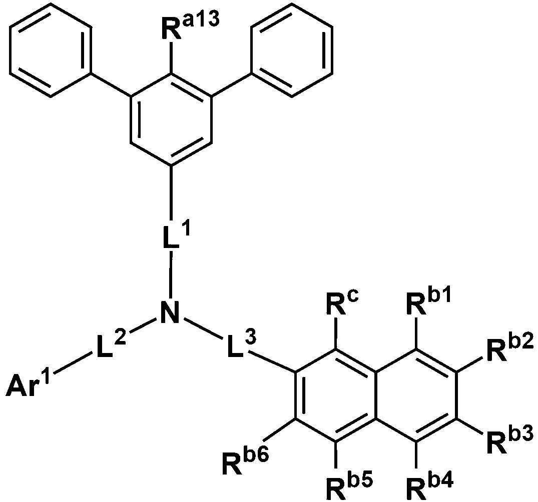

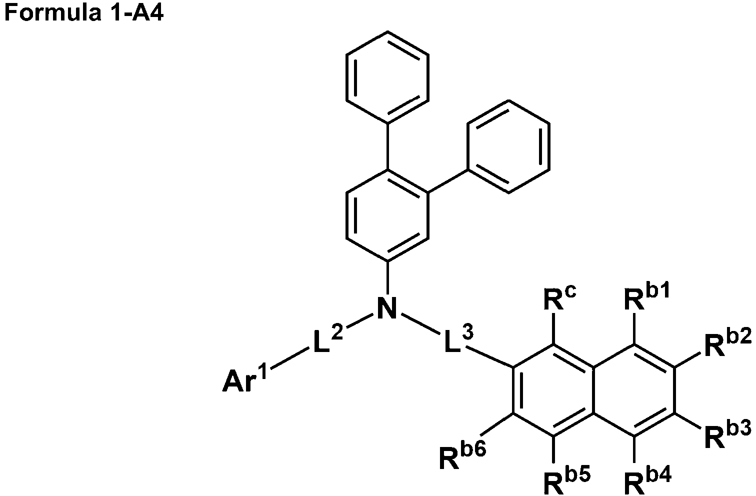

In Formula 1-A3, R^{a13} is hydrogen or fluorine. In Formula 1-A1 to Formula 1-A4, the details described with reference to Formula 1 are similarly applied to L¹ to L³, Ar¹, R^{c}, and R^{b1} to R^{b6}.

For example, in one or more embodiments, in Formula 1-A1 to Formula 1-A4, at least one selected from among L¹ to L³ may be a substituted or unsubstituted phenylene group, and the rest may be a direct linkage. In one or more embodiments, in Formula 1-A1 to Formula 1-A4, at least one selected from among L¹ to L³ may be represented by the above-described L-1 or L-2, and the rest may be direct linkages.

In one or more embodiments, the monoamine compound represented by Formula 1 may be represented by any one selected from among Formula 1-B1 to Formula 1-B6. Formula 1-B1 to Formula 1-B6 may represent embodiments in which any one selected from among R^{b1} to R^{b6} is a phenyl group in Formula 1.

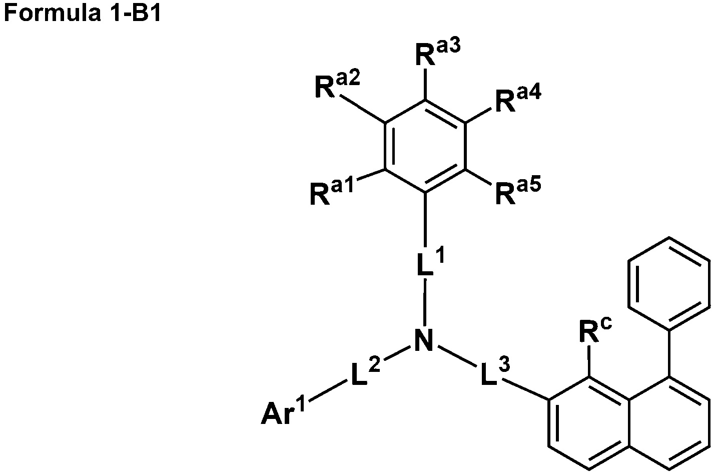

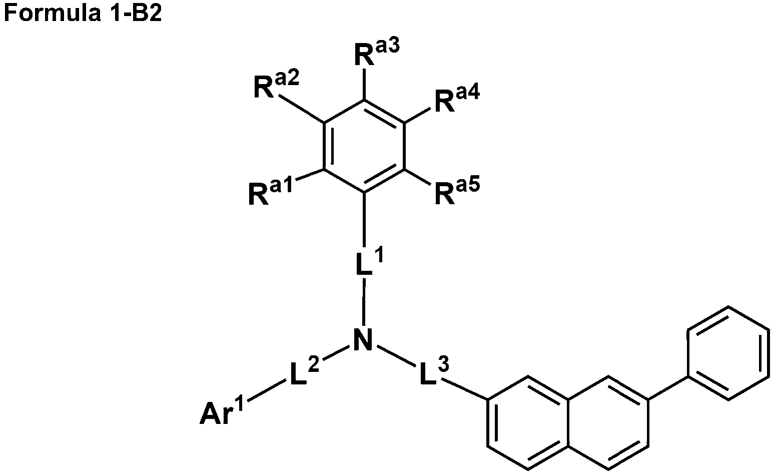

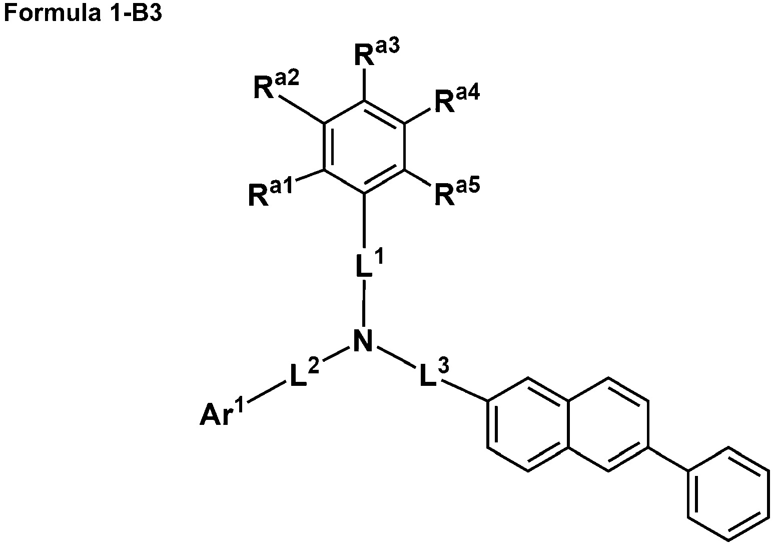

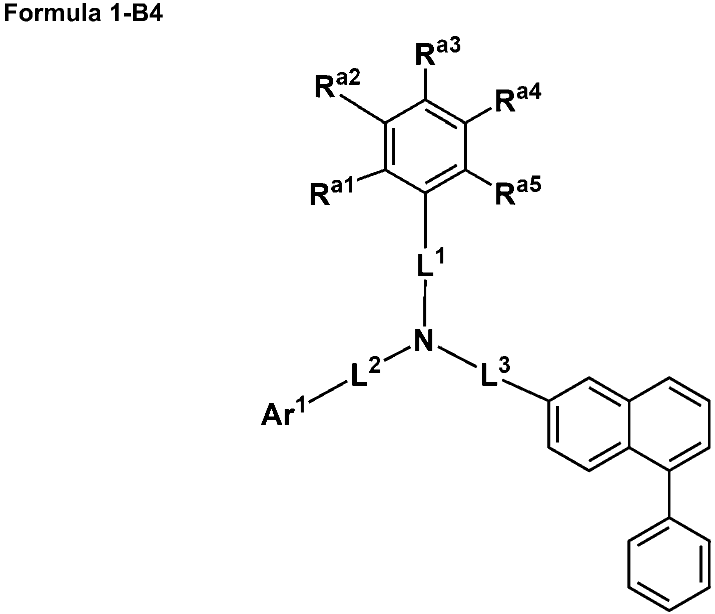

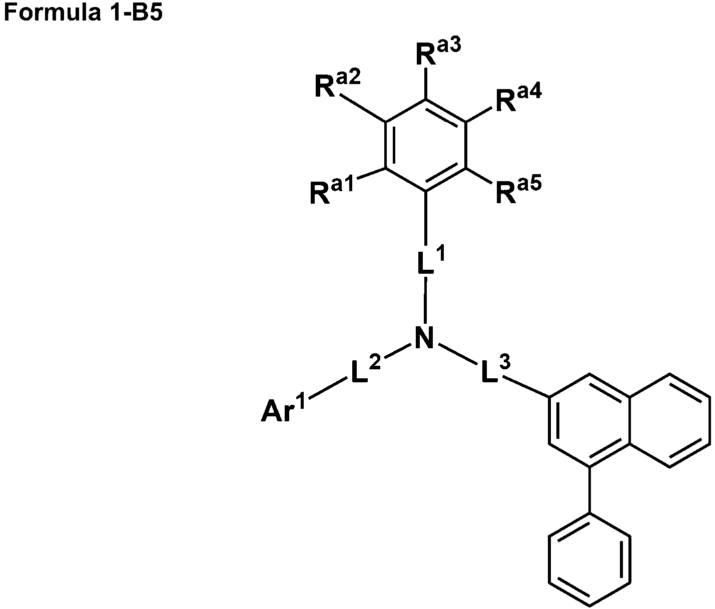

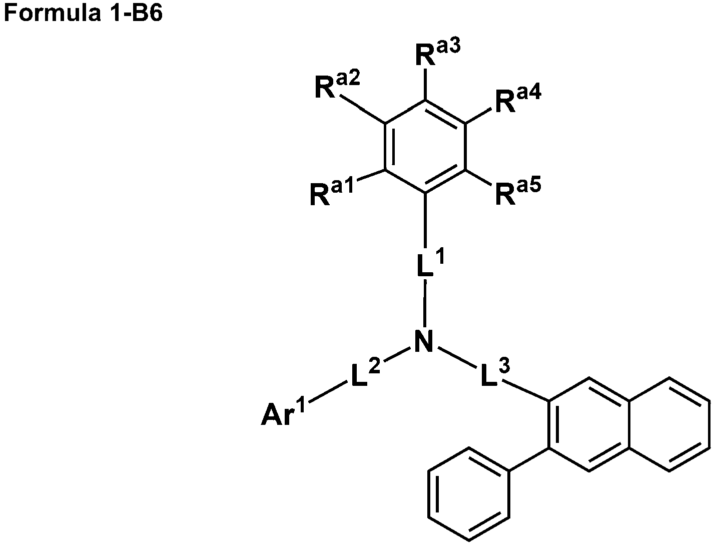

In Formula 1-B1 to Formula 1-B6, the details described with reference to Formula 1 are similarly applied to L¹ to L³, Ar¹, R^{a1} to R^{a5}, and R^{c}.

In one or more embodiments, the monoamine compound represented by Formula 1 may be represented by any one selected from among compounds in Compound Group 1. The monoamine compound according to one or more embodiments may be any one selected from among the compounds in Compound Group 1. The light-emitting element ED according to one or more embodiments may include at least one selected from among the compounds in Compound Group 1. In Compound Group 1, D is deuterium.

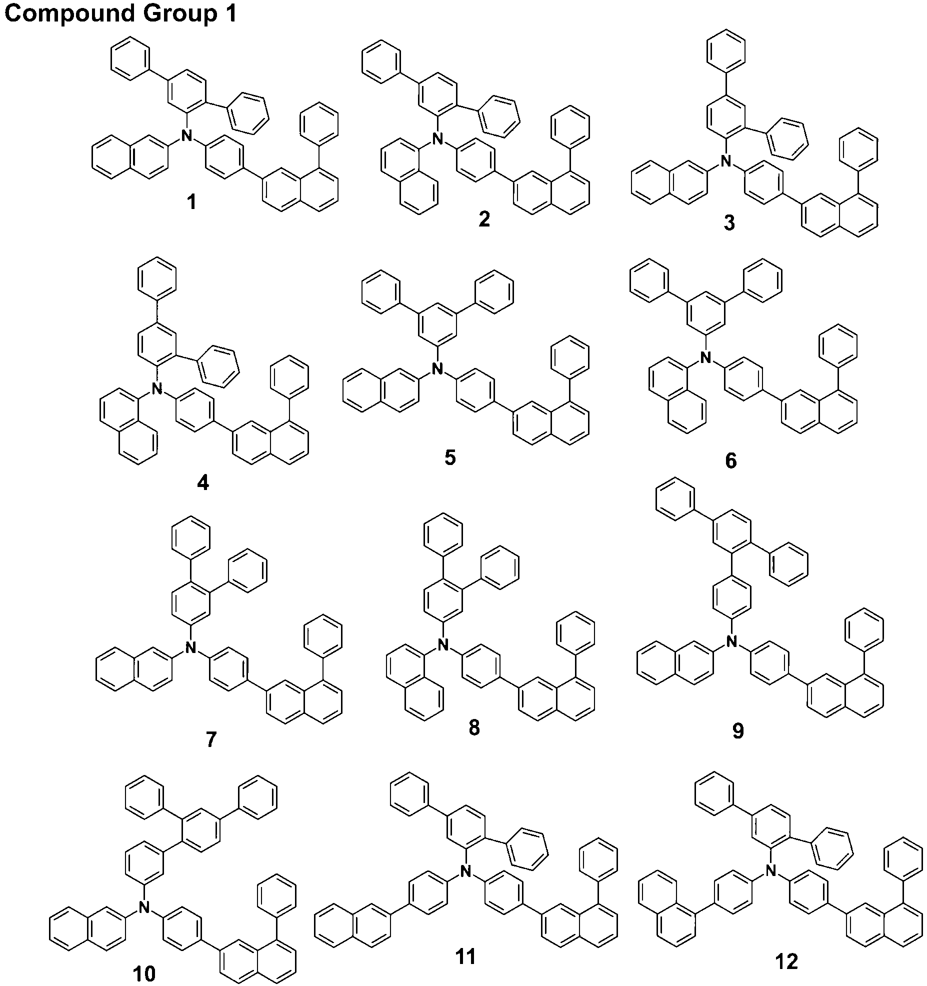

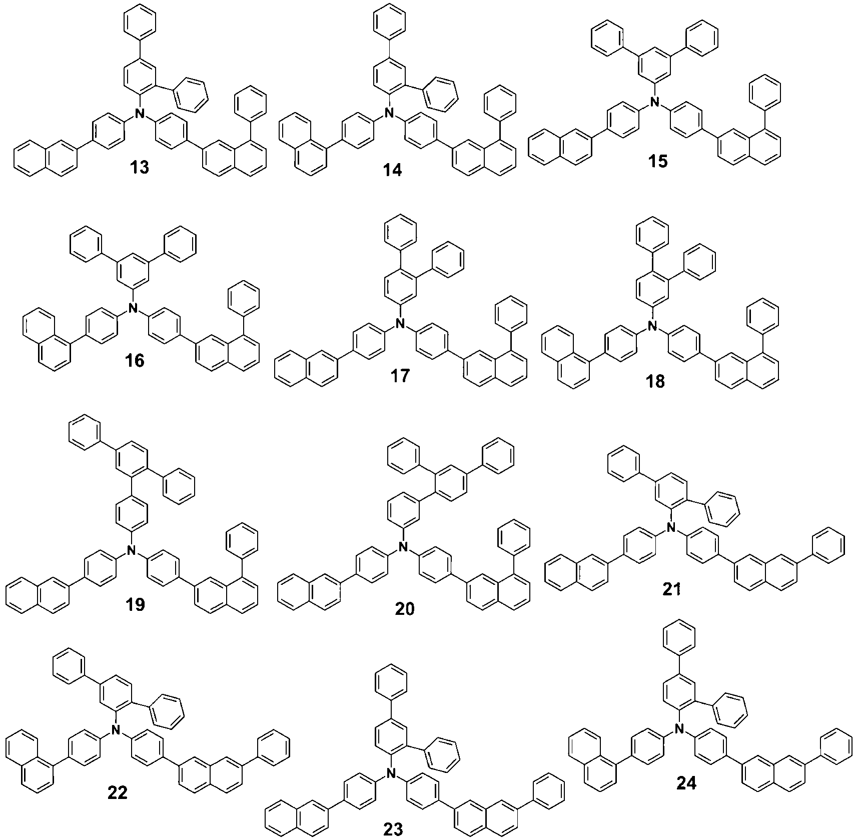

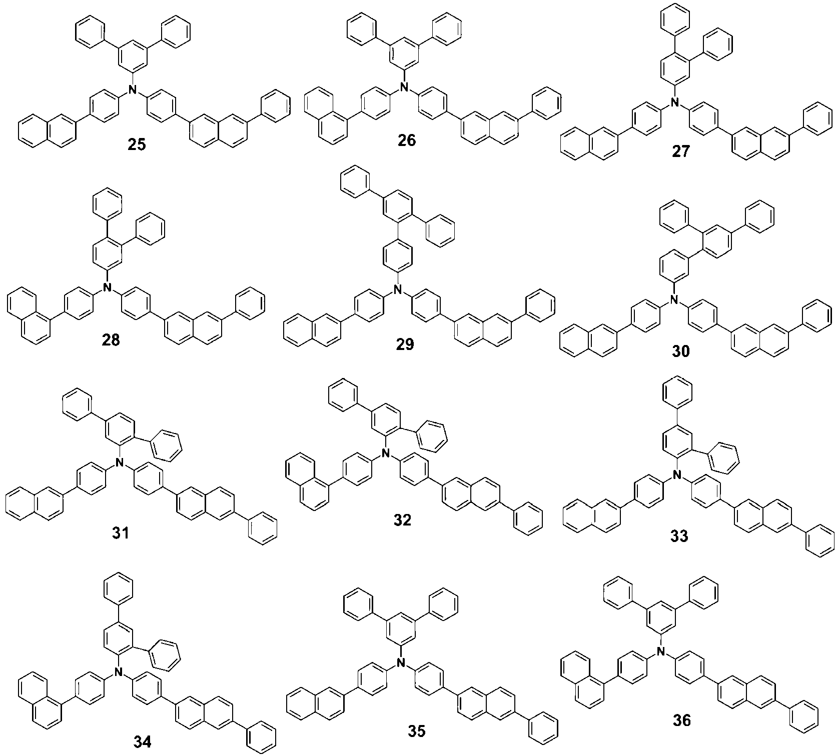

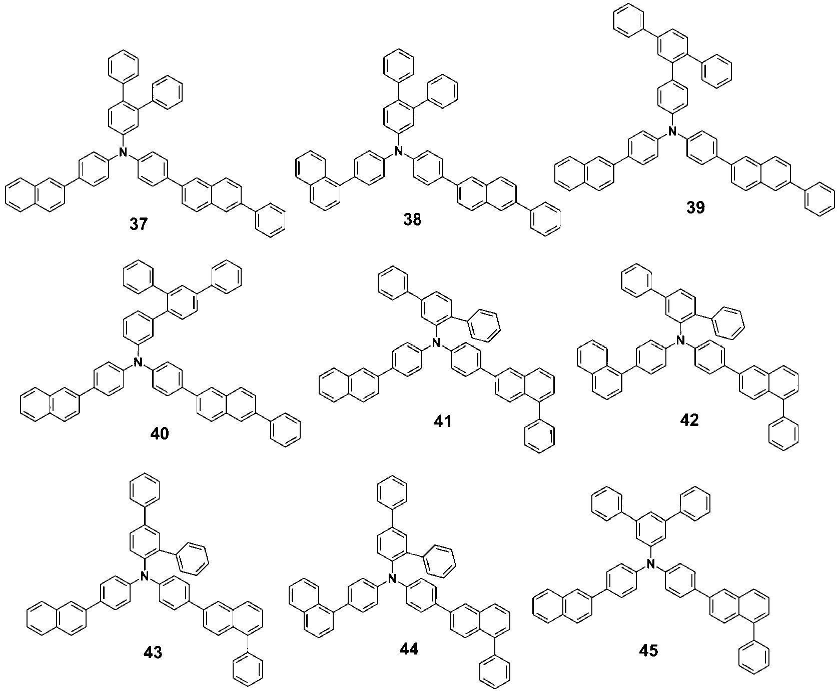

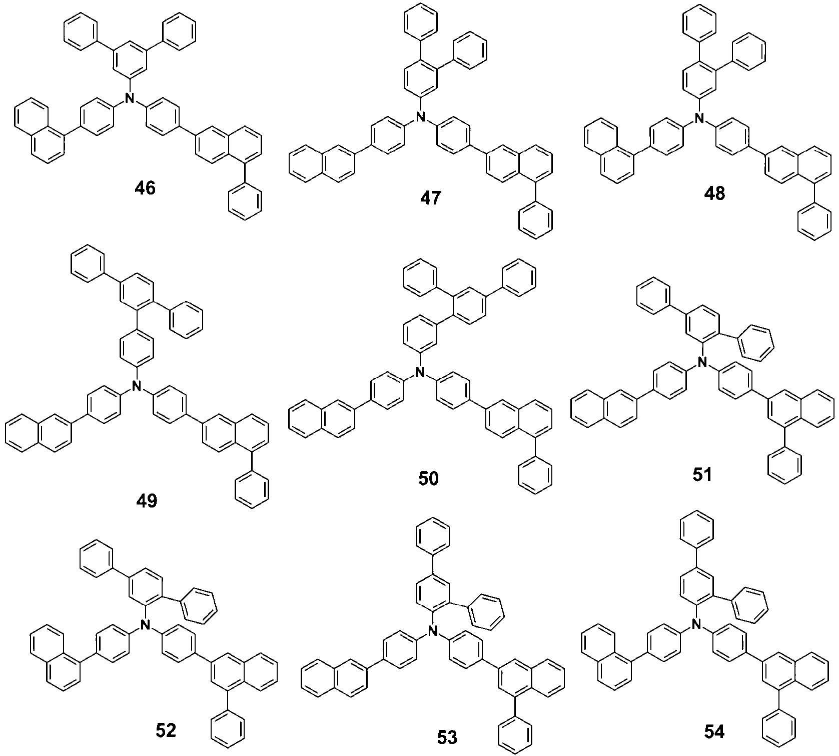

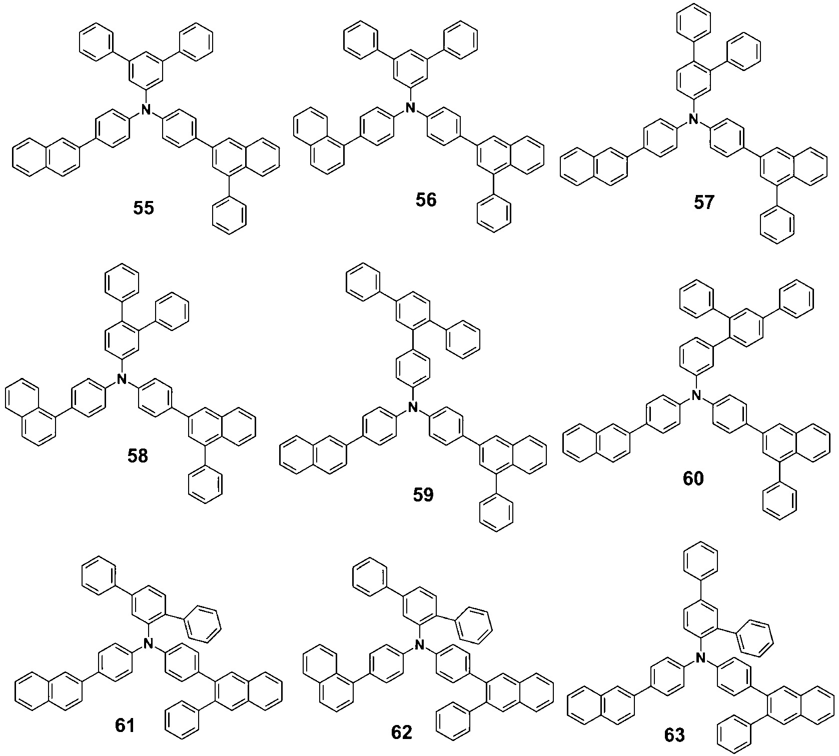

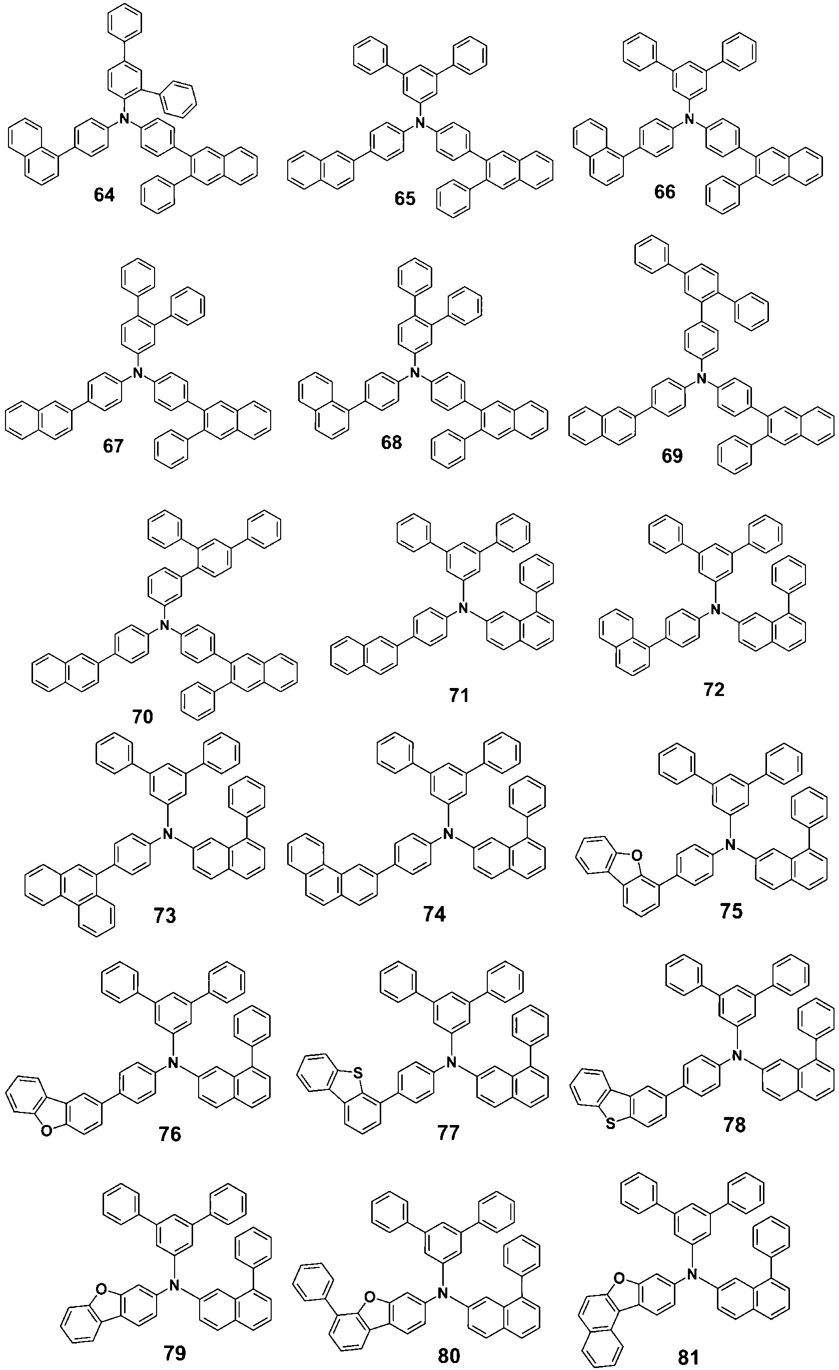

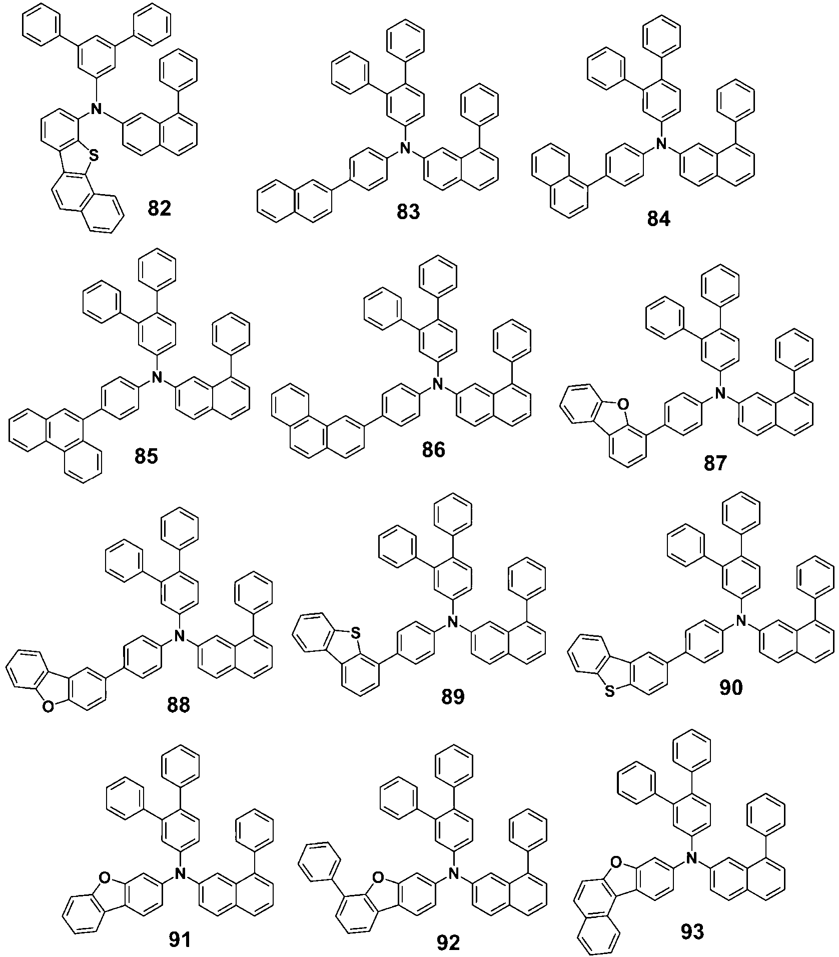

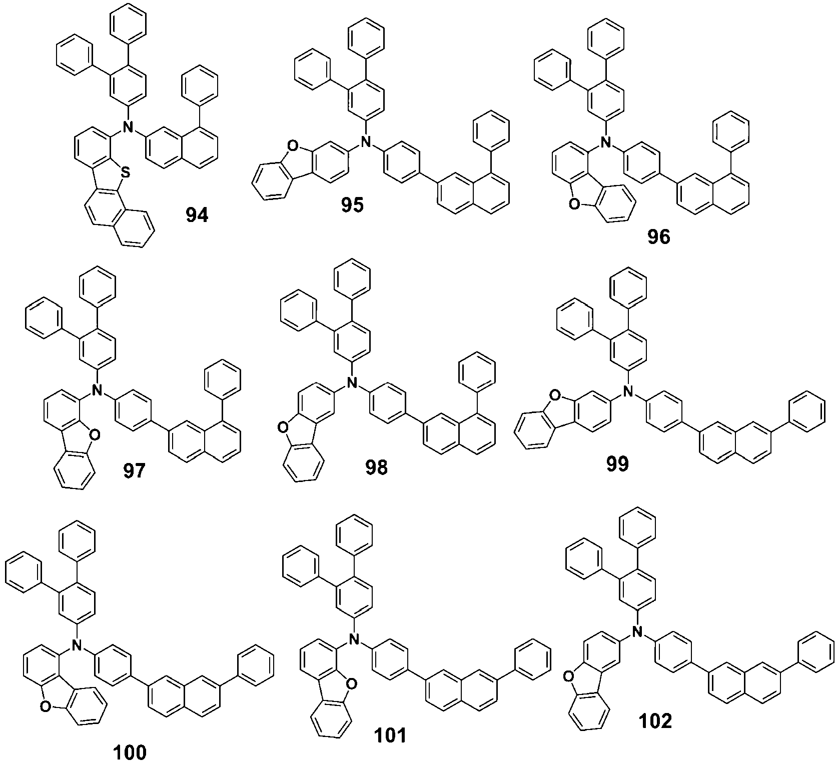

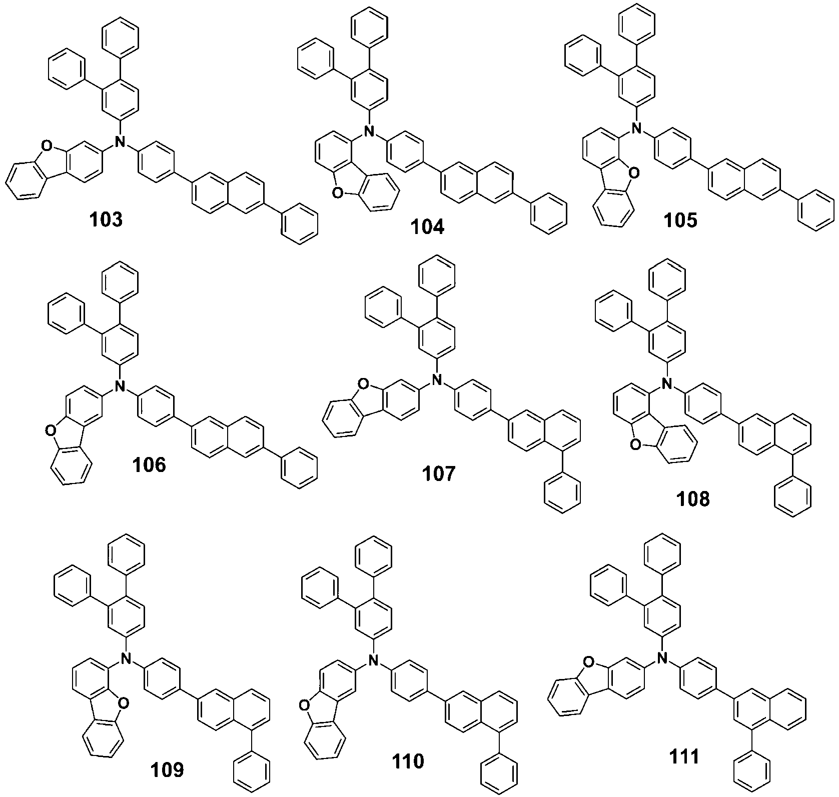

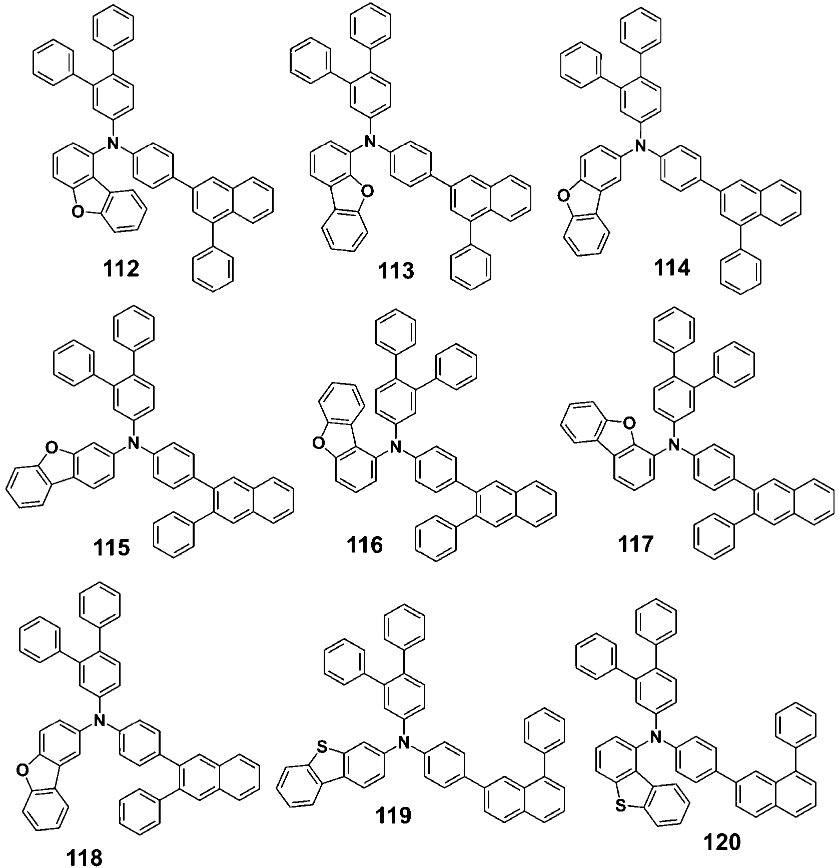

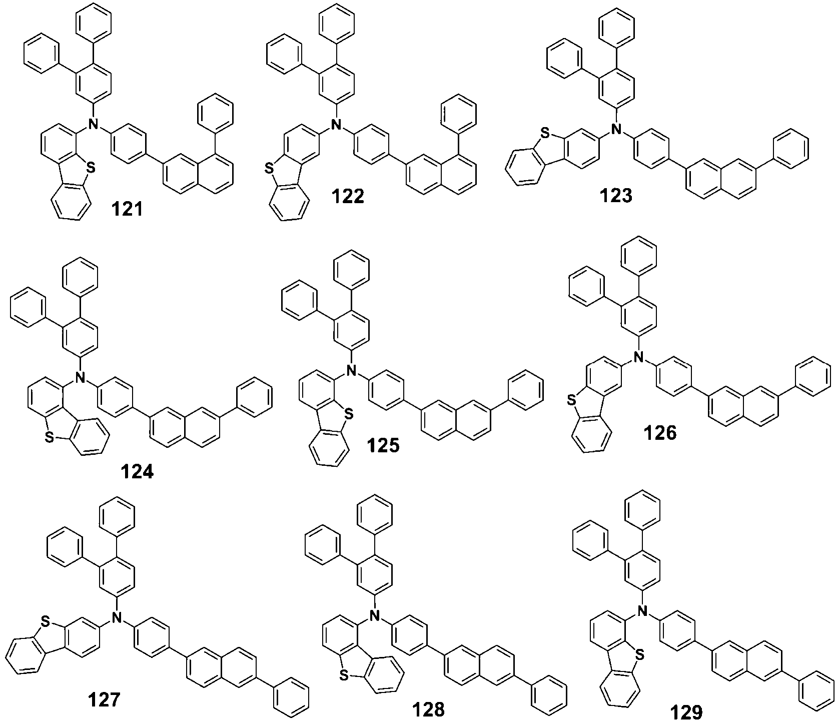

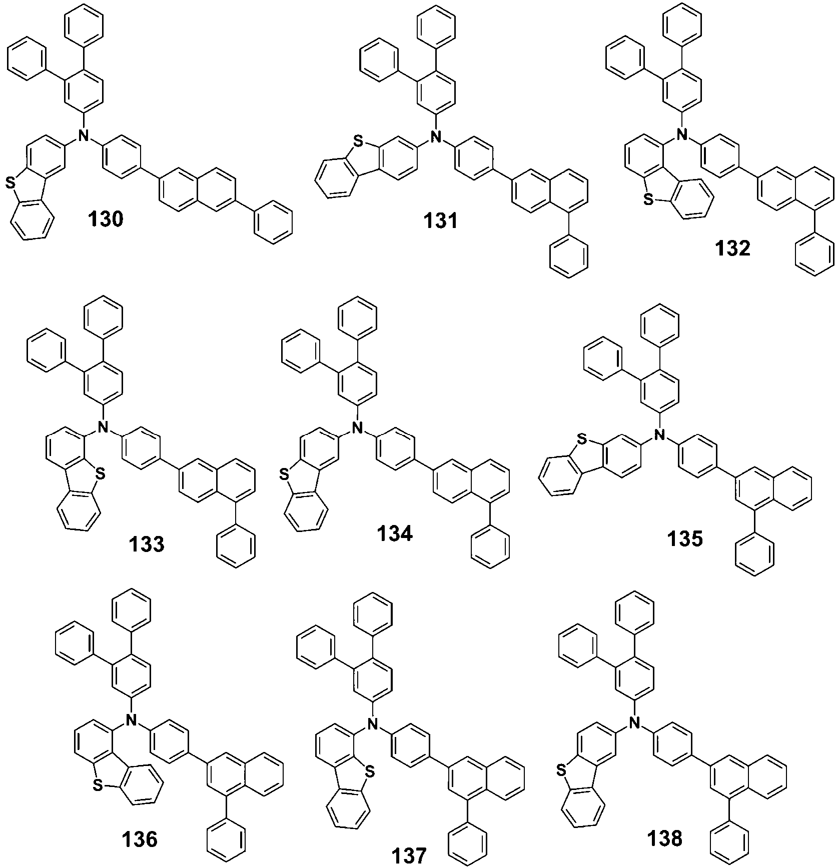

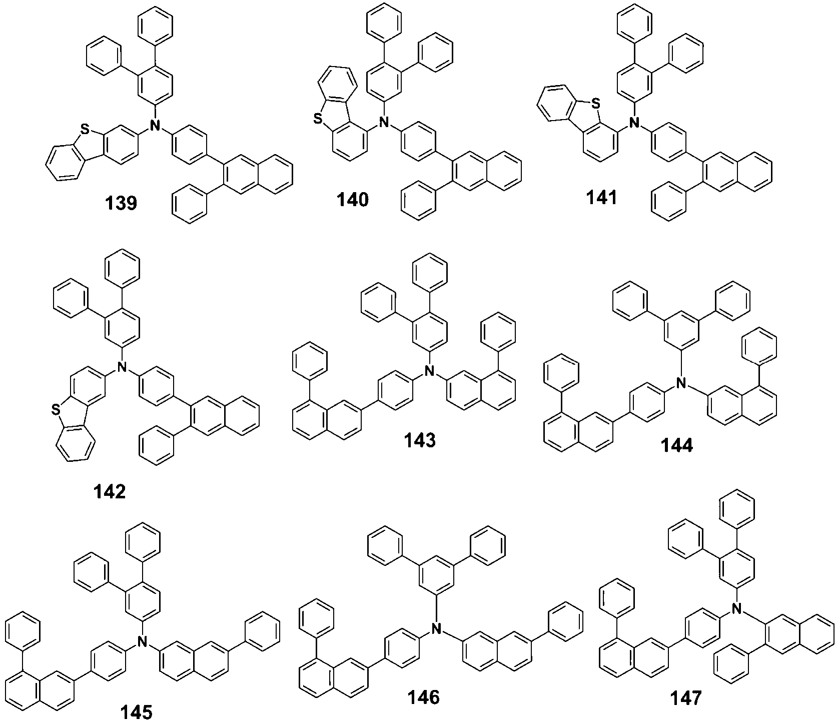

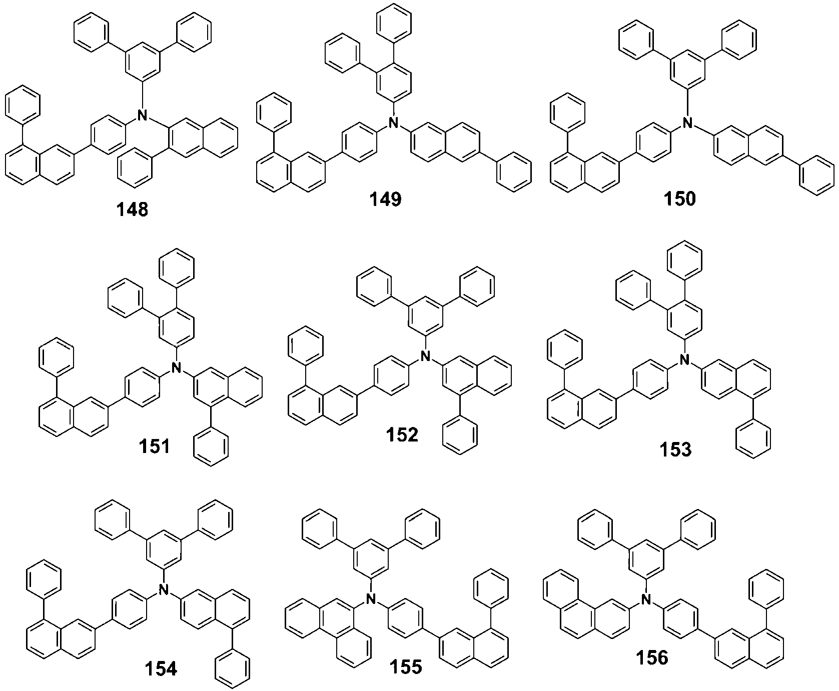

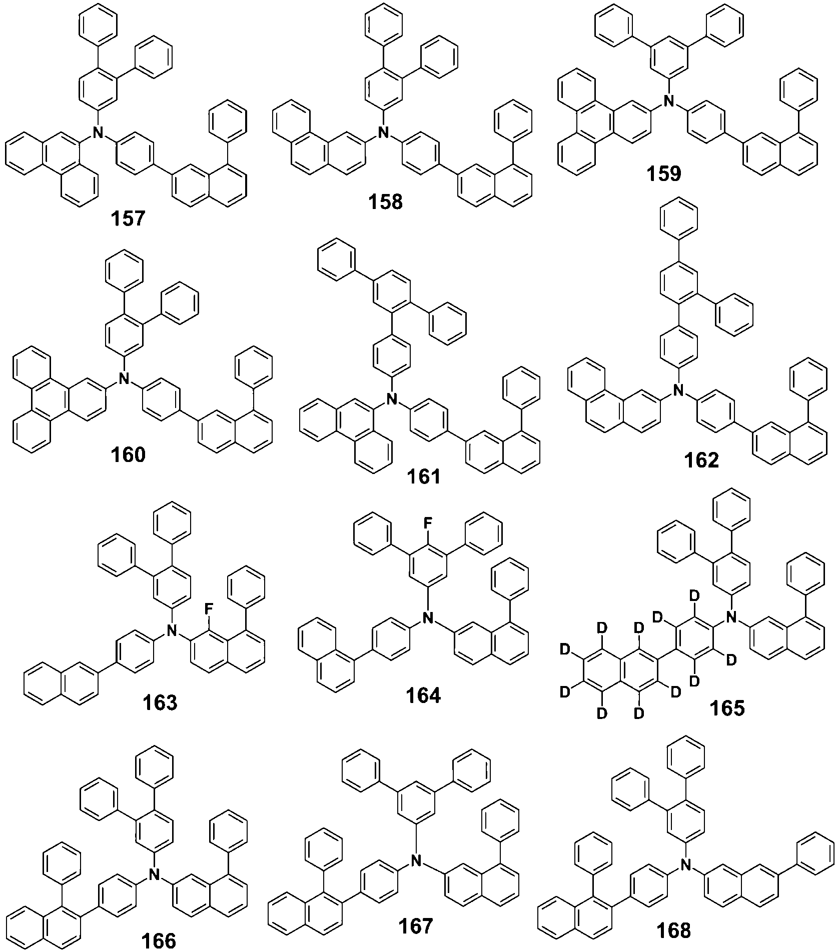

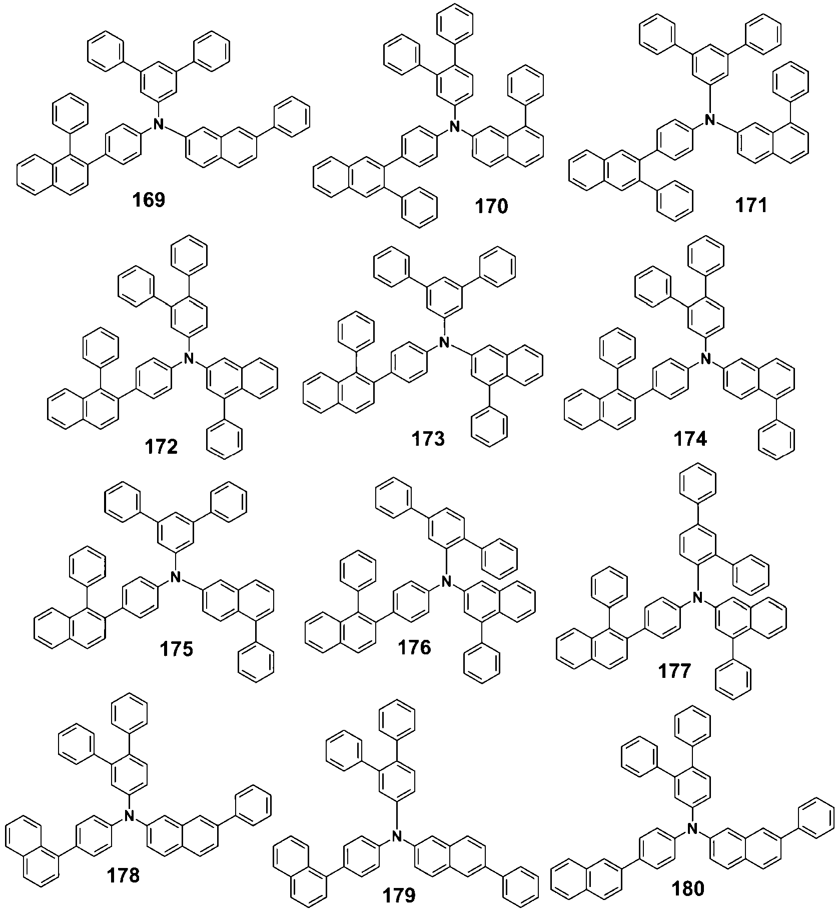

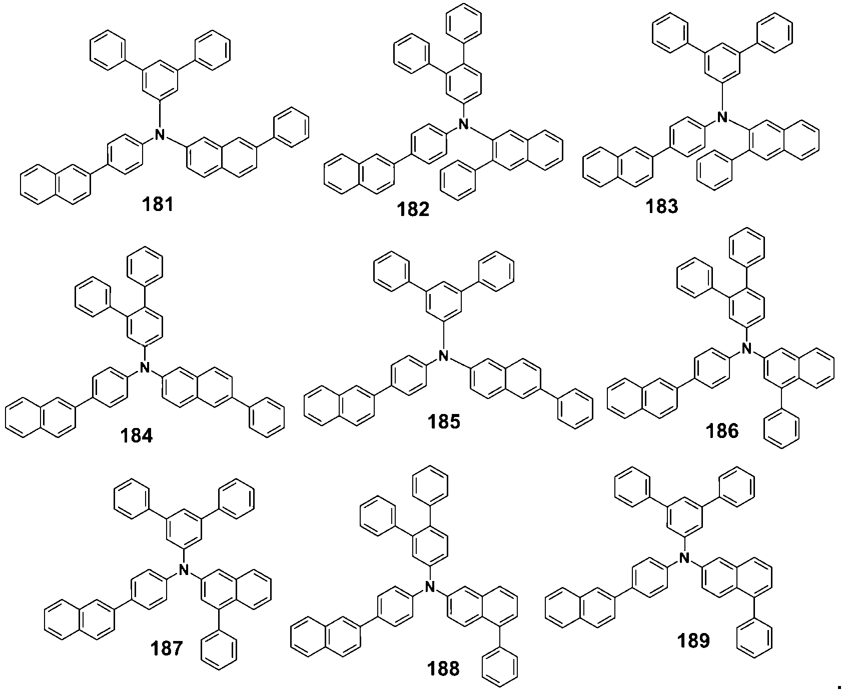

The monoamine compound according to one or more embodiments may include first to third substituents which are directly/indirectly bonded to an amine group. The first substituent may be a diphenylphenyl group (e.g., a terphenyl group), the second substituent may be a phenylnaphthalenyl group, and the third substituent may be an aryl group or a heteroaryl group. In the third substituent, the aryl group may have 10 to 30 ring-forming carbon atoms. In Formula 1, a phenyl group including (e.g., substituted with) R^{a1} to R^{a5} may be a diphenylphenyl group. A naphthalenyl group including (e.g., substituted with) R^{b1} to R^{b6} may be a phenylnaphthalenyl group. Ar¹ may be an aryl group or a heteroaryl group.

The phenylnaphthalenyl group may block intermolecular stacking, and thus a hole transport balance may be improved. The diphenylphenyl group has a branched structure, which increases an intermolecular distance, and thus hole transporting property may be further improved. As a result, the monoamine compound according to one or more embodiments, which includes a diphenylphenyl group and a phenylnaphthalenyl group, may exhibit excellent or suitable material stability and excellent or suitable hole transporting property. The light-emitting element ED including the monoamine compound according to one or more embodiments may exhibit high light-emitting efficiency and long lifespan characteristics.

The hole transport region HTR may include at least one of a hole injection layer HIL, a hole transport layer HTL, a buffer layer, an emission-auxiliary layer, or an electron blocking layer EBL. A thickness of the hole transport region HTR may be, for example, from about 50 Å to about 15,000 Å.

The hole transport region HTR may have a single layer formed of a single material, a single layer formed of a plurality of different materials, or a multilayer structure including a plurality of layers formed of a plurality of different materials.

For example, in one or more embodiments, the hole transport region HTR may have a single layer structure of a hole injection layer HIL or a hole transport layer HTL, or may have a single layer structure formed of a hole injection material and/or a hole transport material. In one or more embodiments, the hole transport region HTR may have a single layer structure formed of a plurality of different materials, or a structure in which a hole injection layer HIL/hole transport layer HTL, a hole injection layer HIL/hole transport layer HTL/buffer layer, a hole injection layer HIL/buffer layer, a hole transport layer HTL/buffer layer, or a hole injection layer HIL/hole transport layer HTL/electron blocking layer EBL are stacked in order from the first electrode EL1, but embodiments of the present disclosure are not limited thereto.

The hole transport region HTR may be formed using one or more suitable methods such as a vacuum deposition method, a spin coating method, a casting method, a Langmuir-Blodgett (LB) method, an inkjet printing method, a laser printing method, and/or a laser induced thermal imaging (LITI) method.

The hole transport region HTR may further include one or more compounds, which will be described later, in addition to the monoamine compound of one or more embodiments.

In one or more embodiments, the hole transport region HTR may include a compound represented by Formula H-1:

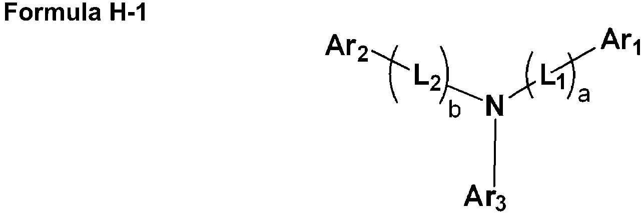

In Formula H-1, L₁ and L₂ may each independently be a direct linkage, a substituted or unsubstituted arylene group having 6 to 30 ring-forming carbon atoms, or a substituted or unsubstituted heteroarylene group having 2 to 30 ring-forming carbon atoms. a and b may each independently be an integer of 0 to 10. If (e.g., when) a or b is an integer of 2 or greater, a plurality of L₁'s and/or a plurality of L₂'s may each independently be a substituted or unsubstituted arylene group having 6 to 30 ring-forming carbon atoms or a substituted or unsubstituted heteroarylene group having 2 to 30 ring-forming carbon atoms.

In Formula H-1, Ar₁ and Ar₂ may each independently be a substituted or unsubstituted aryl group having 6 to 30 ring-forming carbon atoms or a substituted or unsubstituted heteroaryl group having 2 to 30 ring-forming carbon atoms. In addition, in Formula H-1, Ar₃ may be a substituted or unsubstituted aryl group having 6 to 30 ring-forming carbon atoms or a substituted or unsubstituted heteroaryl group having 2 to 30 ring-forming carbon atoms.

In one or more embodiments, the compound represented by Formula H-1 may be a monoamine compound. In one or more embodiments, the compound represented by Formula H-1 may be a diamine compound in which at least one selected from among Ar₁ to Ar₃ includes an amine group as a substituent. In one or more embodiments, the compound represented by Formula H-1 may be a carbazole-based compound including a substituted or unsubstituted carbazole group in at least one of Ar₁ or Ar₂, or a fluorene-based compound including a substituted or unsubstituted fluorene group in at least one of Ar₁ or Ar₂.

The compound represented by Formula H-1 may be any one selected from among compounds in Compound Group H. However, the compounds listed in Compound Group H are mere examples, and the compound represented by Formula H-1 is not limited to those listed in Compound Group H:

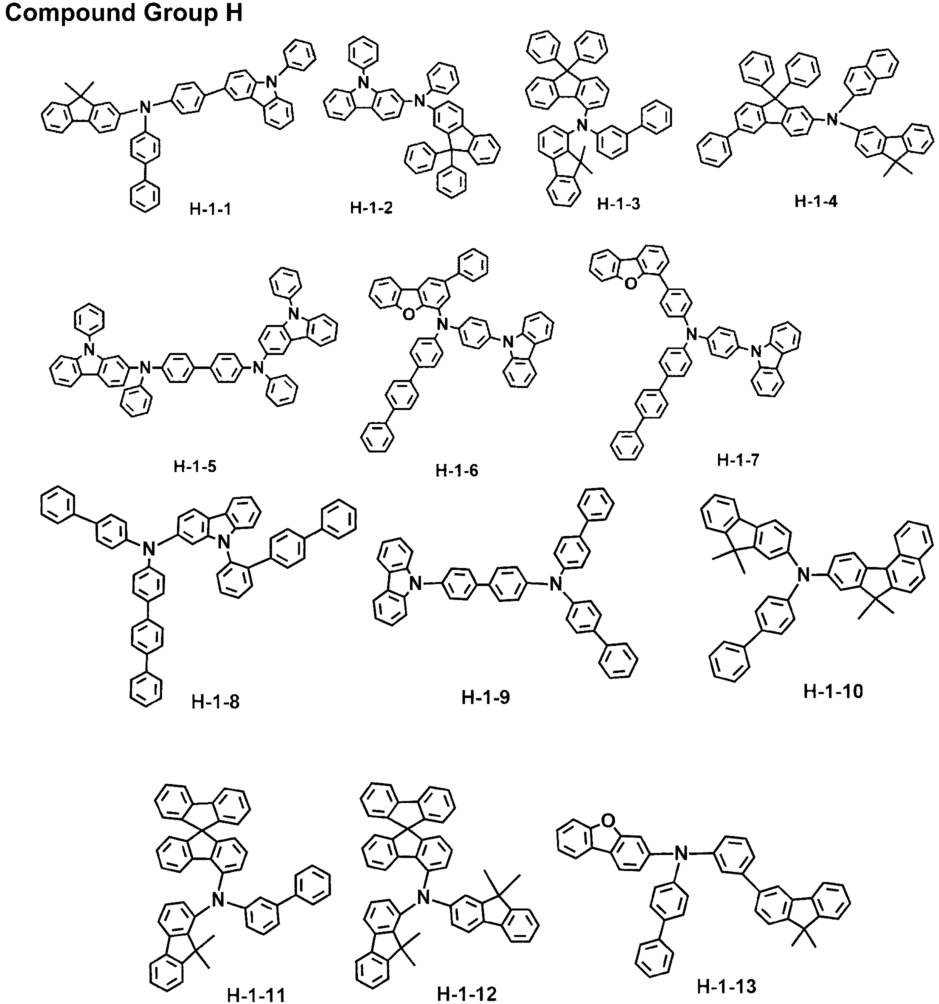

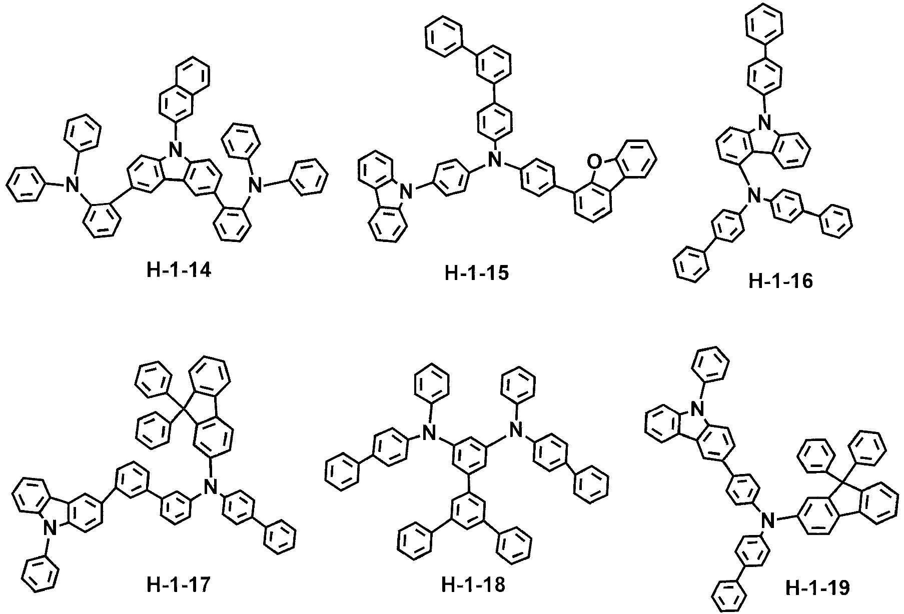

In one or more embodiments, the hole transport region HTR may include one or more selected from among a phthalocyanine compound such as copper phthalocyanine; N¹,N¹'-([1,1'-biphenyl]-4,4'-diyl)bis(N¹-phenyl-N⁴,N⁴-di-m-tolylbenzene-1,4-diamine) (DNTPD), 4,4',4"-[tris(3-methylphenyl)phenylamino] triphenylamine (m-MTDATA), 4,4',4"-tris(N,N-diphenylamino)triphenylamine (TDATA), 4,4',4"-tris[N(2-naphthyl)-N-phenylamino]-triphenylamine (2-TNATA), poly(3,4-ethylenedioxythiophene)/poly(4-styrenesulfonate) (PEDOT/PSS), polyaniline/dodecylbenzenesulfonic acid (PANI/DBSA), polyaniline/camphor sulfonic acid (PANI/CSA), polyaniline/poly(4-styrenesulfonate) (PANI/PSS), N,N'-di(naphthalen-1-yl)-N,N'-diphenyl-benzidine (NPB), triphenylamine-containing polyetherketone (TPAPEK), 4-isopropyl-4'-methyldiphenyliodonium [tetrakis(pentafluorophenyl)borate], dipyrazino[2,3-f: 2',3'-h]quinoxaline-2,3,6,7,10,11-hexacarbonitrile (HATCN), and/or the like.

In one or more embodiments, the hole transport region HTR may include one or more selected from among carbazole-based derivatives such as N-phenyl carbazole and/or polyvinyl carbazole, fluorene-based derivatives, triphenylamine-based derivatives such as N,N'-bis(3-methylphenyl)-N,N'-diphenyl-[1,1-biphenyl]-4,4'-diamine (TPD) and/or 4,4',4"-tris(N-carbazolyl)triphenylamine (TCTA), N,N'-di(naphthalen-1-yl)-N,N'-diphenyl-benzidine (NPB), 4,4'-cyclohexylidene bis[N,N-bis(4-methylphenyl]benzenamine] (TAPC), 4,4'-bis[N,N'-(3-tolyl)amino]-3,3'-dimethylbiphenyl (HMTPD), 1,3-bis(N-carbazolyl)benzene (mCP), and/or the like.

In one or more embodiments, the hole transport region HTR may include one or more selected from among 9-(4-tert-butylphenyl)-3,6-bis(triphenylsilyl)-9H-carbazole (CzSi), 9-phenyl-9H-3,9'-bicarbazole (CCP), 1,3-bis(1,8-dimethyl-9H-carbazol-9-yl)benzene (mDCP), and/or the like.

The hole transport region HTR may include one or more of the above-described compounds of the hole transport region in at least one of a hole injection layer HIL, a hole transport layer HTL, or an electron blocking layer EBL.

The thickness of the hole transport region HTR may be from about 100 Å to about 10,000 Å, for example, from about 100 Å to about 5,000 Å. If (e.g., when) the hole transport region HTR includes a hole injection layer HIL, the hole injection layer HIL may have, for example, a thickness of about 30 Å to about 1,000 Å. If (e.g., when) the hole transport region HTR includes a hole transport layer HTL, the hole transport layer HTL may have a thickness of about 30 Å to about 1,000 Å. For example, if (e.g., when) the hole transport region HTR includes an electron blocking layer EBL, the electron blocking layer EBL may have a thickness of about 10 Å to about 1,000 Å. If (e.g., when) the thicknesses of the hole transport region HTR, the hole injection layer HIL, the hole transport layer HTL, and the electron blocking layer EBL satisfy the above-described ranges, satisfactory hole transport properties may be achieved without a substantial increase in driving voltage.

In one or more embodiments, the hole transport region HTR may further include a charge generating material to increase conductivity in addition to one or more of the above-described materials. The charge generating material may be dispersed uniformly (e.g., substantially uniformly) or non-uniformly in the hole transport region HTR. The charge generating material may be, for example, a p-dopant. The p-dopant may include at least one of a halogenated metal compound (e.g., a metal halide), a quinone derivative, a metal oxide, or a cyano group-containing compound, but embodiments of the present disclosure are not limited thereto. For example, in one or more embodiments, the p-dopant may include one or more selected from among metal halide compounds such as Cul and/or Rbl, quinone derivatives such as tetracyanoquinodimethane (TCNQ) and/or 2,3,5,6-tetrafluoro-7,7'8,8-tetracyanoquinodimethane (F4-TCNQ), metal oxides such as tungsten oxide and/or molybdenum oxide, cyano group-containing compounds such as dipyrazino[2,3-f: 2',3'-h] quinoxaline-2,3,6,7,10,11-hexacarbonitrile (HATCN) and/or 4-[[2,3-bis[cyano-(4-cyano-2,3,5,6-tetrafluorophenyl)methylidene]cyclopropylidene]-cyanomethyl]-2,3,5,6-tetrafluorobenzonitrile (NDP9), and/or the like, but embodiments of the present disclosure are not limited thereto.

As described above, the hole transport region HTR may further include at least one of a buffer layer or an electron blocking layer EBL in addition to the hole injection layer HIL and the hole transport layer HTL. The buffer layer may compensate for a resonance distance according to the wavelength of light emitted from the emission layer EML and may thus increase light emission efficiency. A material that may be included in the hole transport region HTR may be used as a material to be included in the buffer layer. The electron blocking layer EBL is a layer that serves to prevent or reduce the electron injection from the electron transport region ETR to the hole transport region HTR.

The emission layer EML may be provided on the hole transport region HTR. The emission layer EML may have a thickness of, for example, about 100 Å to about 1,000 Å or about 100 Å to about 300 Å. The emission layer EML may have a single layer formed of a single material, a single layer formed of a plurality of different materials, or a multilayer structure having a plurality of layers formed of a plurality of different materials.

In the light-emitting element ED of one or more embodiments, the emission layer EML may include at least one of an anthracene derivative, a pyrene derivative, a fluoranthene derivative, a chrysene derivative, a dihydrobenzanthracene derivative, or a triphenylene derivative. For example, in one or more embodiments, the emission layer EML may include an anthracene derivative or a pyrene derivative.

In each light-emitting element ED of embodiments illustrated in FIGS. 3 to 6, the emission layer EML may further include a suitable host and dopant besides the above-described host and dopant. For example, in one or more embodiments, the emission layer EML may include a compound represented by Formula E-1. The compound represented by Formula E-1 may be used as a fluorescent host material.

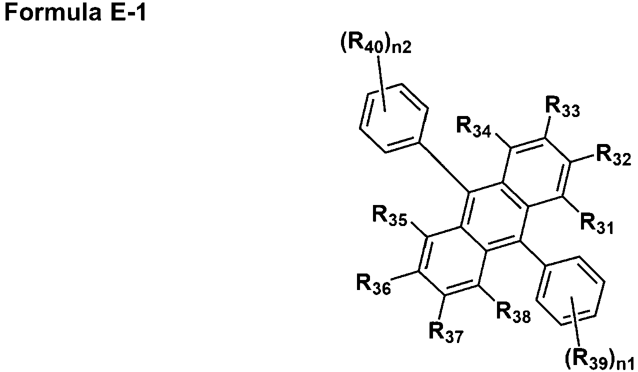

In Formula E-1, n1 and n2 are each independently an integer of 0 to 5. When n1 is an integer of 2 or greater, a plurality of R₃₉'s may all be the same or at least one thereof may be different from the others. When n1 is 0, the embodiment may be the same as an embodiment in which n1 is 5 and five R₃₉'s are all hydrogens. When n2 is an integer of 2 or greater, a plurality of R₄₀'s may all be the same or at least one thereof may be different from the others. When n2 is 0, the embodiment may be the same as an embodiment in which n2 is 5 and five R₄₀'s are all hydrogens.

In Formula E-1, R₃₁ to R₄₀ are each independently hydrogen, deuterium, a halogen, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms, and/or bonded to an adjacent group to form a ring. One or more selected from among R₃₁ to R₄₀ may be each independently bonded to an adjacent group to form a saturated hydrocarbon ring, an unsaturated hydrocarbon ring, a saturated heterocycle, or an unsaturated heterocycle.

The compound represented by Formula E-1 may be any one selected from among Compound E1 to Compound E21:

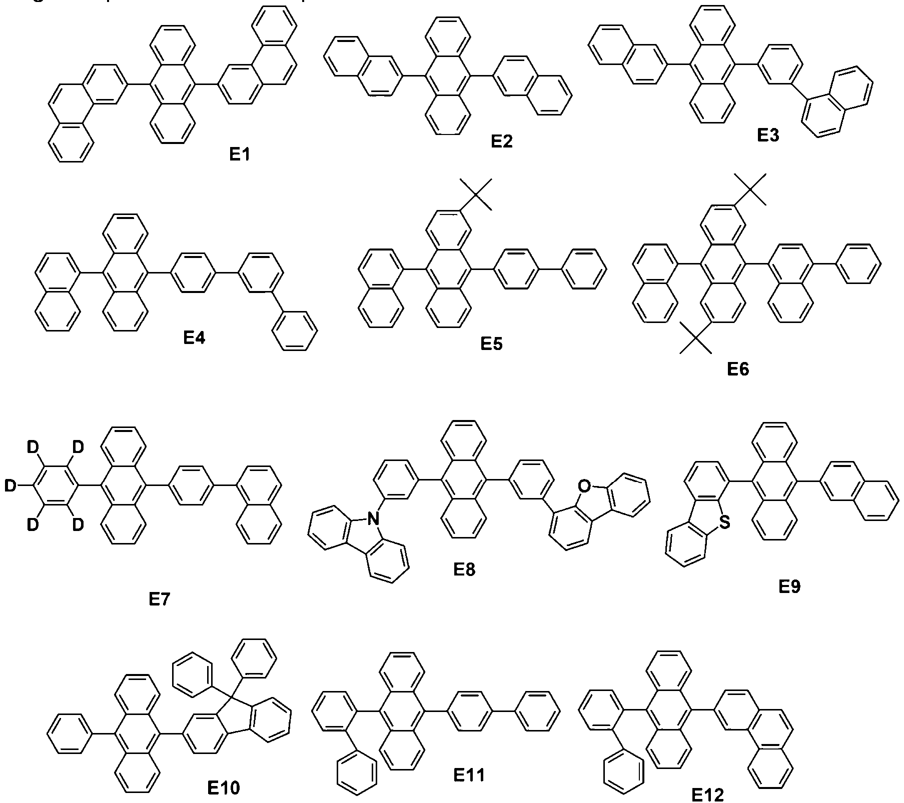

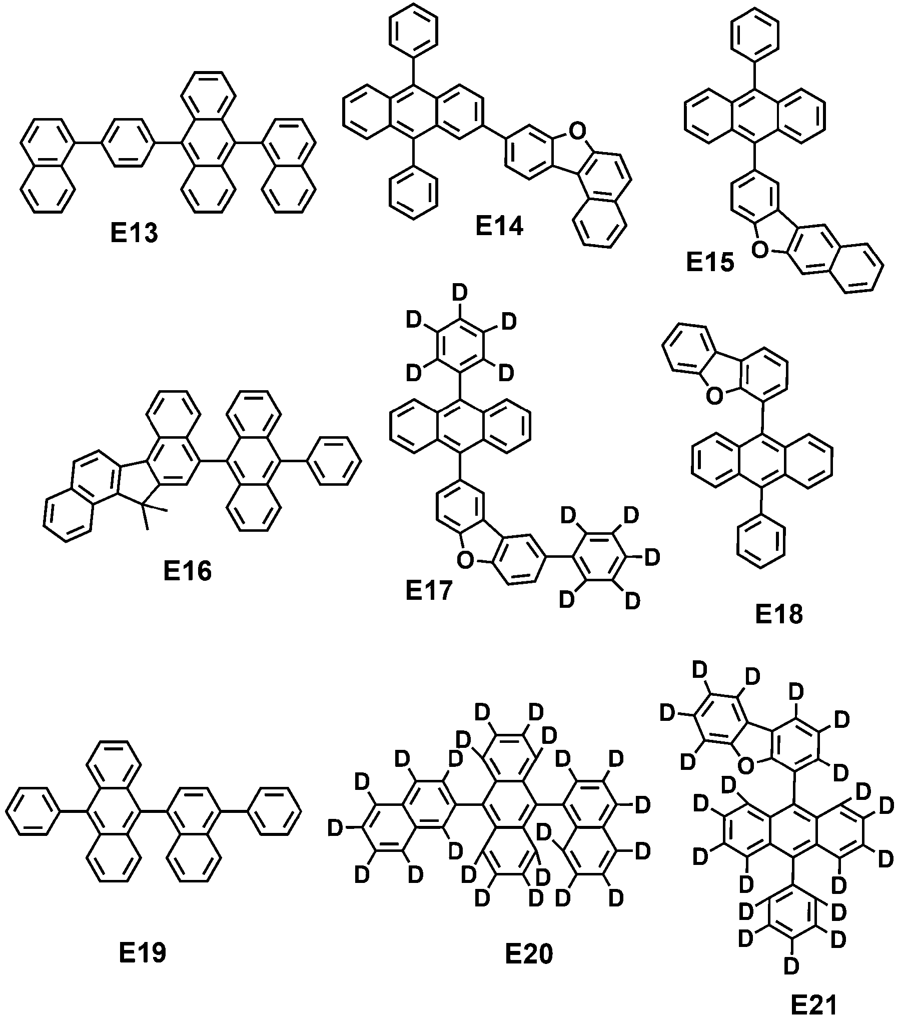

In one or more embodiments, the emission layer EML may include a compound represented by Formula E-2a or Formula E-2b. The compound represented by Formula E-2a or Formula E-2b may be used as a host material of a phosphorescent emission layer.

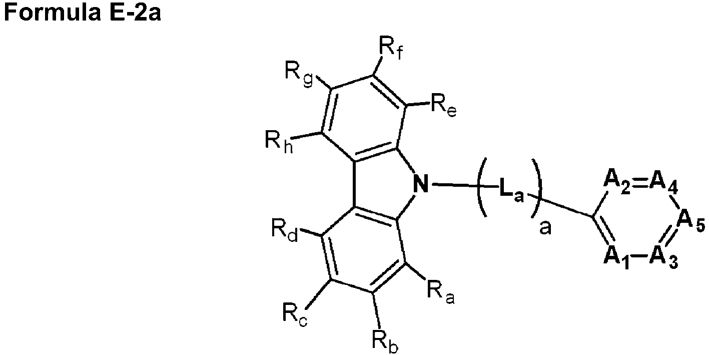

In Formula E-2a, a may be an integer of 0 to 10, and Lₐ may be a direct linkage, a substituted or unsubstituted arylene group having 6 to 30 ring-forming carbon atoms, or a substituted or unsubstituted heteroarylene group having 2 to 30 ring-forming carbon atoms. If (e.g., when) a is an integer of 2 or greater, a plurality of Lₐ's may each independently be a substituted or unsubstituted arylene group having 6 to 30 ring-forming carbon atoms or a substituted or unsubstituted heteroarylene group having 2 to 30 ring-forming carbon atoms.

In addition, in Formula E-2a, A₁ to A₅ may each independently be N or CRᵢ. Rₐ to Rᵢ may each independently be hydrogen, deuterium, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and/or bonded to an adjacent group to form a ring. In one or more embodiments, one or more selected from among Rₐ to Rᵢ may be each independently bonded to an adjacent group to form a hydrocarbon ring or a heterocycle containing N, O, S, and/or the like, as a ring-forming atom.

In one or more embodiments, in Formula E-2a, two or three selected from among A₁ to A₅ may be N, and the rest may be CRᵢ.

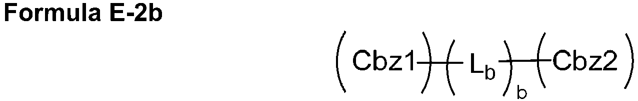

In Formula E-2b, Cbz1 and Cbz2 may each independently be an unsubstituted carbazole group or a carbazole group substituted with an aryl group having 6 to 30 ring-forming carbon atoms. L_{b} may be a direct linkage, a substituted or unsubstituted arylene group having 6 to 30 ring-forming carbon atoms, or a substituted or unsubstituted heteroarylene group having 2 to 30 ring-forming carbon atoms. In Formula E-2b, b is an integer of 0 to 10, and if (e.g., when) b is an integer of 2 or more, a plurality of L_{b}'s may each independently be a substituted or unsubstituted arylene group having 6 to 30 ring-forming carbon atoms or a substituted or unsubstituted heteroarylene group having 2 to 30 ring-forming carbon atoms.

The compound represented by Formula E-2a or Formula E-2b may be any one selected from among compounds of Compound Group E-2. However, the compounds listed in Compound Group E-2 are mere example, and the compound represented by Formula E-2a or Formula E-2b is not limited to those represented in Compound Group E-2.

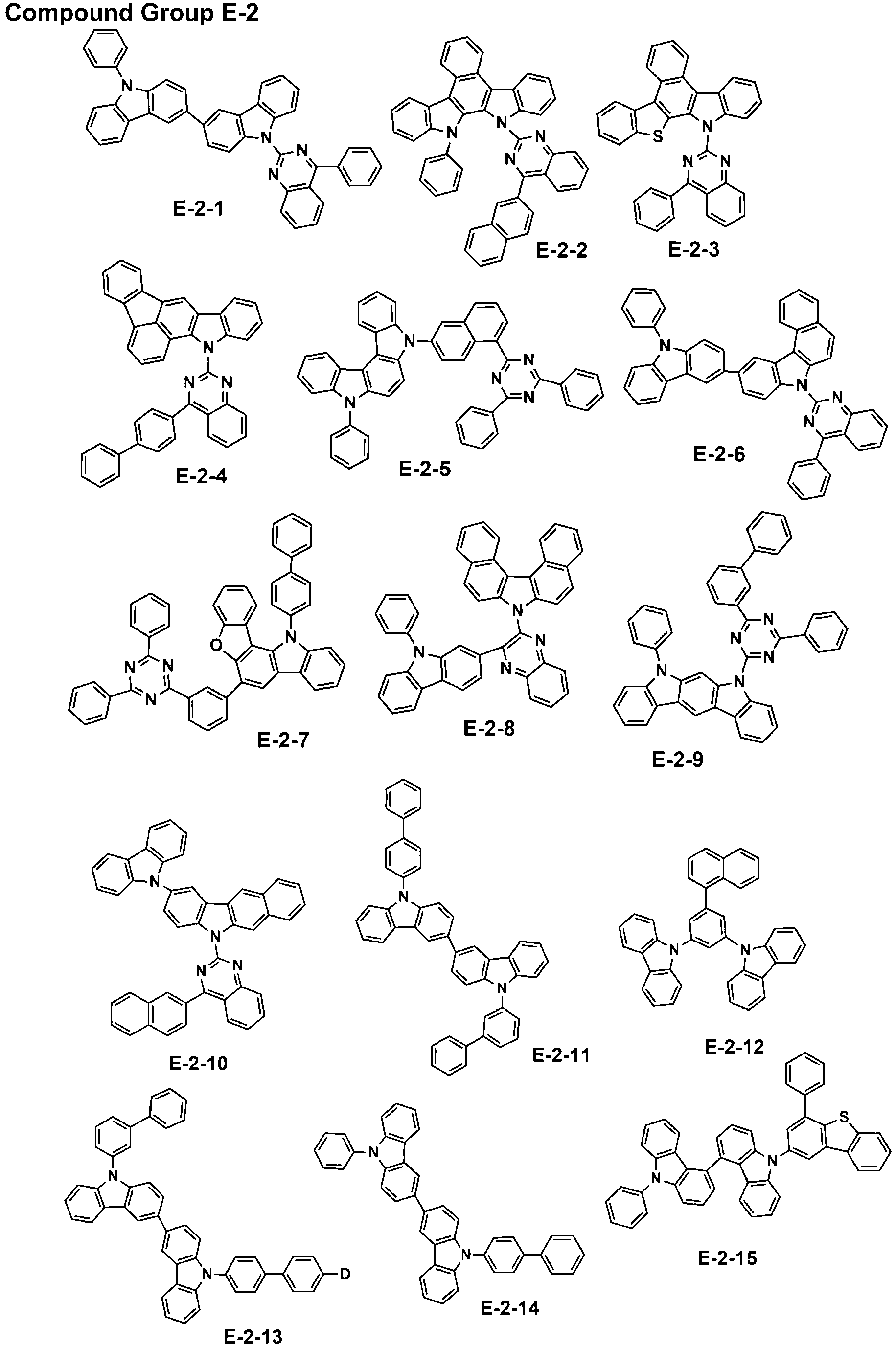

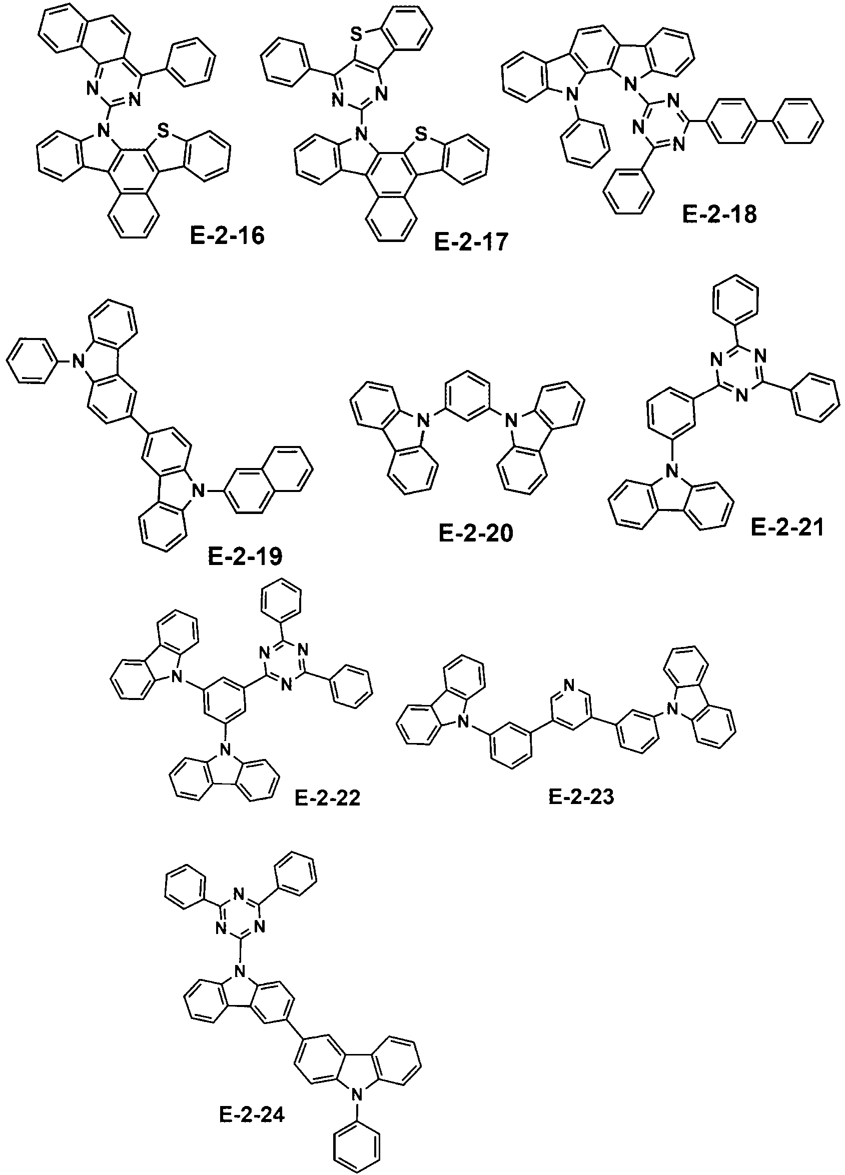

In one or more embodiments, the emission layer EML may further include a general material suitable in the art as a host material. For example, the emission layer EML may include, as a host material, at least one of bis(4-(9H-carbazol-9-yl)phenyl)diphenylsilane (BCPDS), (4-(1-(4-(diphenylamino)phenyl)cyclohexyl)phenyl)diphenyl-phosphine oxide (POPCPA), bis[2-(diphenylphosphino)phenyl]ether oxide (DPEPO), 4,4'-bis(N-carbazolyl)-1,1'-biphenyl (CBP), 1,3-bis(carbazol-9-yl)benzene (mCP), 2,8-bis(diphenylphosphoryl)dibenzo[b,d]furan (PPF), 4,4',4"-tris(carbazol-9-yl)-triphenylamine (TCTA), or 1,3,5-tris(1-phenyl-1H-benzo[d]imidazole-2-yl)benzene (TPBi). However, embodiments of the present disclosure are not limited thereto, for example, tris(8-hydroxyquinolinato)aluminium (Alq₃), 9,10-di(naphthalen-2-yl)anthracene (ADN), 2-tert-butyl-9,10-di(naphth-2-yl)anthracene (TBADN), distyrylarylene (DSA), 4,4'-bis(9-carbazolyl)-2,2'-dimethyl-biphenyl (CDBP), 2-methyl-9,10-bis(naphthalen-2-yl)anthracene (MADN), hexaphenyl cyclotriphosphazene (CP1), 1,4-bis(triphenylsilyl)benzene (UGH2), hexaphenylcyclotrisiloxane (DPSiO₃), octaphenylcyclotetrasiloxane (DPSiO₄), and/or the like may be used as a host material.

In one or more embodiments, the emission layer EML may include a compound represented by Formula M-a or Formula M-b. The compound represented by Formula M-a or Formula M-b may be used as a phosphorescent dopant material.

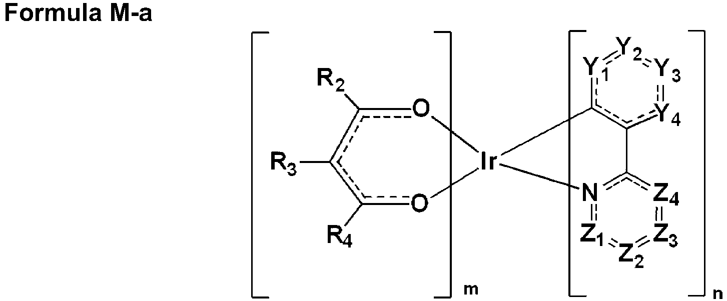

In Formula M-a, Y₁ to Y₄ and Z₁ to Z₄ may each independently be CR₁ or N, R₁ to R₄ may each independently be hydrogen, deuterium, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and/or bonded to an adjacent group to form a ring. In Formula M-a, m is 0 or 1, and n is 2 or 3. In Formula M-a, if (e.g., when) m is 0, n is 3, and if (e.g., when) m is 1, n is 2.

The compound represented by Formula M-a may be used as a phosphorescent dopant.

The compound represented by Formula M-a may be any one selected from among Compound M-a1 to Compound M-a25. However, Compounds M-a1 to M-a25 are mere examples, and the compound represented by Formula M-a is not limited to those represented by Compounds M-a1 to M-a25.

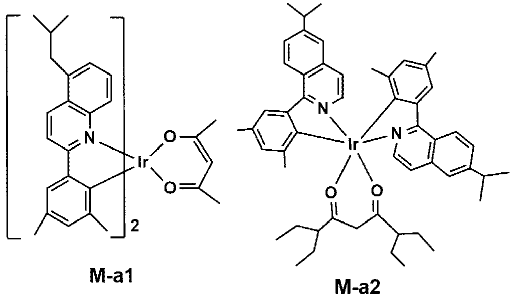

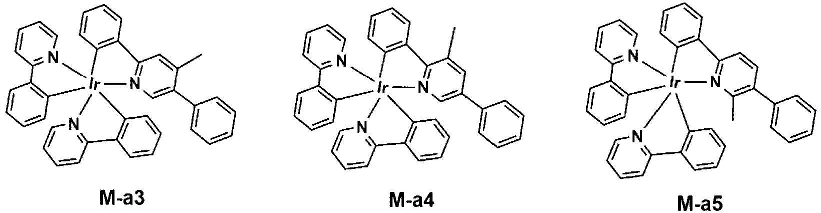

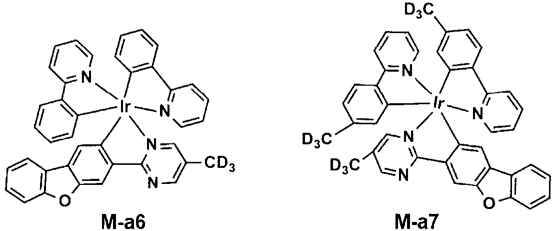

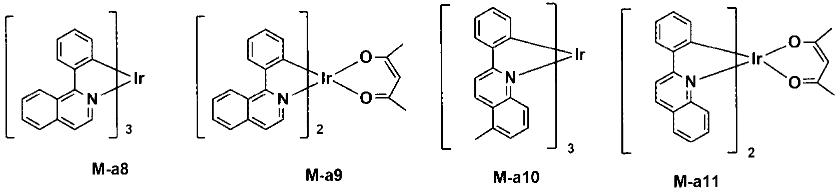

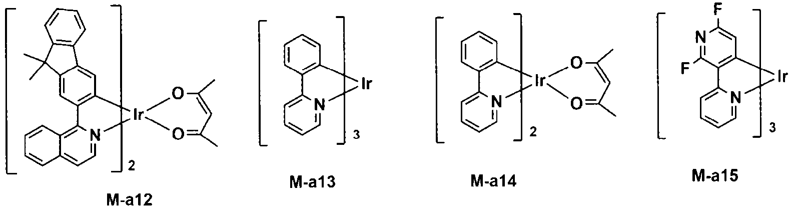

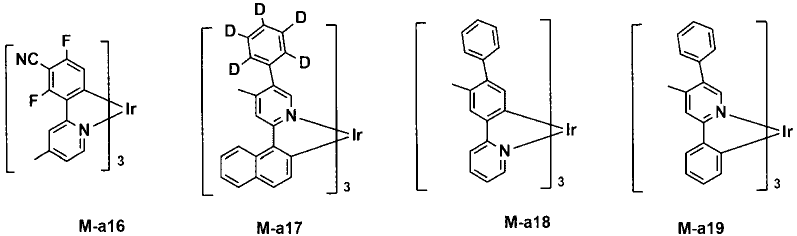

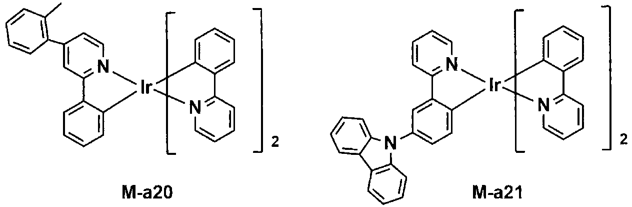

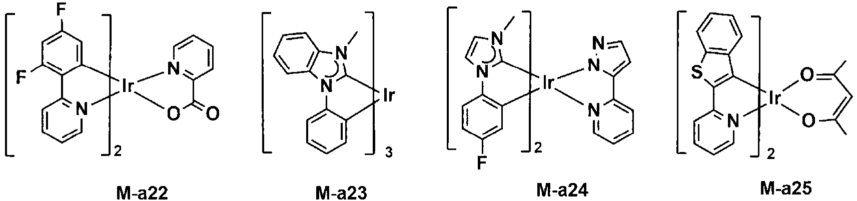

The compounds M-a1 and M-a2 may be used as a red dopant material, and the compounds M-a3 to M-a5 may be used as a green dopant material.

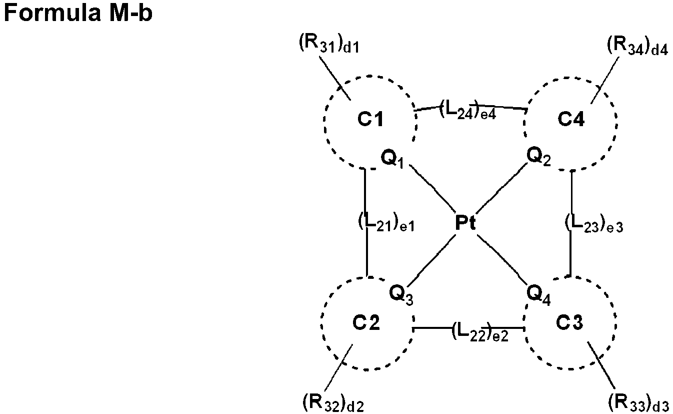

In Formula M-b, Q₁ to Q₄ may each independently be C or N. C1 to C4 may each independently be a substituted or unsubstituted hydrocarbon ring having 5 to 30 ring-forming carbon atoms, a substituted or unsubstituted aryl group having 6 to 30 ring-forming carbon atoms, or a substituted or unsubstituted hetero ring having 2 to 30 ring-forming carbon atoms.

L₂₁ to L₂₄ may each independently be a direct linkage, *-O-*, *-S- *, 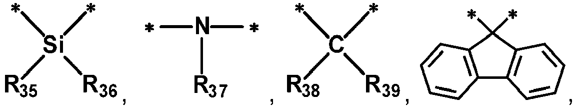 a substituted or unsubstituted alkylene group having 1 to 20 carbons, a substituted or unsubstituted arylene group having 6 to 30 ring-forming carbon atoms, or a substituted or unsubstituted heteroarylene group having 2 to 30 ring-forming carbon atoms. e1 to e4 may each independently be 0 or 1.

R₃₁ to R₃₉ may each independently be hydrogen, deuterium, a halogen, a cyano group, a substituted or unsubstituted amine group, a substituted or unsubstituted alkyl group having 1 to 20 carbons, a substituted or unsubstituted aryl group having 6 to 30 ring-forming carbon atoms, or a substituted or unsubstituted heteroaryl group having 2 to 30 ring-forming carbon atoms, and/or forms a ring by being coupled to an adjacent group. d1 to d4 may each independently be an integer of 0 to 4. When d1 is an integer of 2 or greater, a plurality of R₃₁'s may all be the same or at least one thereof may be different from the others. When d2 is an integer of 2 or greater, a plurality of R₃₂'s may all be the same or at least one thereof may be different from the others. When d3 is an integer of 2 or greater, a plurality of R₃₃'s may all be the same or at least one thereof may be different from the others. When d4 is an integer of 2 or greater, a plurality of R₃₄'s may all be the same or at least one thereof may be different from the others.

The compound represented by Formula M-b may be used as a blue phosphorescent dopant or a green phosphorescent dopant. In addition, the compound represented by Formula M-b may further be included in the emission layer EML as an auxiliary dopant in one or more embodiments. The compound represented by Formula M-b may be any one selected from compounds listed below. However, those compounds are mere example, and the compound represented by Formula M-b is not limited to the compounds listed below. In compounds AD-01 to AD-53, D is deuterium.

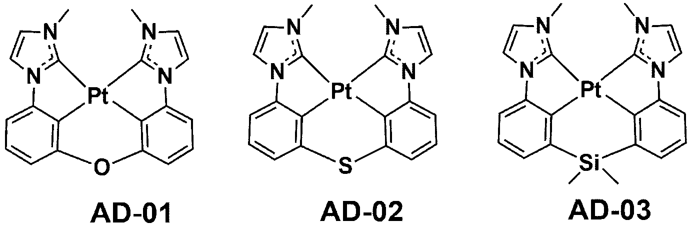

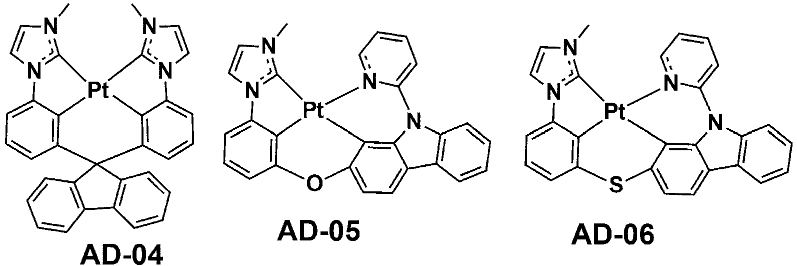

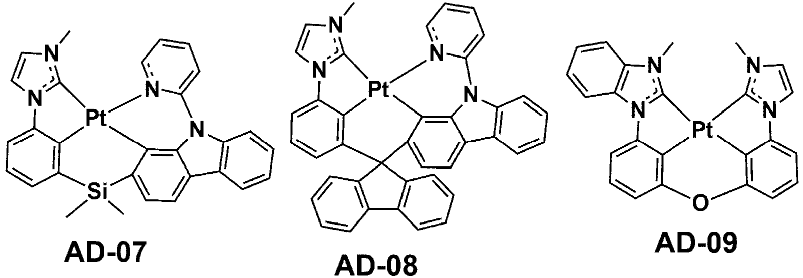

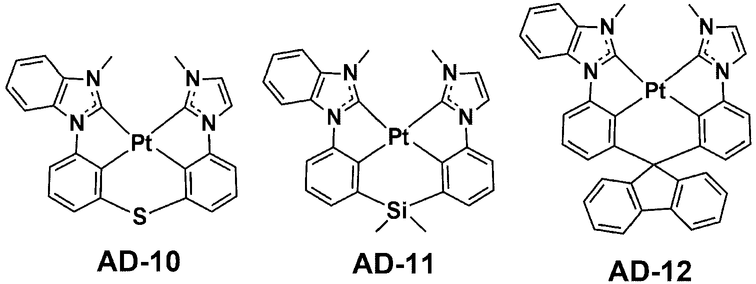

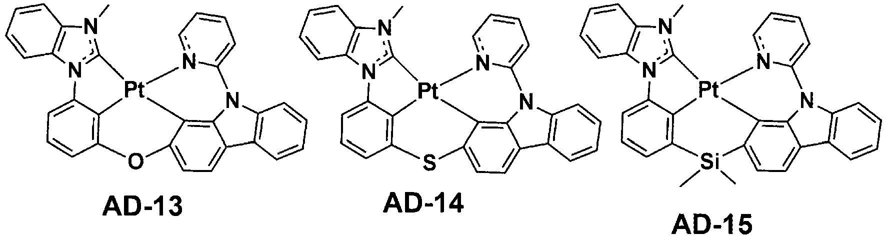

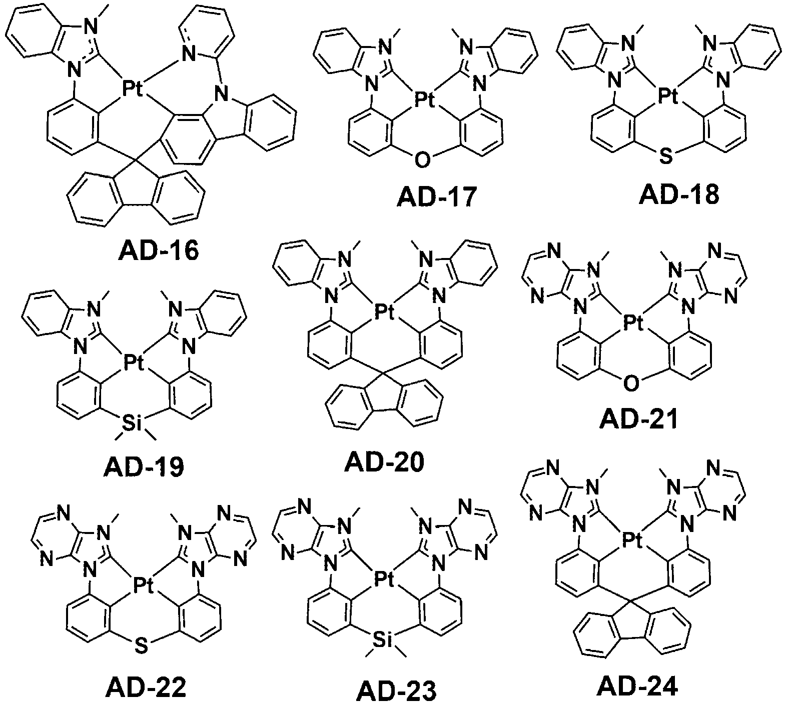

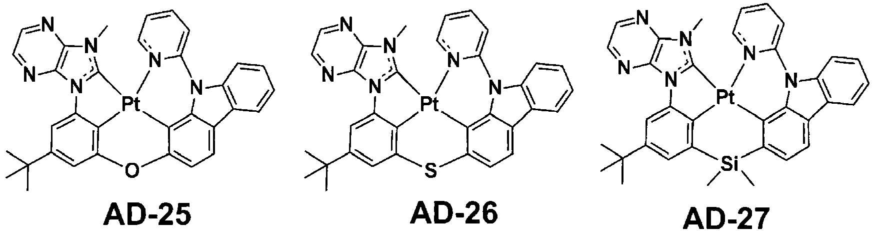

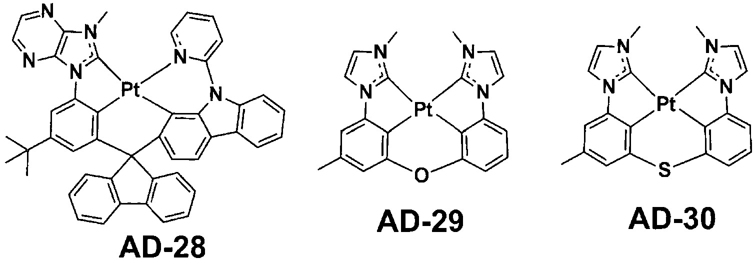

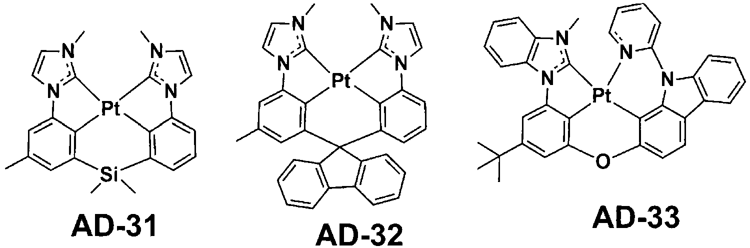

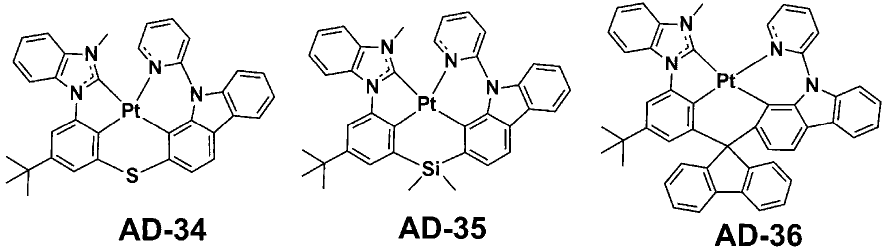

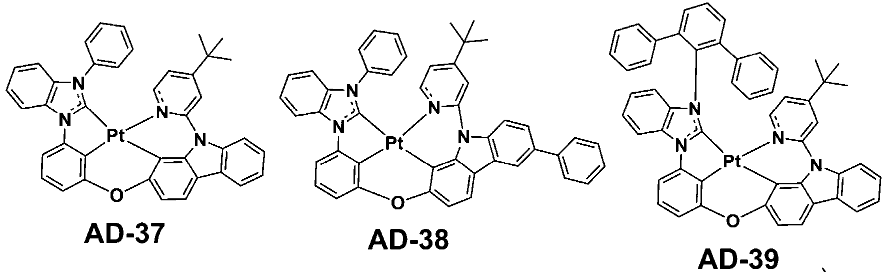

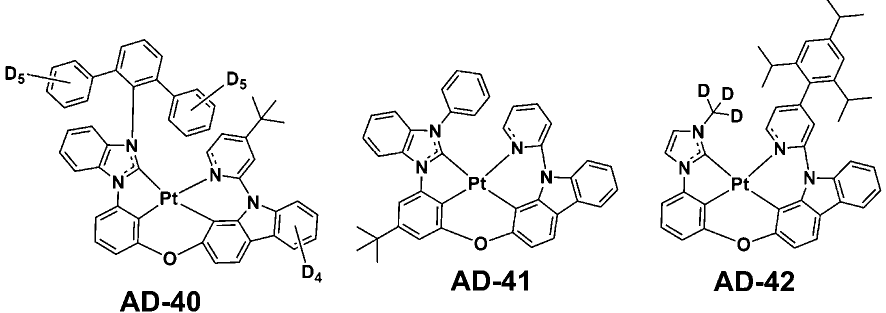

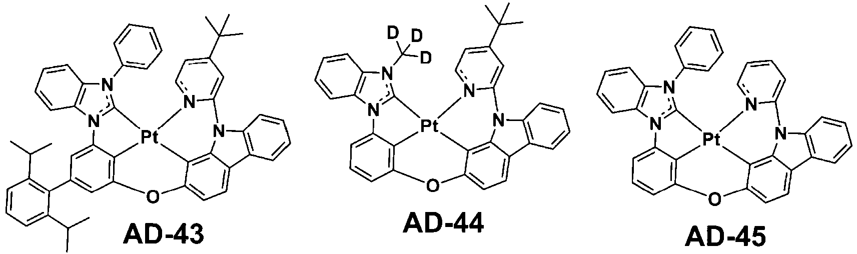

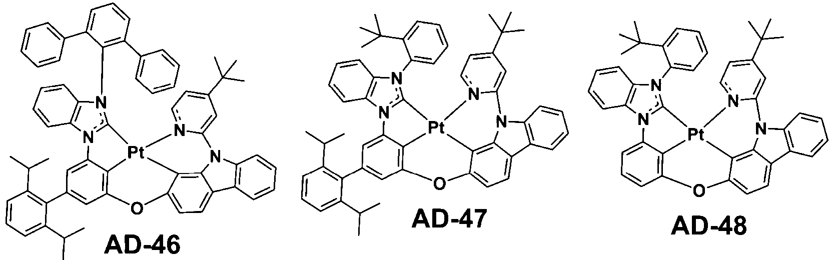

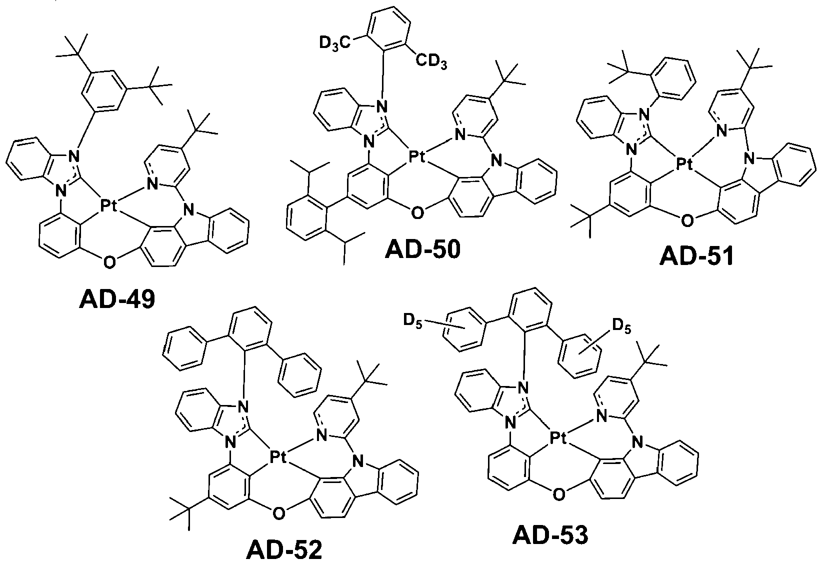

In one or more embodiments, the emission layer EML may include a compound represented by any one selected from among Formula F-a to Formula F-c. The compound represented by Formula F-a to Formula F-c may be used as a fluorescence dopant material.

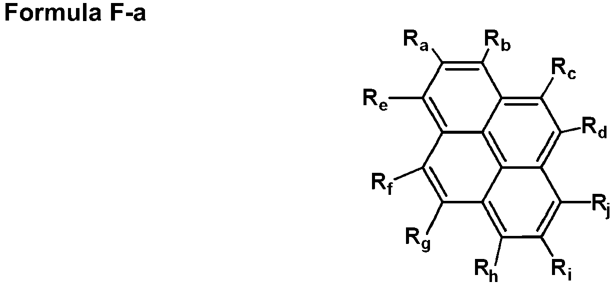

In Formula F-a, two selected from among Rₐ to Rⱼ may each independently be substituted with *-NAr₁Ar₂. The others, which are not substituted with *-NAr₁Ar₂, among Rₐ to Rⱼ may each independently be hydrogen, deuterium, a halogen, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In *-NAr₁Ar₂, Ar₁ and Ar₂ may each independently be a substituted or unsubstituted aryl group having 6 to 30 ring-forming carbon atoms or a substituted or unsubstituted heteroaryl group having 2 to 30 ring-forming carbon atoms. For example, in one or more embodiments, at least one of Ar₁ or Ar₂ may be a heteroaryl group containing O or S as a ring-forming atom.

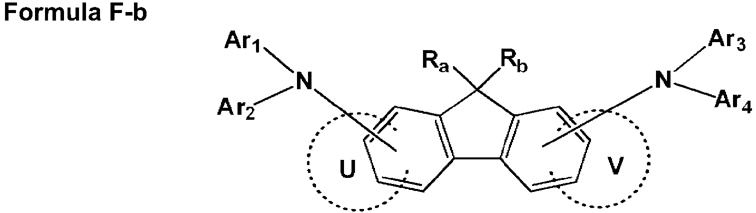

In Formula F-b, Rₐ and R_{b} may each independently be hydrogen, deuterium, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, and/or bonded to an adjacent group to form a ring. Ar₁ to Ar₄ may each independently be a substituted or unsubstituted aryl group having 6 to 30 ring-forming carbon atoms or a substituted or unsubstituted heteroaryl group having 2 to 30 ring-forming carbon atoms.

In Formula F-b, U and V may each independently be a substituted or unsubstituted hydrocarbon ring having 5 to 30 ring-forming carbon atoms or a substituted or unsubstituted heterocycle having 2 to 30 ring-forming carbon atoms. At least one selected from among Ar₁ to Ar₄ may be a heteroaryl group containing O or S as a ring-forming atom.

In Formula F-b, the number of rings represented by U and V may each independently be 0 or 1. For example, in Formula F-b, it refers to that if (e.g., when) the number of U or V is 1, one ring constitutes a part of a fused ring at a portion indicated by U or V, and if (e.g., when) the number of U or V is 0, a ring indicated by U or V does not exist. For example, if (e.g., when) the number of U is 0 and the number of V is 1, or if (e.g., when) the number of U is 1 and the number of V is 0, the fused ring having a fluorene core in Formula F-b may be a cyclic compound having four rings. In one or more embodiments, if (e.g., when) each number of U and V is 0, the fused ring in Formula F-b may be a cyclic compound having three rings. In one or more embodiments, if (e.g., when) each number of U and V is 1, the fused ring having a fluorene core in Formula F-b may be a cyclic compound having five rings.

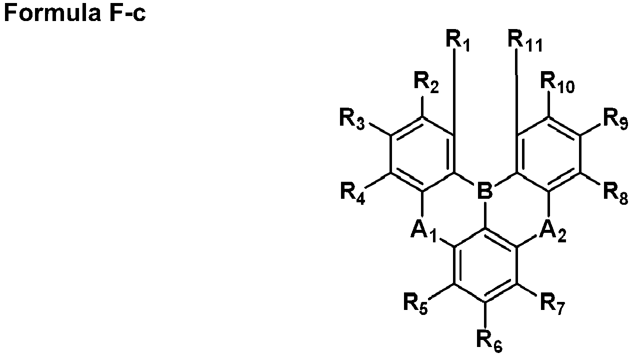

In Formula F-c, A₁ and A₂ may each independently be O, S, Se, or NRₘ, and Rₘ may be hydrogen, deuterium, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. R₁ to R₁₁ may each independently be hydrogen, deuterium, a halogen, a cyano group, a substituted or unsubstituted amine group, a substituted or unsubstituted boryl group, a substituted or unsubstituted oxy group, a substituted or unsubstituted thio group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, and/or independently bonded to an adjacent group to form a ring.

In Formula F-c, A₁ and A₂ may each independently be bonded to substituents of an adjacent ring to form a fused ring. For example, if (e.g., when) A₁ and A₂ are each independently NRₘ, A₁ may be bonded to R₄ or R₅ to form a ring. In addition, A₂ may be bonded to R₇ or R₈ to form a ring.

In one or more embodiments, the emission layer EML may further include, as a suitable dopant material, one or more selected from among styryl derivatives (e.g., 1,4-bis[2-(3-N-ethylcarbazolyl)vinyl]benzene (BCzVB), 4-(di-p-tolylamino)-4'-[(dip-tolylamino)styryl]stilbene (DPAVB), N-(4-((E)-2-(6-((E)-4-(diphenylamino)styryl)naphthalen-2-yl)vinyl)phenyl)-N-phenylbenzenamine (N-BDAVBi), 4,4'-bis[2-(4-(N,N'-diphenylamino)phenyl)vinyl]biphenyl (DPAVBi)), perylene and derivatives thereof (e.g., 2,5,8,11-tetra-t-butylperylene (TBP)), pyrene and derivatives thereof (e.g., 1,1'-dipyrene, 1,4-dipyrenylbenzene, 1,4-bis(N,N'-diphenylamino)pyrene), and/or the like.

In one or more embodiments, the emission layer EML may further include a suitable phosphorescence dopant material. For example, a metal complex containing iridium (Ir), platinum (Pt), osmium (Os), gold (Au), titanium (Ti), zirconium (Zr), hafnium (Hf), europium (Eu), terbium (Tb), or thulium (Tm) may be used as a phosphorescent dopant. For example, iridium(III) bis(4,6-difluorophenylpyridinato-N,C2')picolinate (Flrpic), bis(2,4-difluorophenylpyridinato)-tetrakis(1-pyrazolyl)borate iridium(III) (Fir6), or platinum octaethyl porphyrin (PtOEP) may be used as a phosphorescent dopant. However, embodiments of the present disclosure are not limited thereto.

In one or more embodiments, the emission layer EML may include a quantum dot material. The quantum dot may have a core/shell structure. A core of the quantum dot may be selected from among a group II-VI compound, a group I-II-VI compound, a group II-IV-VI compound, a group I-II-IV-VI compound, a group II-IV-V compound, a group III-VI compound, a group I-III-VI compound, a group III-V compound, a group III-II-V compound, a group IV-VI compound, a group IV element, a group IV compound, and any combination thereof.

The group II-VI compound may be selected from the group consisting of: binary compounds selected from the group consisting of CdSe, CdTe, CdS, ZnS, ZnSe, ZnTe, ZnO, HgS, HgSe, HgTe, MgSe, MgS, and a mixture thereof; ternary compounds selected from the group consisting of CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS and a mixture thereof; and quaternary compounds selected from the group consisting of HgZnTeS, CdZnSeS, CdZnSeTe, CdZnSTe, CdHgSeS, CdHgSeTe, CdHgSTe, HgZnSeS, HgZnSeTe, and a mixture thereof.

In one or more embodiments, the group II-VI compound may further include a group I metal and/or a group IV element. The group I-II-VI compound may be selected from among CuSnS and CuZnS, and as the group II-IV-VI compound, ZnSnS and/or the like may be selected. The group I-II-IV-VI compound may be selected from among quaternary compounds selected from the group consisting of Cu₂ZnSnS₂, Cu₂ZnSnS₄, Cu₂ZnSnSe₄, Ag₂ZnSnS₂ and a mixture thereof.

The group II-IV-V compound may be selected from among ternary compounds selected from the group consisting of ZnSnP, ZnSnP₂, ZnSnAs₂, ZnGeP₂, ZnGeAs₂, CdSnP₂, CdGeP₂, and a mixture thereof.

The group III-VI compound may include: binary compounds such as GaS, Ga₂S₃, GaSe, Ga₂Se₃, GaTe, InTe, InS, InSe, In₂S₃, and ln₂Se₃; ternary compounds such as InGaS₃, and InGaSe₃; or any combination thereof.

The group I-III-VI compound may be selected from among: ternary compounds selected from the group consisting of AgInS, AgInS₂, CulnS, CuInS₂, AgGaS₂, CuGaS₂ CuGaO₂, AgGaO₂, AgAlO₂ and a mixture thereof; and/or quaternary compounds such as AgInGaS₂ and CuInGaS₂.

The group III-V compound may be selected from the group consisting of: binary compounds selected from the group consisting of GaN, GaP, GaAs, GaSb, AlN, AlP, AlAs, AlSb, InN, InP, InAs, InSb, and a mixture thereof; ternary compounds selected from the group consisting of GaNP, GaNAs, GaNSb, GaPAs, GaPSb, AINP, AlNAs, AlNSb, AlPAs, AlPSb, InGaP, InAlP, InNP, InNAs, InNSb, InPAs, InPSb, and a mixture thereof; and quaternary compounds selected from the group consisting of GaAlNP, GaAINAs, GaAINSb, GaAlPAs, GaAlPSb, GalnNP, GalnNAs, GalnNSb, GalnPAs, GalnPSb, InAlNP, InAINAs, InAlNSb, InAIPAs, InAlPSb, and a mixture thereof. In one or more embodiments, the group III-V compound may further include a group II metal. For example, as the group III-II-V compound, InZnP and/or the like may be selected.

The group IV-VI compound may be selected from the group consisting of: binary compounds selected from the group consisting of SnS, SnSe, SnTe, PbS, PbSe, PbTe, and a mixture thereof; ternary compounds selected from the group consisting of SnSeS, SnSeTe, SnSTe, PbSeS, PbSeTe, PbSTe, SnPbS, SnPbSe, SnPbTe, and a mixture thereof; and quaternary compounds selected from the group consisting of SnPbSSe, SnPbSeTe, SnPbSTe, and a mixture thereof. The group IV element may be selected from the group consisting of Si, Ge, and a mixture thereof. The group IV compound may be a binary compound selected from the group consisting of SiC, SiGe, and a mixture thereof.

Each element contained in a multi-element compound such as the binary compound, the ternary compound, or the quaternary compound may be present at a substantially uniform concentration or non-uniform concentration in a particle. For example, the formulas above may represent types (kinds) of elements included in the compound, and an element ratio in the compound may vary. For example, AglnGaS₂ may refer to AgInₓGa₁₋ₓS₂ (where x is a real number between 0 to 1).

The quantum dot may have a single (e.g., monolithic) structure, in which a concentration of each element included in the corresponding quantum dot is substantially uniform, or a core-shell dual structure. For example, a material included in the core may be different from a material included in the shell.

The shell of the quantum dot may serve as a protection layer for preventing or reducing the core from being chemically modified to maintain semiconductor properties and/or a charging layer for imparting electrophoretic properties to the quantum dot. The shell may have a single layer or a multilayer. An interface between the core and the shell may have a concentration gradient in which the concentration of an element present in the shell may decrease gradually toward the core.

In one or more embodiments, the quantum dot may have a core-shell structure that includes the core including nanocrystals and the shell around (e.g., surrounding) the core. Examples of the shell of the quantum dot may include an oxide of a metal or non-metal, a semiconductor compound, a combination thereof, and/or the like.

For example, binary compounds such as SiO₂, Al₂O₃, TiO₂, ZnO, MnO, Mn₂O₃, Mn₃O₄, CuO, FeO, Fe₂O₃, Fe₃O₄, CoO, Co₃O₄, and/or NiO, or ternary compounds such as MgAl₂O₄, CoFe₂O₄, NiFe₂O₄, and/or CoMn₂O₄ may be provided as an example of the oxide of metal or non-metal, but embodiments of the present disclosure are not limited thereto.

Also, examples of the semiconductor compound suitable as a shell may include CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, and/or the like, but embodiments of the present disclosure are not limited thereto.

Each element included in a multi-element compound such as binary compound, the ternary compound, or the quaternary compound may be present at substantially uniform concentration or non-uniform concentration within a particle. For example, the formulas above may represent types (kinds) of elements included in the compound, and an element ratio in the compound may vary.

The quantum dot may have a full width at half maximum (FWHM) of a light-emitting wavelength spectrum (e.g., emission spectrum) of about 45 nm or less, about 40 nm or less, or about 30 nm or less, and if (e.g., when) the FWHM falls within this range, color purity or color reproducibility of the quantum dot may be improved. In addition, light emitted through the quantum dot may be emitted in all directions, and thus an optical viewing angle may be improved.

In addition, the quantum dot may have a shape that is generally used in the art without particular limitation. However, more specifically, the quantum dot may have a form of spherical nanoparticles, pyramidal nanoparticles, multi-armed nanoparticles, cubic nanoparticles, nanotubes, nanowires, nanofibers, or nanoplatelet particles.

Because the energy band gap of the quantum dot may be controlled or selected by controlling the size of the quantum dot or controlling the element ratio in the quantum dot compound, light with one or more suitable wavelength bands may be emitted from a quantum dot emission layer. Accordingly, by using the above-described quantum dot (using quantum dots having one or more suitable sizes or controlling an element ratio variously in a quantum dot compound), a light-emitting element which emits light with one or more suitable wavelengths may be achieved. For example, the size of the quantum dot and/or the element ratio in the quantum dot compound may be controlled or selected such that red, green, and/or blue light is emitted. In addition, the quantum dots may be configured to emit white light by combining one or more suitable colors of light.

In each of the light-emitting elements ED of embodiments illustrated in FIGS. 3 to 6, the electron transport region ETR may be provided on the emission layer EML. The electron transport region ETR may include at least one of a hole blocking layer HBL, an electron transport layer ETL, or an electron injection layer EIL, but embodiments of the present disclosure are not limited thereto.

The electron transport region ETR may have a single layer formed of a single material, a single layer formed of a plurality of different materials, or a multilayer structure including a plurality of layers formed of a plurality of different materials.

For example, in one or more embodiments, the electron transport region ETR may have a single layer structure of an electron injection layer EIL or an electron transport layer ETL, or may have a single layer structure formed of an electron injection material and/or an electron transport material. In one or more embodiments, the electron transport region ETR may have a single layer structure formed of a plurality of different materials, or may have a structure in which an electron transport layer ETL/electron injection layer EIL, or a hole blocking layer HBL/electron transport layer ETL/electron injection layer EIL are stacked in order (e.g., in the stated order) from the emission layer EML, but embodiments of the present disclosure are not limited thereto. The electron transport region ETR may have a thickness, for example, from about 1,000 Å to about 1,500 Å.

The electron transport region ETR may be formed using one or more suitable methods such as a vacuum deposition method, a spin coating method, a casting method, a Langmuir-Blodgett (LB) method, an inkjet printing method, a laser printing method, and/or a laser induced thermal imaging (LITI) method.

In one or more embodiments, the electron transport region ETR may include a compound represented by Formula ET-2:

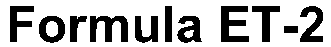

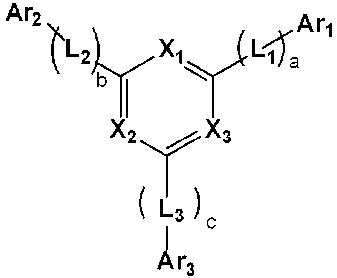

In Formula ET-2, at least one selected from among X₁ to X₃ is N, and the rest may be CRₐ. Rₐ may be hydrogen, deuterium, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. Ar₁ to Ar₃ may each independently be hydrogen, deuterium, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In Formula ET-2, a to c may each independently be an integer of 0 to 10. In Formula ET-2, L₁ to L₃ may each independently be a direct linkage, a substituted or unsubstituted arylene group having 6 to 30 ring-forming carbon atoms, or a substituted or unsubstituted heteroarylene group having 2 to 30 ring-forming carbon atoms. When a to c are each independently an integer of 2 or greater, L₁'s to L₃'s may each independently be a substituted or unsubstituted arylene group having 6 to 30 ring-forming carbon atoms or a substituted or unsubstituted heteroarylene group having 2 to 30 ring-forming carbon atoms.

In one or more embodiments, the electron transport region ETR may include an anthracene-based compound. However, embodiments of the present disclosure are not limited thereto, and the electron transport region ETR may include, for example, tris(8-hydroxyquinolinato)aluminium (Alq₃), 1,3,5-tri[(3-pyridyl)-phen-3-yl]benzene, 2,4,6-tris(3'-(pyridin-3-yl)biphenyl-3-yl)-1,3,5-triazine, 2-(4-(N-phenylbenzimidazol-1-yl)phenyl)-9,10-dinaphthylanthracene, 1,3,5-tri(1-phenyl-1H-benzo[d]imidazol-2-yl)benzene (TPBi), 2,9-dimethyl-4,7-diphenyl-1,10-phenanthroline (BCP), 4,7-diphenyl-1,10-phenanthroline (Bphen), 3-(biphenyl-4-yl)-4-phenyl-5-tert-butylphenyl-1,2,4-triazole (TAZ), 4-(naphthalen-1-yl)-3,5-diphenyl-4H-1,2,4-triazole (NTAZ), 2-(biphenyl-4-yl)-5-(4-tert-butylphenyl)-1,3,4-oxadiazole (tBu-PBD), bis(2-methyl-8-quinolinolato-N1,O8)-(1,1'-biphenyl-4-olato)aluminium (BAlq), beryllium bis(benzoquinolin-10-olate) (Bebq₂), 9,10-di(naphthalen-2-yl)anthracene (ADN), 1,3-bis[3,5-di(pyridin-3-yl)phenyl]benzene (BmPyPhB), or a mixture thereof.

In one or more embodiments, the electron transport region ETR may include at least one selected from among Compound ET1 to Compound ET36:

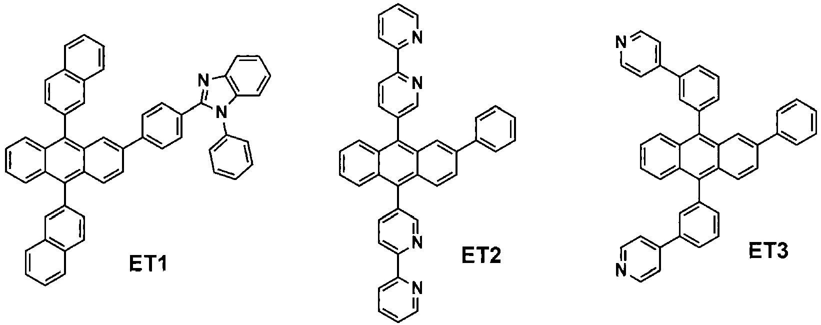

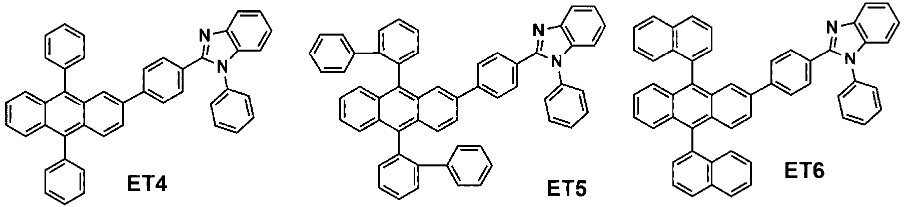

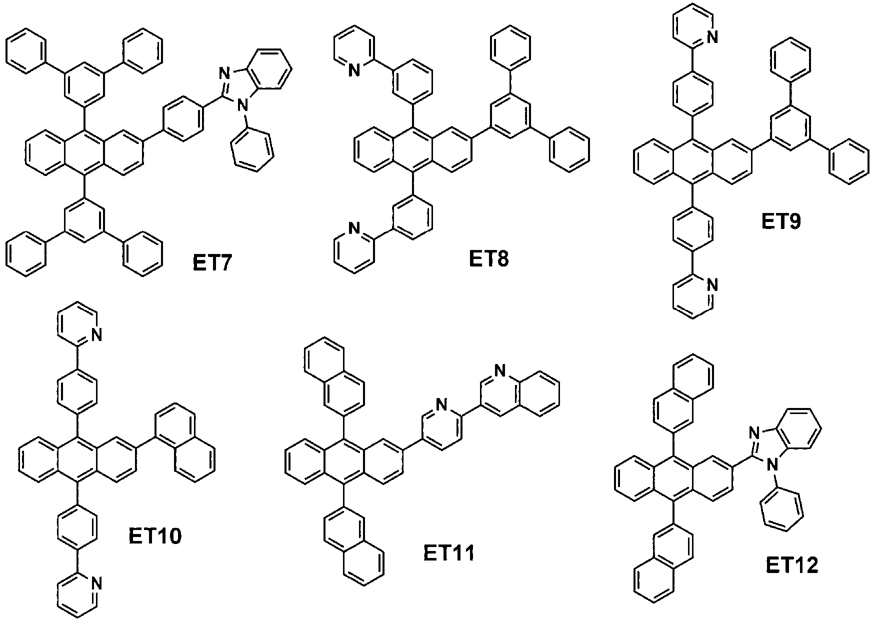

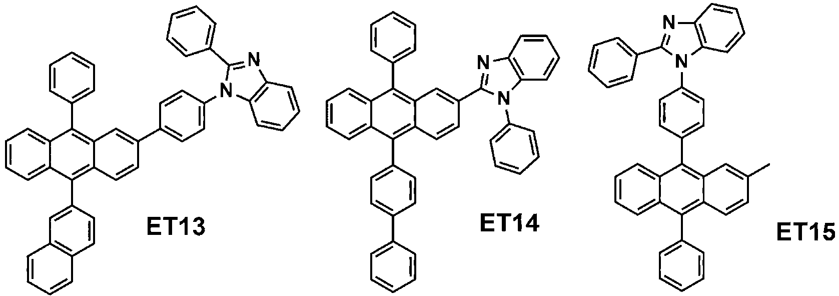

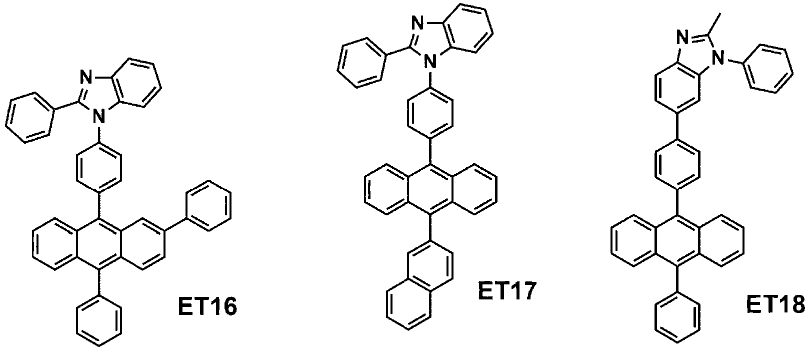

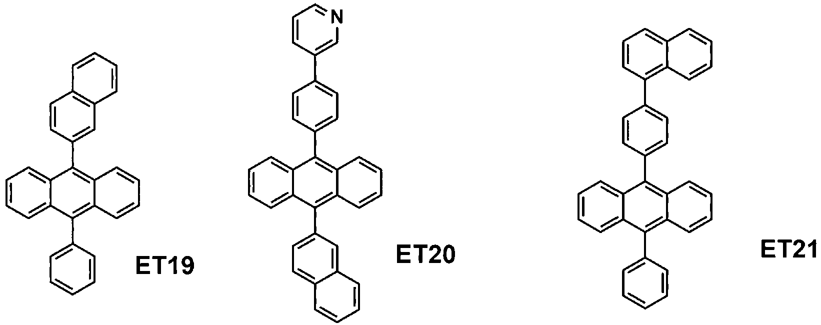

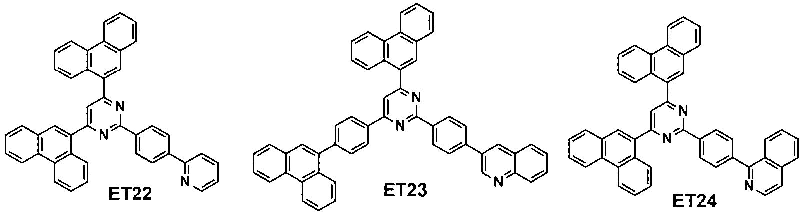

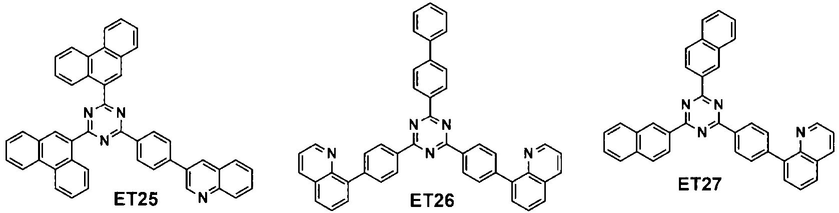

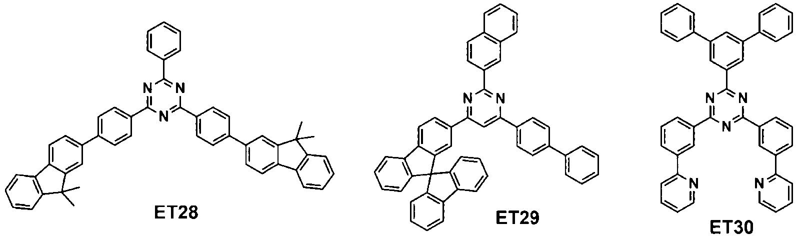

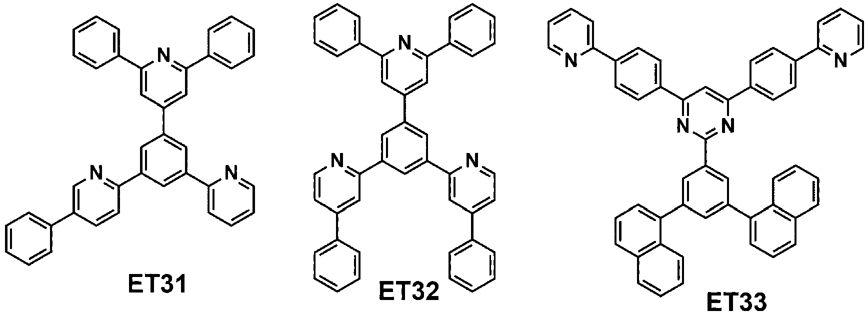

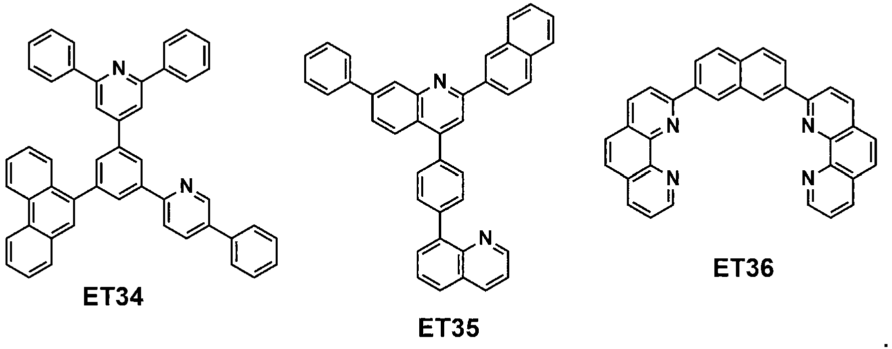

In one or more embodiments, the electron transport region ETR may include a metal halide such as LiF, NaCl, CsF, RbCl, Rbl, Cul, and/or Kl, a lanthanide metal such as Yb, or a co-deposited material of the metal halide and the lanthanide metal. For example, in one or more embodiments, the electron transport region ETR may include KI:Yb, Rbl:Yb, LiF:Yb, and/or the like, as a co-deposited material. in one or more embodiments, the electron transport region ETR may be formed using a metal oxide such as Li₂O and/or BaO, or lithium-8-hydroxyquinolinolate (Liq), and/or the like, but embodiments of the present disclosure are not limited thereto. The electron transport region ETR may also be formed of a mixture material of an electron transport material and an insulating organometallic salt. The organometallic salt may be a material having an energy band gap of about 4 eV or more. For example, the organometallic salt may include, for example, a metal acetate, a metal benzoate, a metal acetoacetate, a metal acetylacetonate, and/or a metal stearate.

In one or more embodiments, the electron transport region ETR may further include at least one of 2,9-dimethyl-4,7-diphenyl-1,10-phenanthroline (BCP), diphenyl(4-(triphenylsilyl)phenyl)phosphine oxide (TSPO1), or 4,7-diphenyl-1,10-phenanthroline (Bphen) in addition to one or more of the above-described materials, but embodiments of the present disclosure are not limited thereto.

The electron transport region ETR may include one or more of the above-described compounds of the electron transport region in at least one of the electron injection layer EIL, the electron transport layer ETL, or the hole blocking layer HBL.

If (e.g., when) the electron transport region ETR includes the electron transport layer ETL, the electron transport layer ETL may have a thickness of about 100 Å to about 1,000 Å, for example, about 150 Å to about 500 Å. If (e.g., when) the thickness of the electron transport layer ETL satisfies the aforementioned range, satisfactory electron transport characteristics may be obtained without a substantial increase in driving voltage. If (e.g., when) the electron transport region ETR includes the electron injection layer EIL, the electron injection layer EIL may have a thickness of about 1 Å to about 100 Å, for example, about 3 Å to about 90 Å. If (e.g., when) the thickness of the electron injection layer EIL satisfies the above-described range, satisfactory electron injection characteristics may be obtained without a substantial increase in driving voltage.

The second electrode EL2 may be provided on the electron transport region ETR. The second electrode EL2 may be a common electrode. The second electrode EL2 may be a cathode or an anode, but embodiments of the present disclosure are not limited thereto. For example, if (e.g., when) the first electrode EL1 is an anode, the second electrode EL2 may be a cathode, and if (e.g., when) the first electrode EL1 is a cathode, the second electrode EL2 may be an anode.

The second electrode EL2 may be a transmissive electrode, a transflective electrode, or a reflective electrode. When the second electrode EL2 is the transmissive electrode, the second electrode EL2 may be formed of a transparent metal oxide, for example, indium tin oxide (ITO), indium zinc oxide (IZO), zinc oxide (ZnO), indium tin zinc oxide (ITZO), and/or the like.

When the second electrode EL2 is the transflective electrode or the reflective electrode, the second electrode EL2 may include Ag, Mg, Cu, Al, Pt, Pd, Au, Ni, Nd, Ir, Cr, Li, Ca, LiF/Ca, LiF/Al, Mo, Ti, Yb, W, or a compound or a mixture thereof (e.g., AgMg, AgYb, or MgYb). In one or more embodiments, the second electrode EL2 may have a multilayer structure including a reflective film or a transflective film formed of one or more of the above-described materials, and a transparent conductive film formed of ITO, IZO, ZnO, ITZO, and/or the like. For example, in one or more embodiments, the second electrode EL2 may include one of the above-described metal materials, a combination of at least two metal materials of the above-described metal materials, an oxide of the above-described metal materials, and/or the like.

In one or more embodiments, the second electrode EL2 may be connected with an auxiliary electrode. If (e.g., when) the second electrode EL2 is connected with the auxiliary electrode, the resistance of the second electrode EL2 may be decreased.

In one or more embodiments, a capping layer CPL may further be arranged on the second electrode EL2 of the light-emitting element ED. The capping layer CPL may include a multilayer or a single layer.

In one or more embodiments, the capping layer CPL may be an organic layer or an inorganic layer. For example, if (e.g., when) the capping layer CPL contains an inorganic material, the inorganic material may include an alkali metal compound (e.g., LiF), an alkaline earth metal compound (e.g., MgF₂), SiON, SiNₓ, SiO_{y}, and/or the like.

In one or more embodiments, if (e.g., when) the capping layer CPL includes an organic material, the organic material may include 2,2'-dimethyl-N,N'-di-[(1-naphthyl)-N,N'-diphenyl]-1,1'-biphenyl-4,4'-diamine (α-NPD), NPB, TPD, m-MTDATA, Alq₃, CuPc, N4,N4,N4',N4'-tetra(biphenyl-4-yl)biphenyl-4,4'-diamine (TPD15), 4,4',4"-tris(carbazol-9-yl)triphenylamine (TCTA), and/or the like, or may include an epoxy resin, and/or an acrylate such as methacrylate. However, embodiments of the present disclosure are not limited thereto, for example, in one or more embodiments, the capping layer CPL may include at least one selected from among Compounds P1 to P5:

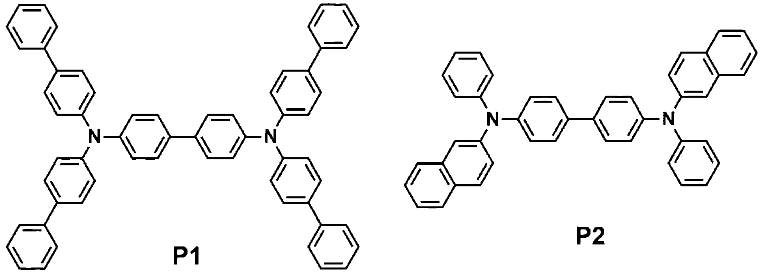

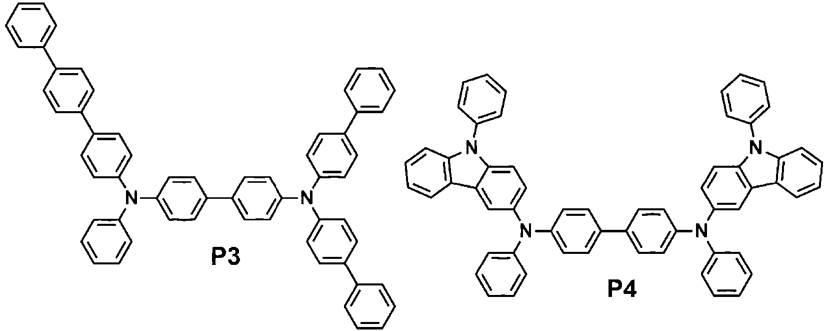

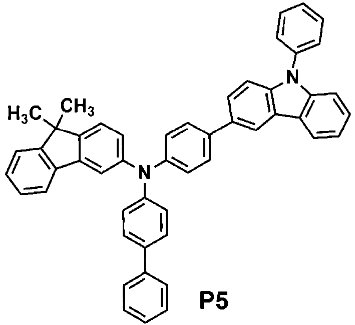

The refractive index of the capping layer CPL may be about 1.6 or more. For example, the refractive index of the capping layer CPL may be about 1.6 or more with respect to light in a wavelength range of about 550 nm to about 660 nm.

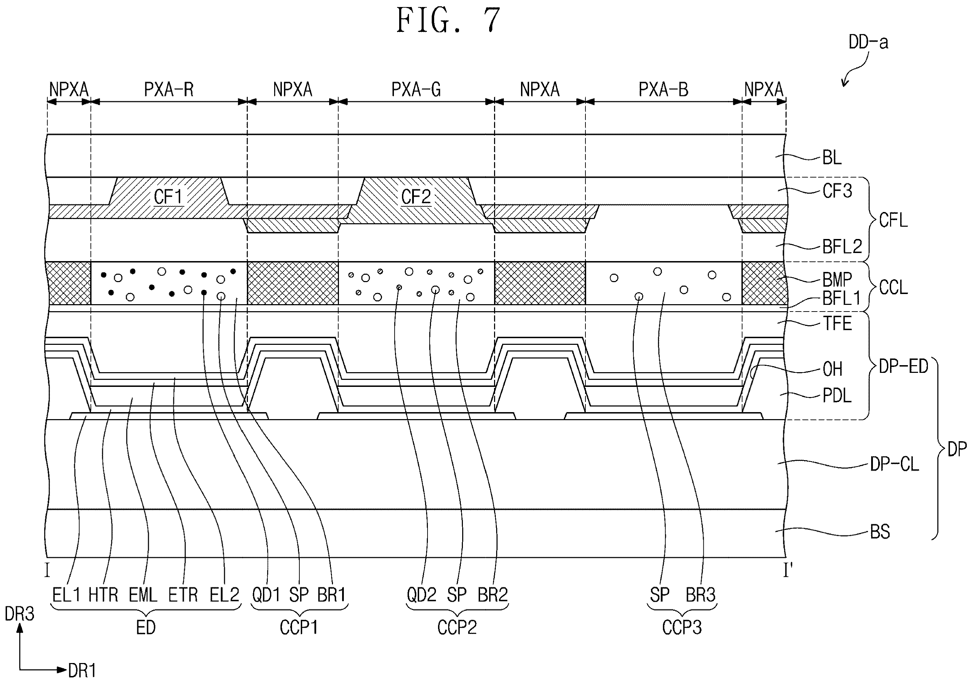

Each of FIGS. 7 to 10 is a cross-sectional view of a display device according to one or more embodiments of the present disclosure. Hereinafter, in describing the display devices of embodiments with reference to FIGS. 7 to 10, the duplicated features which have been described in FIGS. 1 to 6 are not described again, but their differences will be mainly described.

Referring to FIG. 7, a display device DD-a according to one or more embodiments may include a display panel DP including a display element layer DP-ED, a light control layer CCL arranged on the display panel DP, and a color filter layer CFL. In one or more embodiments illustrated in FIG. 7, the display panel DP may include a base layer BS, a circuit layer DP-CL provided on the base layer BS, and the display element layer DP-ED, and the display element layer DP-ED may include a light-emitting element ED.

The light-emitting element ED may include a first electrode EL1, a hole transport region HTR arranged on the first electrode EL1, an emission layer EML arranged on the hole transport region HTR, an electron transport region ETR arranged on the emission layer EML, and a second electrode EL2 arranged on the electron transport region ETR. The structures of the light-emitting elements of FIGS. 3 to 6 as described above may be equally applied to the structure of the light-emitting element ED illustrated in FIG. 7. The light-emitting element ED shown in FIG. 7 may include a monoamine compound of one or more embodiments of the present disclosure. The light-emitting element ED including the monoamine compound of one or more embodiments may exhibit high luminous efficiency and long life characteristics.

Referring to FIG. 7, the emission layer EML may be arranged in an opening OH defined in a pixel defining film PDL. For example, the emission layer EML which is divided by the pixel defining film PDL and provided corresponding to each of light emitting regions PXA-R, PXA-G, and PXA-B may be to emit light in substantially the same wavelength range. In the display device DD-a of one or more embodiments, the emission layer EML may be to emit blue light. Unlike the configuration illustrated, in one or more embodiments, the emission layer EML may be provided as a common layer across the entire light emitting regions PXA-R, PXA-G, and PXA-B.

The light control layer CCL may be arranged on the display panel DP. The light control layer CCL may include a light conversion body. The light conversion body may be a quantum dot, a phosphor, and/or the like. The light conversion body may be to emit provided light by converting the wavelength thereof. For example, the light control layer CCL may be a layer containing the quantum dot or a layer containing the phosphor.

The light control layer CCL may include a plurality of light control parts CCP1, CCP2, and CCP3. The light control parts CCP1, CCP2, and CCP3 may be spaced apart (e.g., spaced and/or apart) from one another.

Referring to FIG. 7, divided patterns BMP may be arranged between the light control parts CCP1, CCP2, and CCP3 which are spaced apart (e.g., spaced and/or apart) from one another, but embodiments of the present disclosure are not limited thereto. FIG. 7 illustrates that the divided patterns BMP do not overlap the light control parts CCP1, CCP2, and CCP3, but, in one or more embodiments, at least a portion of the edges of each of the light control parts CCP1, CCP2, and CCP3 may overlap the divided patterns BMP.

The light control layer CCL may include a first light control part CCP1 containing a first quantum dot QD1 which converts first color light provided from the light-emitting element ED into second color light, a second light control part CCP2 containing a second quantum dot QD2 which converts the first color light into third color light, and a third light control part CCP3 which transmits the first color light.

In one or more embodiments, the first light control part CCP1 may provide red light that is the second color light, and the second light control part CCP2 may provide green light that is the third color light. The third light control part CCP3 may provide blue light by transmitting blue light that is the first color light provided from the light-emitting element ED. For example, the first quantum dot QD1 may be a red quantum dot to emit red light, and the second quantum dot QD2 may be a green quantum dot to emit green light. The same as described above on quantum dots may be applied with respect to the quantum dots QD1 and QD2.

In one or more embodiments, the light control layer CCL may further include a scatterer SP. The first light control part CCP1 may include the first quantum dot QD1 and the scatterer SP, the second light control part CCP2 may include the second quantum dot QD2 and the scatterer SP, and the third light control part CCP3 may not include (e.g., may exclude) any quantum dot but include the scatterer SP.

The scatterer SP may be inorganic particles. For example, the scatterer SP may include at least one of TiO₂, ZnO, Al₂O₃, SiO₂, or hollow sphere silica. In one or more embodiments, the scatterer SP may include any one selected from among TiO₂, ZnO, Al₂O₃, SiO₂, and hollow sphere silica, or may be a mixture of at least two materials selected from among TiO₂, ZnO, Al₂O₃, SiO₂, and hollow sphere silica.

The first light control part CCP1, the second light control part CCP2, and the third light control part CCP3 may respectively include base resins BR1, BR2, and BR3 in which the quantum dots QD1 and QD2 and the scatterer SP are dispersed accordingly. In one or more embodiments, the first light control part CCP1 may include the first quantum dot QD1 and the scatterer SP dispersed in a first base resin BR1, the second light control part CCP2 may include the second quantum dot QD2 and the scatterer SP dispersed in a second base resin BR2, and the third light control part CCP3 may include the scatterer SP dispersed in a third base resin BR3.

The base resins BR1, BR2, and BR3 are media in which the quantum dots QD1 and QD2 and the scatterer SP are dispersed accordingly, and may each be formed of one or more suitable resin compositions, which may be generally referred to as a binder. For example, the base resins BR1, BR2, and BR3 may be each independently selected from among acrylic-based resins, urethane-based resins, silicone-based resins, epoxy-based resins, and/or the like. The base resins BR1, BR2, and BR3 may each be a transparent resin. In one or more embodiments, the first base resin BR1, the second base resin BR2, and the third base resin BR3 may be the same as or different from one another.

In one or more embodiments, the light control layer CCL may include a barrier layer BFL1. The barrier layer BFL1 may serve to prevent or reduce the penetration of moisture and/or oxygen (hereinafter, referred to as 'moisture/oxygen'). The barrier layer BFL1 may block the light control parts CCP1, CCP2 and CCP3 from being exposed to moisture/oxygen. The barrier layer BFL1 may cover the light control parts CCP1, CCP2, and CCP3. In one or more embodiments, the barrier layer BFL2 may be provided between the light control parts CCP1, CCP2, and CCP3 and the color filter layer CFL.

The barrier layers BFL1 and BFL2 may each include at least one inorganic layer. For example, in one or more embodiments, the barrier layers BFL1 and BFL2 may each include an inorganic material. For example, the barrier layers BFL1 and BFL2 may each independently include a silicon nitride, an aluminium nitride, a zirconium nitride, a titanium nitride, a hafnium nitride, a tantalum nitride, a silicon oxide, an aluminium oxide, a titanium oxide, a tin oxide, a cerium oxide, a silicon oxynitride, a metal thin film which secures a transmittance, and/or the like. In one or more embodiments, the barrier layers BFL1 and BFL2 may each independently further include an organic film. The barrier layers BFL1 and BFL2 may each independently be formed of a single layer or a plurality of layers.

In the display device DD-a of one or more embodiments, the color filter layer CFL may be arranged on the light control layer CCL. For example, in one or more embodiments, the color filter layer CFL may be directly arranged on the light control layer CCL. In these embodiments, the barrier layer BFL2 may not be provided.

The color filter layer CFL may include filters CF1, CF2, and CF3. For example, in one or more embodiments, the color filter layer CFL may include a first filter CF1 configured to transmit the second color light, a second filter CF2 configured to transmit the third color light, and a third filter CF3 configured to transmit the first color light. For example, the first filter CF1 may be a red filter, the second filter CF2 may be a green filter, and the third filter CF3 may be a blue filter. The filters CF1, CF2, and CF3 each may include a polymeric photosensitive resin and a pigment and/or a dye. For example, in one or more embodiments, the first filter CF1 may include a red pigment and/or a red dye, the second filter CF2 may include a green pigment and/or a green dye, and the third filter CF3 may include a blue pigment and/or a blue dye.

Embodiments of the present disclosure are not limited thereto, for example, the third filter CF3 may not include (e.g., may exclude) any pigment or any dye. The third filter CF3 may include a polymeric photosensitive resin and may not include (e.g., may exclude) any pigment or any dye. The third filter CF3 may be transparent. The third filter CF3 may be formed of a transparent photosensitive resin.

Furthermore, in one or more embodiments, the first filter CF1 and the second filter CF2 may each be a yellow filter. The first filter CF1 and the second filter CF2 may not be separated but be provided as one filter.

In one or more embodiments, the color filter layer CFL may further include a light shielding part. The light shielding part may be a black matrix. The light shielding part may include an organic light shielding material and/or an inorganic light shielding material, each containing a black pigment and/or a black dye. The light shielding part may prevent or reduce light leakage, and may separate boundaries between adjacent filters CF1, CF2, and CF3.

The first to third filters CF1, CF2, and CF3 may be arranged corresponding to the red light emitting region PXA-R, the green light emitting region PXA-G, and the blue light emitting region PXA-B, respectively.

A base substrate BL may be arranged on the color filter layer CFL. The base substrate BL may be a member which provides a base surface on which the color filter layer CFL, the light control layer CCL, and/or the like are arranged. The base substrate BL may be a glass substrate, a metal substrate, a plastic substrate, and/or the like. However, embodiments of the present disclosure are not limited thereto, and the base substrate BL may be an inorganic layer, an organic layer, or a composite material layer. In one or more embodiments, the base substrate BL may not be provided.

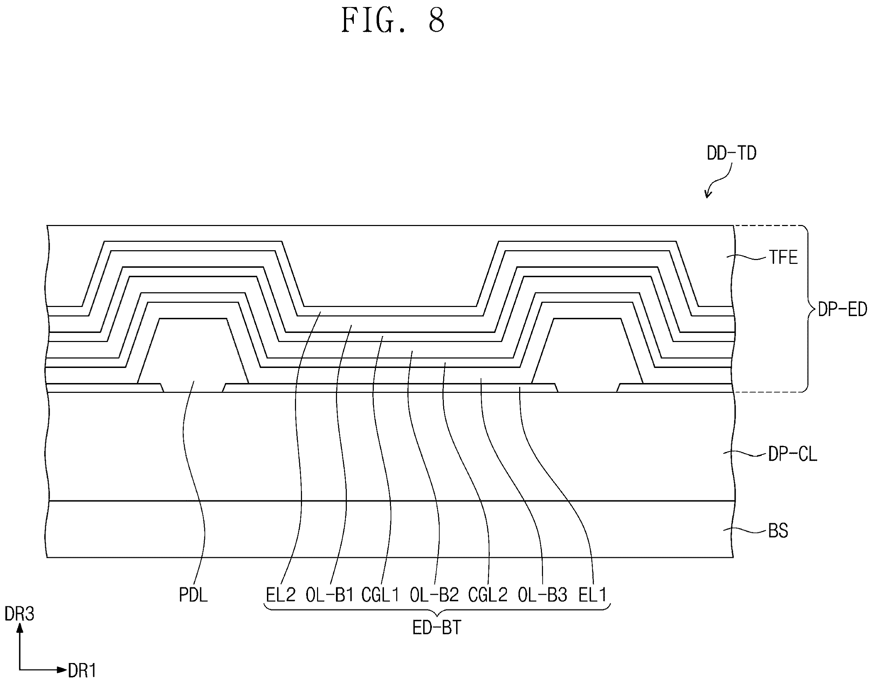

FIG. 8 is a cross-sectional view illustrating a portion of a display device according to one or more embodiments of the present disclosure. In a display device DD-TD of one or more embodiments, a light-emitting element ED-BT may include a plurality of light emitting structures OL-B1, OL-B2, and OL-B3. The light-emitting element ED-BT may include a first electrode EL1 and a second electrode EL2 which face each other, and the plurality of light emitting structures OL-B1, OL-B2, and OL-B3 sequentially stacked in a thickness direction between the first electrode EL1 and the second electrode EL2. The light emitting structures OL-B1, OL-B2, and OL-B3 each may include an emission layer EML (FIG. 7) and a hole transport region HTR and an electron transport region ETR arranged with the emission layer EML (FIG. 7) located therebetween. For example, the light-emitting element ED-BT included in the display device DD-TD of one or more embodiments may be a light-emitting element having a tandem structure and including a plurality of emission layers.

The light-emitting element ED-BT shown in FIG. 8 may include a monoamine compound of one or more embodiments of the present disclosure. The light-emitting element ED-BT including the monoamine compound of one or more embodiments may exhibit high luminous efficiency and long life characteristics.

In one or more embodiments illustrated in FIG. 8, all light beams respectively emitted from the light emitting structures OL-B1, OL-B2, and OL-B3 may be blue light. However, embodiments of the present disclosure are not limited thereto, for example, the light beams respectively emitted from the light emitting structures OL-B1, OL-B2, and OL-B3 may have wavelength ranges different from one another. For example, in one or more embodiments, the light-emitting element ED-BT including the plurality of light emitting structures OL-B1, OL-B2, and OL-B3 which emit light beams having wavelength ranges different from one another may be to emit white light (e.g., combined white light).

Charge generation layers CGL1 and CGL2 may be respectively arranged between two of the neighboring light emitting structures OL-B1, OL-B2, and OL-B3. The charge generation layers CGL1 and CGL2 may include a p-type (kind) charge generation layer and/or an n-type (kind) charge generation layer.

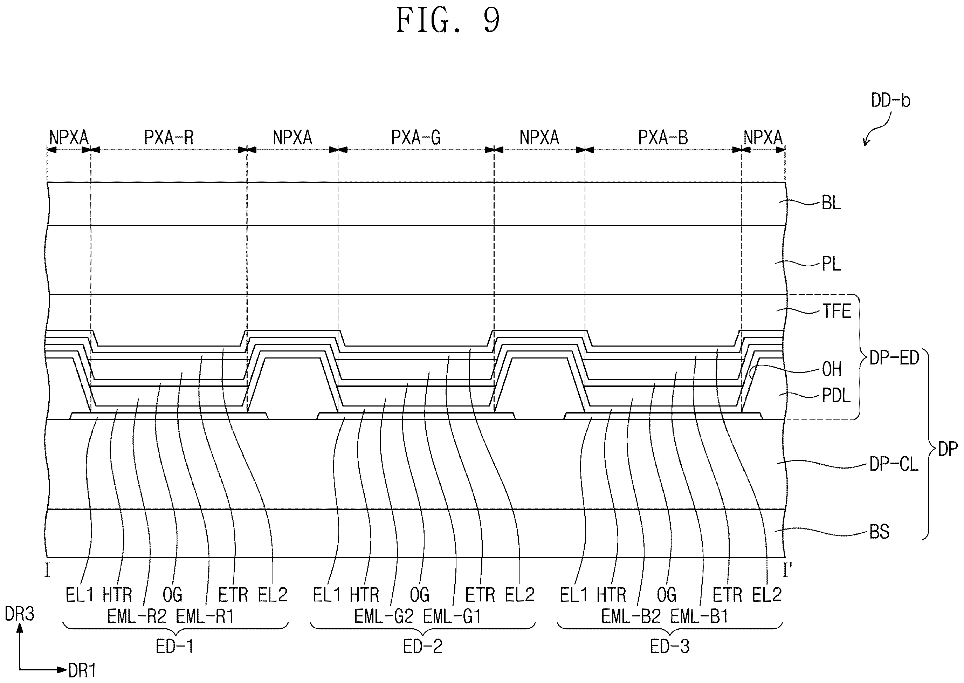

Referring to FIG. 9, a display device DD-b according to one or more embodiments may include light-emitting elements ED-1, ED-2, and ED-3 in each of which two emission layers are stacked. Compared with the display device DD illustrated in FIG. 2, the embodiment illustrated in FIG. 9 has a difference in that the first to third light-emitting elements ED-1, ED-2, and ED-3 each include two emission layers stacked in a thickness direction. In each of the first to third light-emitting elements ED-1, ED-2, and ED-3, the two emission layers may be to emit light in substantially the same wavelength region. At least one selected from among the first to third light-emitting elements ED-1, ED-2, and ED-3 may include the monoamine compound of one or more embodiments. The light-emitting element (at least one of ED-1, ED-2, or ED-3) including the monoamine compound of one or more embodiments may exhibit high luminous efficiency and long life characteristics.

In one or more embodiments, the first light-emitting element ED-1 may include a first red emission layer EML-R1 and a second red emission layer EML-R2. The second light-emitting element ED-2 may include a first green emission layer EML-G1 and a second green emission layer EML-G2. In addition, the third light-emitting element ED-3 may include a first blue emission layer EML-B1 and a second blue emission layer EML-B2. An emission auxiliary part OG may be arranged between the first red emission layer EML-R1 and the second red emission layer EML-R2, between the first green emission layer EML-G1 and the second green emission layer EML-G2, and between the first blue emission layer EML-B1 and the second blue emission layer EML-B2.

The emission auxiliary part OG may include a single layer or a multilayer. The emission auxiliary part OG may include a charge generation layer. For example, in one or more embodiments, the emission auxiliary part OG may include an electron transport region, a charge generation layer, and a hole transport region that are sequentially stacked (e.g., in the stated order). The emission auxiliary part OG may be provided as a common layer in the whole of the first to third light-emitting elements ED-1, ED-2, and ED-3. However, embodiments of the present disclosure are not limited thereto, and the emission auxiliary part OG may be provided by being patterned within openings OH defined in the pixel defining film PDL.

The first red emission layer EML-R1, the first green emission layer EML-G1, and the first blue emission layer EML-B1 may each be arranged between the emission auxiliary part OG and the electron transport region ETR. The second red emission layer EML-R2, the second green emission layer EML-G2, and the second blue emission layer EML-B2 may each be arranged between the hole transport region HTR and the emission auxiliary part OG.

For example, in one or more embodiments, the first light-emitting element ED-1 may include a first electrode EL1, a hole transport region HTR, a second red emission layer EML-R2, an emission auxiliary part OG, a first red emission layer EML-R1, an electron transport region ETR, and a second electrode EL2, which are sequentially stacked (e.g., in the stated order). The second light-emitting element ED-2 may include a first electrode EL1, the hole transport region HTR, a second green emission layer EML-G2, the emission auxiliary part OG, a first green emission layer EML-G1, the electron transport region ETR, and the second electrode EL2, which are sequentially stacked (e.g., in the stated order). The third light-emitting element ED-3 may include a first electrode EL1, the hole transport region HTR, a second blue emission layer EML-B2, the emission auxiliary part OG, a first blue emission layer EML-B1, the electron transport region ETR, and the second electrode EL2, which are sequentially stacked (e.g., in the stated order).

An optical auxiliary layer PL may be arranged on the display element layer DP-ED. The optical auxiliary layer PL may include a polarizing layer. The optical auxiliary layer PL may be arranged on the display panel DP and control reflected light in the display panel DP due to external light. In one or more embodiments, the optical auxiliary layer PL in the display device may not be provided.

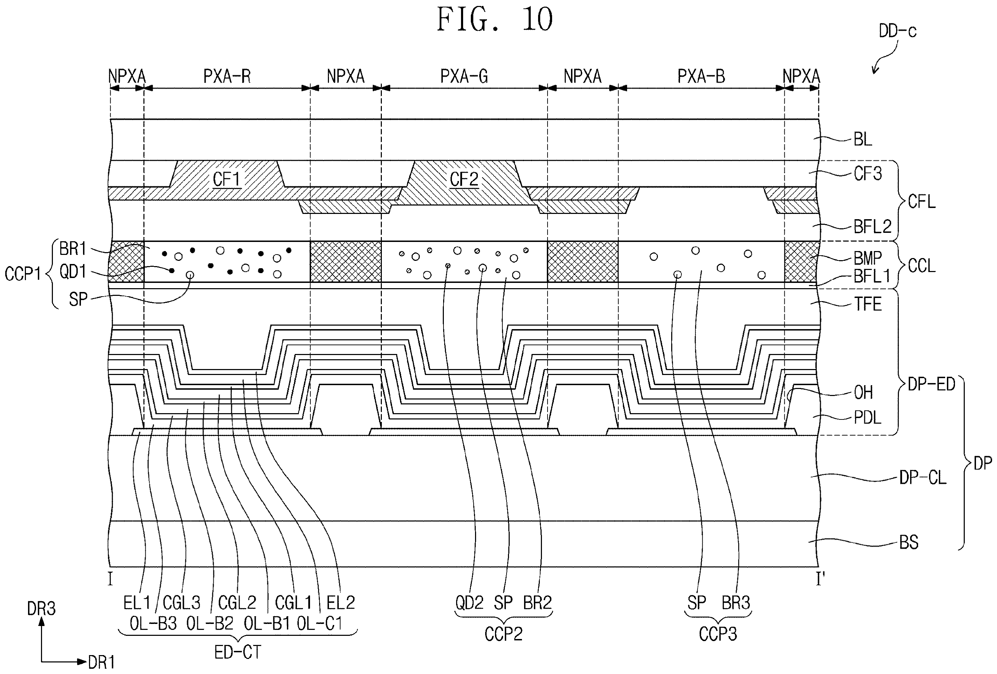

Different from FIG. 8 and FIG. 9, FIG. 10 illustrates that a display device DD-c includes four light emitting structures OL-B1, OL-B2, OL-B3, and OL-C1. A light-emitting element ED-CT may include a first electrode EL1 and a second electrode EL2 which face each other, and first to fourth light emitting structures OL-B1, OL-B2, OL-B3, and OL-C1 that are stacked in the thickness direction between the first electrode EL1 and the second electrode EL2. The third light emitting structure OL-B3, the second light emitting structure OL-B2, the first light emitting structure OL-B1, and the fourth light emitting structure OL-C1 are sequentially stacked in a thickness direction between the first electrode EL1 and the second electrode EL2. A first charge generation layer CGL1 may be arranged between the first light emitting structure OL-B1 and the fourth light emitting structure OL-C1. A second charge generation layer CGL2 may be arranged between the second light emitting structure OL-B2 and the first light emitting structure OL-B1. A third charge generation layer CGL3 may be arranged between the third light emitting structure OL-B3 and the second light emitting structure OL-B2. The light-emitting element ED-CT shown in FIG. 10 may include a monoamine compound of one or more embodiments of the present disclosure. The light-emitting element ED-CT including the monoamine compound of one or more embodiments may exhibit high luminous efficiency and long life characteristics.

In one or more embodiments, among the four light emitting structures, the first to third light emitting structures OL-B1, OL-B2, and OL-B3 may be to emit blue light, and the fourth light emitting structure OL-C1 may be to emit green light. However, embodiments of the present disclosure are not limited thereto, for example, in one or more embodiments, the first to fourth light emitting structures OL-B1, OL-B2, OL-B3, and OL-C1 may be to emit light beams in different wavelength regions.

The charge generation layers CGL1, CGL2, and CGL3 arranged between adjacent light emitting structures OL-C1, OL-B1, OL-B2, and OL-B3 may each include a p-type (kind) charge generation layer and/or an n-type (kind) charge generation layer.

In one or more embodiments, an electronic apparatus may include a display device including a plurality of light-emitting elements, and a control part which controls the display device. The electronic apparatus of one or more embodiments may be a device that is activated according to an electrical signal. The electronic apparatus may include display devices of one or more suitable embodiments. For example, the electronic apparatus may include not only large-sized electronic apparatuses such as a television set, a monitor, or an outdoor billboard but also include small- and medium-sized electronic apparatuses such as a personal computer, a laptop computer, a personal digital terminal, a display device for a vehicle, a game console, a portable electronic device, or a camera.

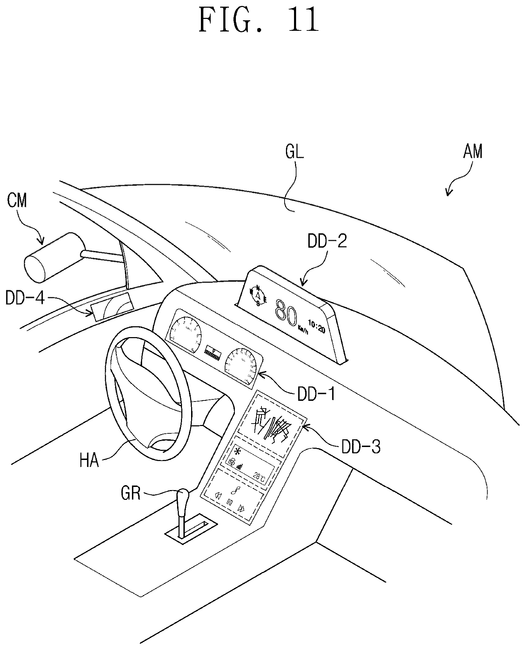

FIG. 11 is a view illustrating a vehicle AM in which first to fourth display devices DD-1, DD-2, DD-3, and DD-4 are arranged. At least one selected from among the first to fourth display devices DD-1, DD-2, DD-3, and DD-4 may include a same configuration as those of the display devices DD, DD-TD, DD-a, DD-b, and DD-c described with reference to FIGS. 1, 2, and 7 to 10.

FIG. 11 illustrates a vehicle AM, but this is merely an example, and the first to fourth display devices DD-1, DD-2, DD-3, and DD-4 may be arranged in other transportation apparatuses, such as bicycles, motorcycles, trains, ships, and airplanes. In one or more embodiments, at least one selected from among the first to fourth display devices DD-1, DD-2, DD-3, and DD-4 including the same configuration as those of the display devices DD, DD-TD, DD-a, DD-b, and DD-c of one or more embodiments may be employed in a personal computer, a laptop computer, a personal digital terminal, a game console, a portable electronic device, a television, a monitor, an outdoor billboard, and/or the like. In addition, these are merely provided as embodiments, and thus may be employed in other electronic apparatuses unless departing from the disclosure.

At least one selected from among the first to fourth display devices DD-1, DD-2, DD-3, and DD-4 may include the light-emitting element ED of one or more embodiments described with reference to FIGS. 3 to 6. At least one selected from among the first to fourth display devices DD-1, DD-2, DD-3, and DD-4 may include a monoamine compound of one or more embodiments of the present disclosure. The display device (at least one of DD-1, DD-2, DD-3, or DD-4) including the monoamine compound of one or more embodiments may exhibit excellent or suitable display efficiency and display lifespan.

Referring to FIG. 11, the vehicle AM may include a steering wheel HA and a gear GR for driving the vehicle AM. In addition, the vehicle AM may include a front window GL arranged so as to face a driver.

The first display device DD-1 may be arranged in a first region overlapping the steering wheel HA. For example, the first display device DD-1 may be a digital cluster which displays first information of the vehicle AM. The first information may include a first scale which indicates a driving speed of the vehicle AM, a second scale which indicates an engine speed (that is, revolutions per minute (RPM)), an image which indicates a fuel state, and/or the like. The first scale and the second scale may be indicated as a digital image.

The second display device DD-2 may be arranged in a second region opposite to (e.g., facing) a driver seat and overlapping the front window GL. The driver seat may be a seat in which the steering wheel HA faces. For example, the second display device DD-2 may be a head up display (HUD) which displays second information of the vehicle AM. The second display device DD-2 may be optically transparent. The second information may include digital numbers which indicate a driving speed, and may further include information such as the current time. Unlike the configuration illustrated, in one or more embodiments, the second information of the second display device DD-2 may be projected to the front window GL to be displayed.

The third display device DD-3 may be arranged in a third region adjacent to the gear GR. For example, the third display device DD-3 may be arranged between the driver seat and a passenger seat and may be a center information display (CID) for the vehicle for displaying third information. The passenger seat may be a seat spaced and/or apart from the driver seat with the gear GR arranged therebetween. The third information may include information about traffic (*e.g.*, navigation information), playing music or radio or a video (or an image), temperatures inside the vehicle AM, and/or the like.

The fourth display device DD-4 may be spaced and/or apart from the steering wheel HA and the gear GR, and may be arranged in a fourth region adjacent to a side of the vehicle AM. For example, the fourth display device DD-4 may be a digital side-view mirror which displays fourth information. The fourth display device DD-4 may display an image outside the vehicle AM taken by a camera module CM arranged outside the vehicle AM. The fourth information may include an image outside the vehicle AM.

The above-described first to fourth information may be examples, and the first to fourth display devices DD-1, DD-2, DD-3, and DD-4 may further display information about the inside and outside of the vehicle AM. The first to fourth information may include different information. However, embodiments of the present disclosure are not limited thereto, and a part of the first to fourth information may include the same information as one another.

In one or more embodiments, an electronic apparatus may include a display device including a plurality of light-emitting elements, and a control unit that controls the display device. The electronic apparatus according to one or more embodiments may be activated in response to an electrical signal. The electronic apparatus may include display devices according to one or more embodiments of the present disclosure. For example, the electronic apparatus may include small- and medium-sized display devices such as a personal computer, a laptop computer, a personal digital assistant, a vehicular display device, a game console, a portable electronic device, or a camera, as well as large-sized display devices such as a television, a monitor, or an outdoor billboard. In one or more embodiments, the electronic apparatus may include at least one of a flat panel display, a curved display, a computer monitor, a medical monitor, a television, a billboard, an indoor light, an outdoor light, a light for signaling, a head-up display, a fully transparent display, a partially transparent display, a flexible display, a rollable display, a foldable display, a stretchable display, a laser printer, a telephone, a portable phone, a tablet personal computer, a phablet, a personal digital assistant, a wearable device, a laptop computer, a digital camera, a camcorder, a viewfinder, a micro display, a three-dimension (3D) display, a virtual reality display, an augmented reality display, a vehicle, a video wall with multiple displays tiled together, a theater screen, a stadium screen, a phototherapy device, or a signboard.

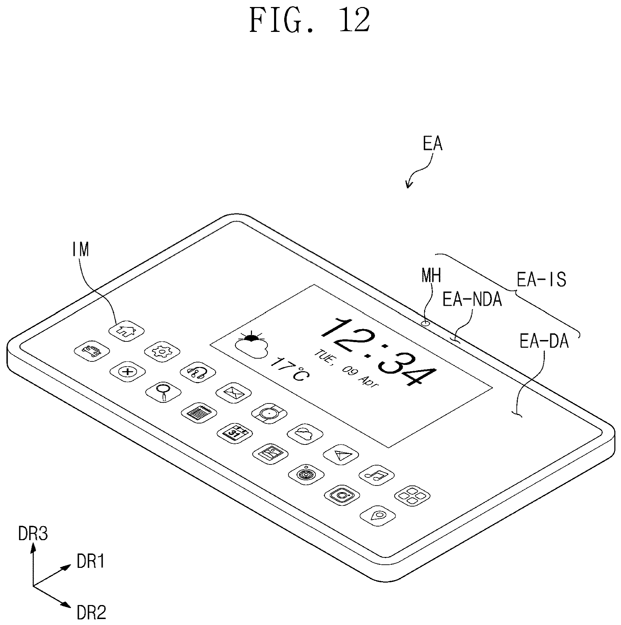

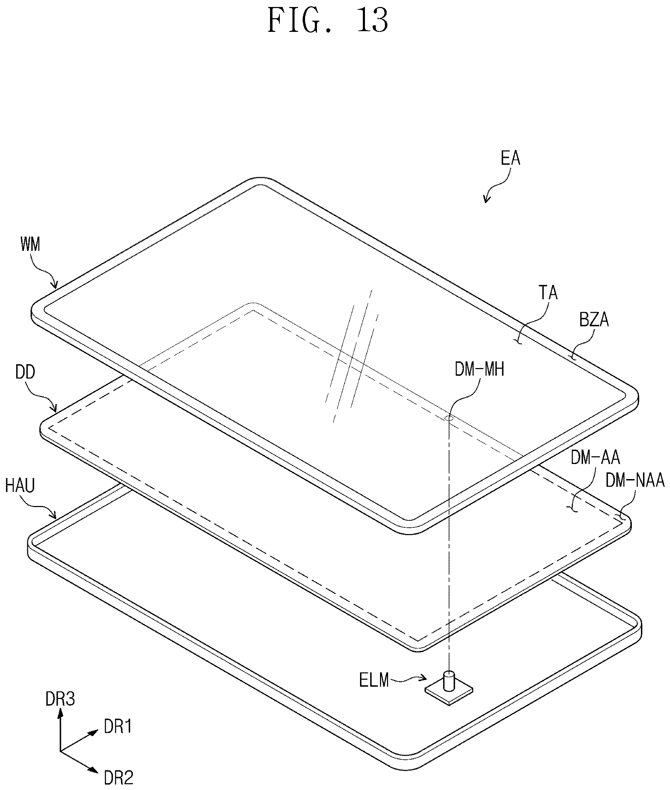

FIG. 12 is a perspective view illustrating an electronic apparatus according to one or more embodiments of the present disclosure. FIG. 13 is an exploded perspective view illustrating an electronic apparatus according to one or more embodiments.

An electronic apparatus EA may display an image IM through a display surface EA-IS. The image IM may include a still image as well as a dynamic image. The display surface EA-IS may be parallel to a plane defined by a first direction axis DR1 and a second direction axis DR2. FIG. 12 illustrates the electronic apparatus EA equipped with a flat display surface EA-IS, but embodiments of the present disclosure are not limited thereto. For example, in one or more embodiments, the electronic apparatus EA may include a curved display surface or 3-dimensional display surface. The 3-dimensional display surface may include a plurality of display regions indicating different directions.

The display surface EA-IS may include a display region EA-DA and a non-display region EA-NDA. The display surface EA-IS may further include a sub region MH. The electronic apparatus EA may display the image IM through the display region EA-DA.

The non-display region EA-NDA may not be an optically transparent region and may have a color. The non-display region EA-NDA may be adjacent to the display region EA-DA. The non-display region EA-NDA may surround the display region EA-DA. Therefore, the shape of the display region EA-DA may be defined substantially by the non-display region EA-NDA. However, FIG. 12 is for illustrative purposes, and the non-display region EA-NDA may be arranged adjacent to only one side of the display region EA-DA or may not be provided.

The sub region MH may detect an external object received through the display surface EA-IS or provide a sound signal such as voice to the outside through the display surface EA-IS. An optical signal such as visible light or infrared light may travel through the sub region MH.

In one or more embodiments, the sub region MH may be arranged in the display region EA-DA. However, this is an example, and an arrangement of the sub region MH is not limited to any specific arrangement. For example, the sub region MH may be surrounded by the non-display region EA-NDA or may be surrounded by the display region EA-DA and the non-display region EA-NDA. FIG. 12 illustrates one sub region MH, but the sub region MH may be provided in plurality.

Various electronic modules ELM (see FIG. 13) may be arranged so as to correspond to the sub region MH. For example, the electronic module ELM (FIG. 13) may include at least one of a camera, a speaker, a light detection sensor, or a heat detection sensor. In one or more embodiments, the electronic apparatus EA may include an electronic module ELM (FIG. 13) which captures an external image using visible light passing through the sub region MH or determines an approach of an external object using infrared light. The electronic module ELM (FIG. 13) may include a plurality of components and is not limited to any one embodiment.

Referring to FIG. 13, the electronic apparatus EA may include a display device DD. In addition, the electronic apparatus EA may further include an electronic module ELM, a window member WM, and a housing HAU.

The window member WM may cover an entire exterior of the electronic apparatus EA. The window member WM may include a transmission region TA and a bezel region BZA. A front surface of the window member WM including the transmission region TA and the bezel region BZA may correspond to a front surface of the electronic apparatus EA. The transmission region TA may correspond to the display region EA-DA of the electronic apparatus EA illustrated in FIG. 12, and the bezel region BZA may correspond to the non-display region EA-NDA of the electronic apparatus EA illustrated in FIG. 12.

The transmission region TA may be an optically transparent region. The bezel region BZA may be a region having a relatively lower light transmittance than the transmission region TA. The bezel region BZA may not be an optically transparent and may have a color. The bezel region BZA may be adjacent to the transmission region TA and surround the transmission region TA. The bezel region BZA may define the shape of the transmission region TA. However, embodiments of the present disclosure are not limited to what is illustrated, for example, the bezel region BZA may be arranged adjacent to only one side of the transmission region TA, or a portion thereof may not be provided.

The housing HAU may include a material having relatively high rigidity. For example, in one or more embodiments, the housing HAU may include a frame and/or a plate composed of glass, plastic, or metal. The frame and/or the plate may be provided in plurality. The housing HAU may provide an accommodating space. The display device DD may be accommodated in the accommodating space to be protected from an external impact.

The display device DD may include the same configuration as at least one selected from among the display devices DD, DD-TD, DD-a, DD-b, and DD-c, according to one or more embodiments, described with reference to FIGS. 1, 2, and 7 to 10. The display device DD may include the light-emitting element ED described with reference to FIG. 3 to FIG. 6. Therefore, the electronic apparatus EA including the display device DD according to one or more embodiments may exhibit excellent or suitable reliability.

In the display device DD, an active region DM-AA, a peripheral region DM-NAA, and a module region DM-MH may be defined. The active region DM-AA may overlap the display region EA-DA illustrated in FIG. 12 and the peripheral region DM-NAA may overlap the non-display region EA-NDA illustrated in FIG. 12. The module region DM-MH may overlap the sub region MH illustrated in FIG. 12.

The active region DM-AA may be a region activated in response to an electrical signal. The peripheral region DM-NAA may be a region arranged adjacent to at least one side of the active region DM-AA. The active region DM-AA may include a non-light emitting region NPXA and light emitting regions PXA-R, PXA-G, and PXA-B, illustrated in FIG. 1. The peripheral region DM-NAA may be arranged to surround the active region DM-AA. However, embodiments of the present disclosure are not limited thereto, for example, in one or more embodiments, a portion of the peripheral region DM-NAA may not be provided. In the peripheral region DM-NAA, a driving circuit, a driving line, and/or the like for driving the active region DM-AA may be arranged.

An optical signal such as visible light or infrared light may travel through the module region DM-MH. In one or more embodiments, the module region DM-MH may be arranged in the active region DM-AA. In one or more embodiments, the module region DM-MH may be surrounded by the peripheral region DM-NAA or may be surrounded by the active region DM-AA and peripheral region DM-NAA.

The electronic module ELM may be an electronic component that outputs or receives an optical signal. The electronic module ELM may include a camera module and/or a proximity sensor. The camera module may capture an external image through the module region DM-MH. However, embodiments of the present disclosure are not limited thereto, for example, in one or more embodiments, the electronic module ELM may further include a built-in module and/or an external module. The built-in module may include a sensor module, an antenna module, and/or an audio output module. The external module may include a light module and/or a communication module.

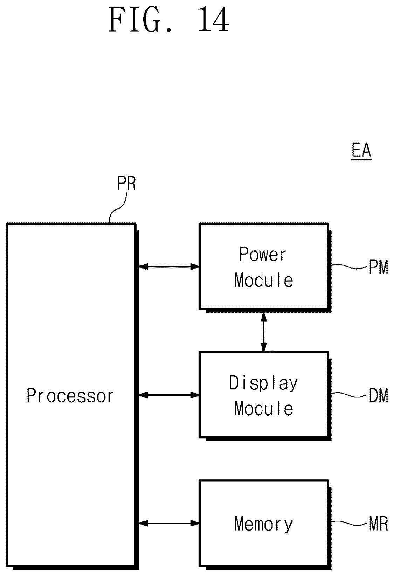

FIG. 14 is a block diagram of an electronic apparatus according to one or more embodiments of the present disclosure. Referring to FIG. 14, an electronic apparatus EA according to one or more embodiments may include a display module DM, a processor PR, a memory MR, and a power module PM.

The processor PR may include at least one of a central processing unit (CPU), an application processor (AP), a graphic processing unit (GPU), a communication processor (CP), an image signal processor (ISP), or a controller.

The memory MR may store data information desired or required for the operation of the processor PR and/or the display module DM. When the processor PR executes an application stored in the memory MR, image data signals and/or input control signals are transmitted to the display module DM, and the display module DM may process the received signal and output image information through a display screen.

The power module PM may include a power supply module such as a power adapter or a battery device, and a power conversion module that converts power supplied by the power supply module to generate power desired or required for the operation of the electronic apparatus EA.

At least one of the components of the electronic apparatus EA described above may be included in the display device according to the above-described embodiments. In one or more embodiments, some of the individual modules functionally included in one module may be included in the display device, and others may be separately provided from the display device. For example, the display device may include a display module DM, and the processor PR, the memory MR and the power module PM may be provided in the form of other devices within the electronic apparatus EA, rather than the display device.

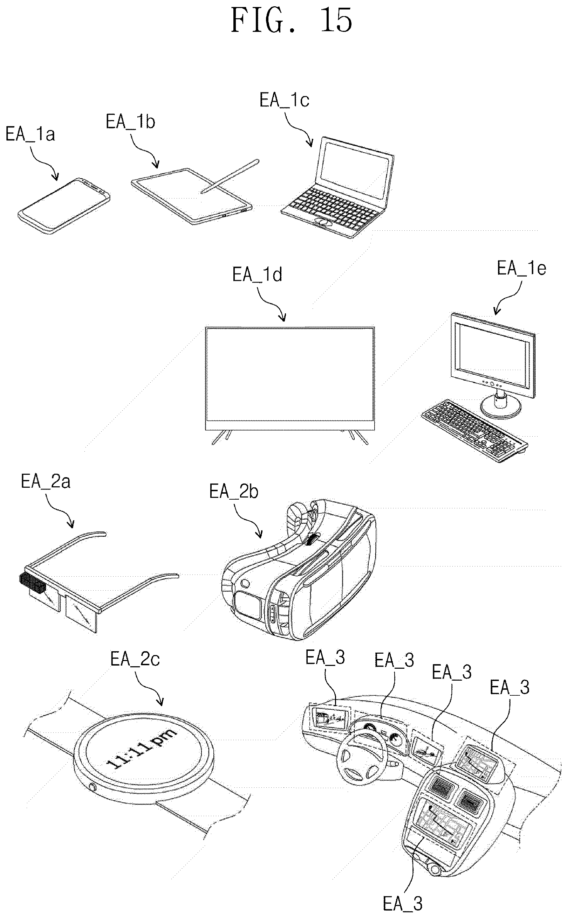

FIG. 15 shows schematic views of electronic apparatuses according to one or more embodiments of the present disclosure. Referring to FIG. 15, one or more suitable electronic apparatuses to which the display device according to one or more embodiments is applied may include an electronic apparatus for displaying images, such as a smart phone EA_1a, a tablet PC EA_1b, a laptop computer EA_1c, a TV EA_1d, and a desk monitor EA_1e, a wearable electronic apparatus including a display module such as a smart glasses EA_2a, a head mounted display EA_2b, and a smart watch EA_2c, and a vehicle electronic apparatus EA_3 including a display module such as a center information display (CID) and a room mirror display arranged on an instrument panel, a center fascia, or a dashboard of a vehicle.

Hereinafter, with reference to Examples and Comparative Examples, the monoamine compound according to one or more embodiments of the present disclosure and the light-emitting element according to one or more embodiments will be described in more detail. In addition, Examples shown herein are exemplified only for helping the understanding of the disclosure, and the scope of the disclosure is not limited thereto.

### Examples

### 1. Synthesis of monoamine compound according to one or more embodiments

A synthetic method of the monoamine compounds according to one or more embodiments will be explained by exemplifying the synthetic methods of Monoamine Compounds 6, 9, 11, 15, 17, 68, 75, 83, 86, 141, 157, 166, and 170. In addition, the synthetic methods of the monoamine compounds explained hereinafter are illustrative embodiments, and the synthetic method of the monoamine compound according to one or more embodiments of the present disclosure are not limited to these illustrative embodiments.

### (1) Synthesis of Monoamine Compound 6

Monoamine Compound 6 according to one or more embodiments may be synthesized according to, for example, steps in Reaction Scheme 1.

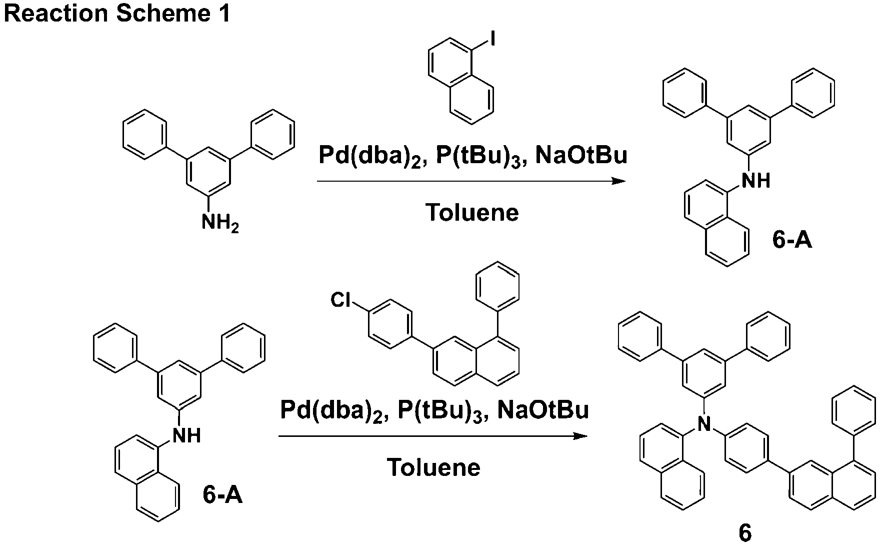

### Synthesis of Intermediate Compound 6-A

In an Ar atmosphere, [1,1':3',1"-terphenyl]-5'-amine (10.0 g), 1-iodonaphthalene (10.3 g), bis(dibenzylideneacetone)palladium(0) (Pd(dba)₂, 0.3 g), and sodium tert-butoxide (NaOtBu, 5.8 g) were put into a 500 mL three-neck flask, and dissolved in toluene (200 mL), then tri-tert-butylphosphine (P(tBu)₃, 2.0 M in toluene, 0.4 mL) was added, and the resultant mixture was stirred at room temperature for about 8 hours. Water was added to the solution, and an organic layer was obtained through extraction with dichloromethane (CH₂Cl₂). The obtained organic layers were all together dried over anhydrous magnesium sulfate (MgSO₄), and then a solvent was removed by distillation under reduced pressure. The obtained crude product was purified through silica gel column chromatography and recrystallization to obtain 6.8 g of Intermediate Compound 6-A (yield of 45%). A molecular weight of Intermediate Compound 6-A, as measured by Fast atom bombardment-Mass spectroscopy (FAB-MS), was 371.

### Synthesis of Compound 6

In an Ar atmosphere, Intermediate Compound 6-A (5.0 g), 7-(4-chlorophenyl)-1-phenylnaphthalene (4.3 g), Pd(dba)₂ (0.07 g), and NaOtBu (1.9 g) were put into a 300 mL three-neck flask, and dissolved in toluene (100 mL), then P(tBu)₃ (2.0 M in toluene, 0.1 mL) was added, and the resultant mixture was heated under reflux for about 4 hours. After cooling, water was added to the solution, and an organic layer was obtained through extraction with CH₂Cl₂. The obtained organic layers were all together dried over anhydrous MgSO₄, and then a solvent was removed by distillation under reduced pressure. The obtained crude product was purified through silica gel column chromatography to obtain 7.4 g of Compound 6 (yield of 85%). A molecular weight of Compound 6, as measured by FAB-MS, was 649.

### (2) Synthesis of Monoamine Compound 9

Monoamine Compound 9 according to one or more embodiments may be synthesized according to, for example, steps in Reaction Scheme 2.

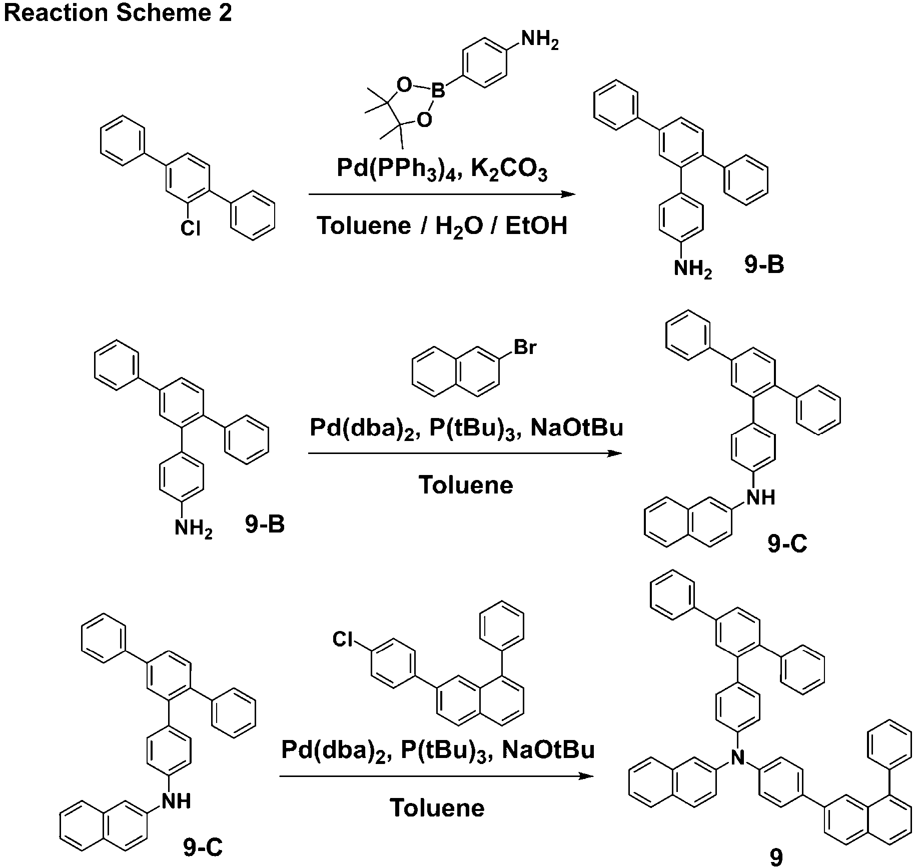

### Synthesis of Intermediate Compound 9-B

In an Ar atmosphere, 2'-chloro-1,1':4',1"-terphenyl (20.0 g), 4-(4,4,5,5-tetramethyl-1,3,2-dioxaborolan-2-yl)aniline (16.6 g), tetrakis(triphenylphosphine)palladium(0) (Pd(PPh₃)₄, 4.4 g), and potassium carbonate (K₂CO₃, 20.9 g) were put into a 1 L three-neck flask, and dissolved in a mixed solvent of toluene, water, and ethanol (weight ratio of 10:2:1, 400 mL), and the resultant mixture was heated and stirred at about 80 °C for about 16 hours. After cooling, water was added to the solution, and an organic layer was obtained through extraction with CH₂Cl₂. The obtained organic layers were all together dried over anhydrous MgSO₄, and then a solvent was removed by distillation under reduced pressure. The obtained crude product was purified through silica gel column chromatography to obtain 15.7 g of Intermediate Compound 9-B (yield of 65%). A molecular weight of Intermediate Compound 9-B, as measured by FAB-MS, was 321.

### Synthesis of Intermediate Compound 9-C

7.6 g of Intermediate Compound 9-C (yield of 55%) was obtained in substantially the same manner as synthesis of Intermediate Compound 6-A except that Intermediate Compound 9-B (10.0 g) was used in place of [1,1':3',1"-terphenyl]-5'-amine (10.0 g), and 2-bromonaphthalene (6.4 g) was used in place of 1-iodonaphthalene (10.3 g). A molecular weight of Intermediate Compound 9-C, as measured by FAB-MS, was 447.

### Synthesis of Compound 9

6.8 g of Compound 9 (yield of 84%) was obtained in substantially the same manner as synthesis of Compound 6 except that Intermediate 9-C (5.0 g) was used in place of Intermediate Compound 6-A (5.0 g) and 7-(4-chlorophenyl)-1-phenylnaphthalene (3.6 g) was used in place of 7-(4-chlorophenyl)-1-phenylnaphthalene (4.3 g). A molecular weight of Compound 9, as measured by FAB-MS, was 725.

### (3) Synthesis of Monoamine Compound 11

Monoamine Compound 11 according to one or more embodiments may be synthesized according to, for example, steps in Reaction Scheme 3.

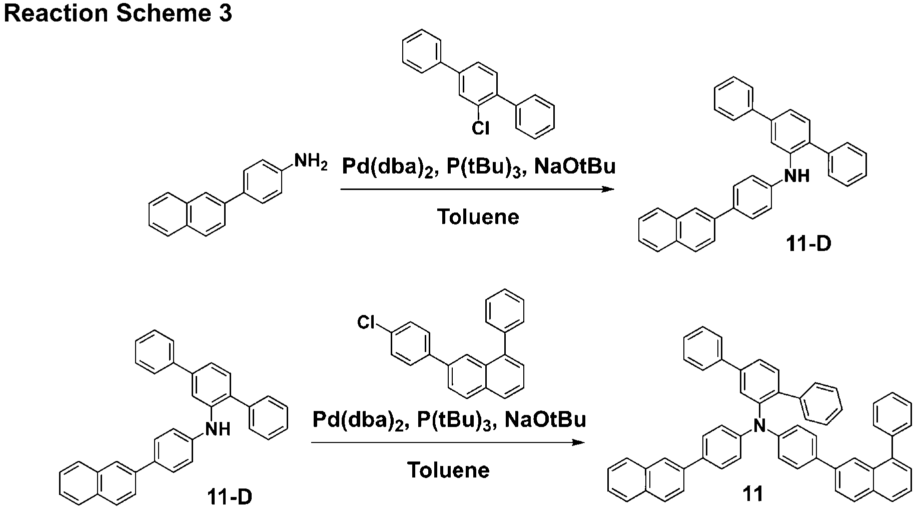

### Synthesis of Intermediate Compound 11-D

In an Ar atmosphere, 4-(naphthalen-2-yl)aniline (10.0 g), 2'-chloro-1,1':4',1"-terphenyl (12.1 g), Pd(dba)₂ (0.26 g), and NaOtBu (6.5 g) were put into a 500 mL three-neck flask, and dissolved in toluene (200 mL), then P(tBu)₃ (2.0 M in toluene, 0.4 mL) was added, and the resultant mixture was heated under reflux for about 8 hours. After cooling, water was added to the solution, and an organic layer was obtained through extraction with CH₂Cl₂. The obtained organic layers were all together dried over anhydrous MgSO₄, and then a solvent was removed by distillation under reduced pressure. The obtained crude product was purified through silica gel column chromatography to obtain 15.9 g of Intermediate Compound 11-D (yield of 78%). A molecular weight of Intermediate Compound 11-D, as measured by FAB-MS, was 447.

### Synthesis of Compound 11

6.0 g of Compound 11 (yield of 75%) was obtained in substantially the same manner as synthesis of Compound 6 except that Intermediate Compound 11-D (5.0 g) was used in place of Intermediate 6-A (5.0 g) and 7-(4-chlorophenyl)-1-phenylnaphthalene (3.5 g) was used in place of 7-(4-chlorophenyl)-1-phenylnaphthalene (4.3 g). A molecular weight of Compound 11, as measured by FAB-MS, was 725.

### (4) Synthesis of Monoamine Compound 15

Monoamine Compound 15 according to one or more embodiments may be synthesized according to, for example, steps in Reaction Scheme 4.

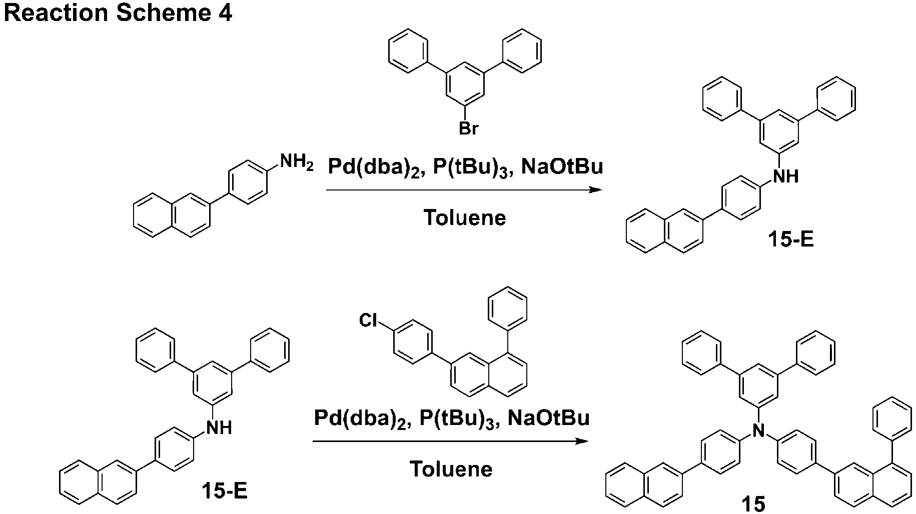

### Synthesis of Intermediate Compound 15-E

12.4 g of Intermediate Compound 15-E (yield of 61%) was obtained in substantially the same manner as synthesis of Intermediate Compound 6-A except that 4-(naphthalen-2-yl)aniline (10.0 g) was used in place of [1,1':3',1"-terphenyl]-5'-amine (10.0 g) and 5'-bromo-1,1':3',1"-terphenyl (14.1 g) was used in place of 1-iodonaphthalene (10.3 g). A molecular weight of Intermediate Compound 15-E, as measured by FAB-MS, was 447.

### Synthesis of Compound 15

6.5 g of Compound 15 (yield of 81%) was obtained in substantially the same manner as synthesis of Compound 6 except that Intermediate Compound 15-E (5.0 g) was used in place of Intermediate Compound 6-A (5.0 g) and 7-(4-chlorophenyl)-1-phenylnaphthalene (3.5 g) was used in place of 7-(4-chlorophenyl)-1-phenylnaphthalene (4.3 g). A molecular weight of Compound 15, as measured by FAB-MS, was 725.

### (5) Synthesis of Monoamine Compound 17

Monoamine Compound 17 according to one or more embodiments may be synthesized according to, for example, steps in Reaction Scheme 5.

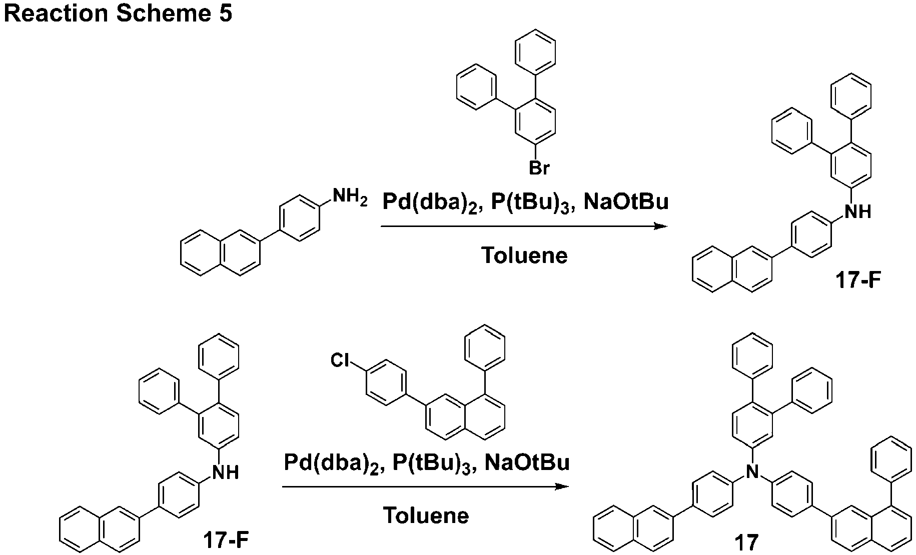

### Synthesis of Intermediate Compound 17-F

11.8 g of Intermediate Compound 17-F (yield of 58%) was obtained in substantially the same manner as synthesis of Intermediate Compound 6-A except that 4-(naphthalen-2-yl)aniline (10.0 g) was used in place of [1,1':3',1"-terphenyl]-5'-amine (10.0 g) and 4'-bromo-1,1':2',1"-terphenyl (14.1 g) was used in place of 1-iodonaphthalene (10.3 g). A molecular weight of Intermediate Compound 17-F, as measured by FAB-MS, was 447.

### Synthesis of Compound 17

6.4 g of Compound 17 (yield of 80%) was obtained in substantially the same manner as synthesis of Compound 6 except that Intermediate Compound 17-F (5.0 g) was used in place of Intermediate 6-A (5.0 g) and 7-(4-chlorophenyl)-1-phenylnaphthalene (3.5 g) was used in place of 7-(4-chlorophenyl)-1-phenylnaphthalene (4.3 g). A molecular weight of Compound 17, as measured by FAB-MS, was 725.

### (6) Synthesis of Monoamine Compound 68

Monoamine Compound 68 according to one or more embodiments may be synthesized according to, for example, steps in Reaction Scheme 6.

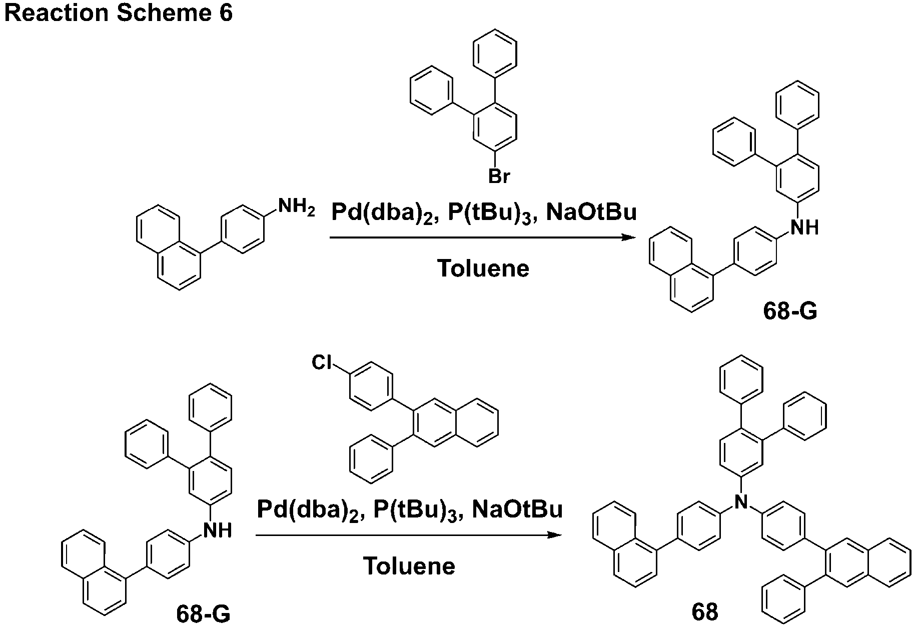

### Synthesis of Intermediate Compound 68-G

10.4 g of Intermediate Compound 68-G (yield of 51%) was obtained in substantially the same manner as synthesis of Intermediate Compound 6-A except that 4-(naphthalen-1-yl)aniline (10.0 g) was used in place of [1,1':3',1"-terphenyl]-5'-amine (10.0 g) and 4'-bromo-1,1':2',1"-terphenyl (14.1 g) was used in place of 1-iodonaphthalene (10.3 g). A molecular weight of Intermediate Compound 68-G, as measured by FAB-MS, was 447.

### Synthesis of Compound 68

6.8 g of Compound 68 (yield of 85%) was obtained in substantially the same manner as synthesis of Compound 6 except that Intermediate Compound 68-G (5.0 g) was used in place of Intermediate Compound 6-A (5.0 g) and 2-(4-chlorophenyl)-3-phenylnaphthalene (3.5 g) was used in place of 7-(4-chlorophenyl)-1-phenylnaphthalene (4.3 g). A molecular weight of Compound 68, as measured by FAB-MS, was 725.

### (7) Synthesis of Monoamine Compound 75

Monoamine Compound 75 according to one or more embodiments may be synthesized according to, for example, steps in Reaction Scheme 7.

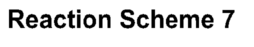

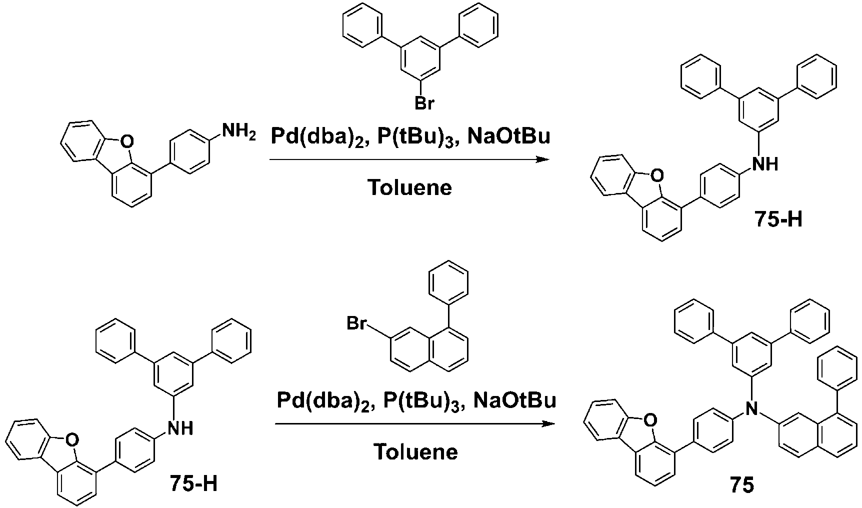

### Synthesis of Intermediate Compound 75-H

10.7 g of Intermediate Compound 75-H (yield of 57%) was obtained in substantially the same manner as synthesis of Intermediate Compound 6-A except that 4-(dibenzofuran-4-yl)aniline (10.0 g) was used in place of [[1,1':3',1"-terphenyl]-5'-amine (10.0 g) and 5'-bromo-1,1':3',1"-terphenyl (11.9 g) was used in place of 1-iodonaphthalene (10.3 g). A molecular weight of Intermediate Compound 75-H, as measured by FAB-MS, was 487.

### Synthesis of Compound 75

4.7 g of Compound 75 (yield of 67%) was obtained in substantially the same manner as synthesis of Compound 6 except that Intermediate 75-H (5.0 g) was used in place of Intermediate Compound 6-A (5.0 g) and 7-bromo-1-phenylnaphthalene (2.9 g) was used in place of 7-(4-chlorophenyl)-1-phenylnaphthalene (4.3 g). A molecular weight of Compound 75, as measured by FAB-MS, was 689.

### (8) Synthesis of Monoamine Compound 83

Monoamine Compound 83 according to one or more embodiments may be synthesized according to, for example, a step in Reaction Scheme 8.

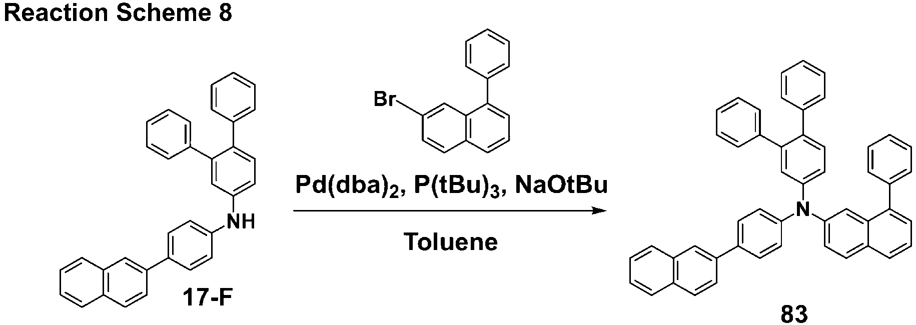

5.8 g of Compound 83 (yield of 80%) was obtained in substantially the same manner as synthesis of Compound 6 except that Intermediate Compound 17-F (5.0 g) was used in place of Intermediate 6-A (5.0 g) and 7-bromo-1-phenylnaphthalene (3.2 g) was used in place of 7-(4-chlorophenyl)-1-phenylnaphthalene (4.3 g). A molecular weight of Compound 83, as measured by FAB-MS, was 649.

### (9) Synthesis of Monoamine Compound 86

Monoamine Compound 86 according to one or more embodiments may be synthesized according to, for example, steps in Reaction Scheme 9.

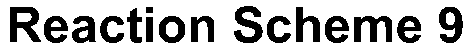

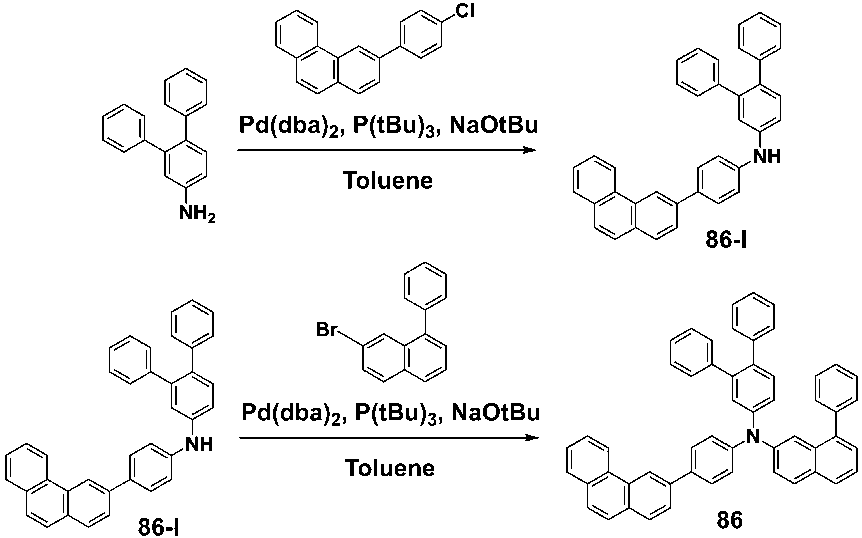

### Synthesis of Intermediate Compound 86-I

9.1 g of Intermediate Compound 86-I (yield of 45%) was obtained in substantially the same manner as synthesis of Intermediate Compound 11-D except that [1,1':2',1"-terphenyl]-4'-amine (10.0 g) was used in place of 4-(naphthalen-2-yl)aniline (10.0 g) and 3-(4-chlorophenyl)phenanthrene (11.7 g) was used in place of 2'-chloro-1,1':4',1"-terphenyl (12.1 g). A molecular weight of Intermediate Compound 86-I, as measured by FAB-MS, was 497.

### Synthesis of Compound 86

5.5 g of Compound 86 (yield of 79%) was obtained in substantially the same manner as synthesis of Compound 6 except that Intermediate Compound 86-I (5.0 g) was used in place of Intermediate Compound 6-A (5.0 g) and 7-bromo-1-phenylnaphthalene (2.8 g) was used in place of 7-(4-chlorophenyl)-1-phenylnaphthalene (4.3 g). A molecular weight of Compound 86, as measured by FAB-MS, was 699.

### (10) Synthesis of Monoamine Compound 141

Monoamine Compound 141 according to one or more embodiments may be synthesized according to, for example, steps in Reaction Scheme 10.

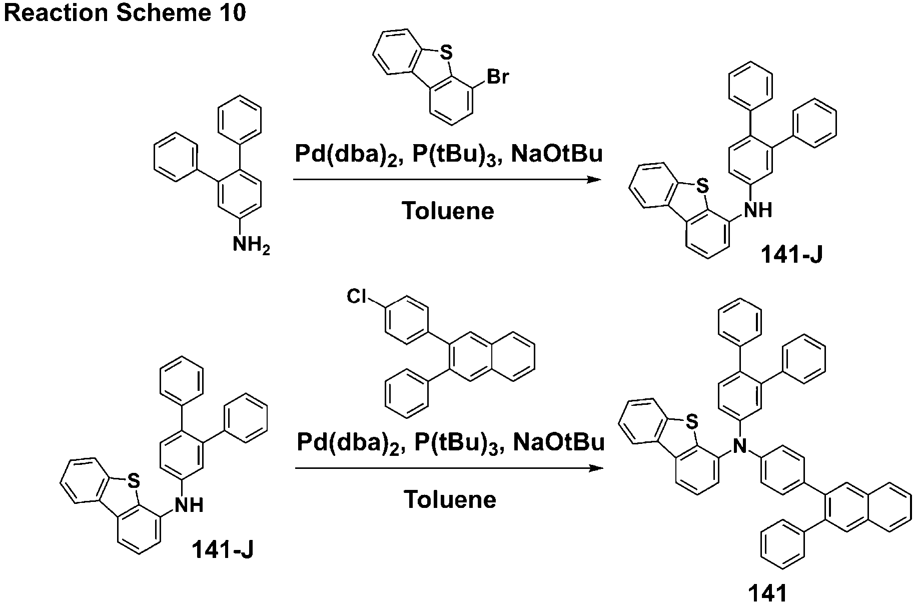

### Synthesis of Intermediate Compound 141-J

13.7 g of Intermediate Compound 141-J (yield of 79%) was obtained in substantially the same manner as synthesis of Intermediate Compound 6-A except that [1,1':2',1"-terphenyl]-4'-amine (10.0 g) was used in place of [1,1':3',1"-terphenyl]-5'-amine (10.0 g) and 4-bromodibenzothiophene (10.7 g) was used in place of 1-iodonaphthalene (10.3 g). A molecular weight of Intermediate Compound 141-J, as measured by FAB-MS, was 427.

### Synthesis of Compound 141

6.1 g of Compound 141 (yield of 74%) was obtained in substantially the same manner as synthesis of Compound 6 except that Intermediate Compound 141-J (5.0 g) was used in place of Intermediate Compound 6-A (5.0 g) and 2-(4-chlorophenyl)-3-phenylnaphthalene (3.7 g) was used in place of 7-(4-chlorophenyl)-1-phenylnaphthalene (4.3 g). A molecular weight of Compound 141, as measured by FAB-MS, was 705.

### (11) Synthesis of Monoamine Compound 157

Monoamine Compound 157 according to one or more embodiments may be synthesized according to, for example, steps in Reaction Scheme 11.

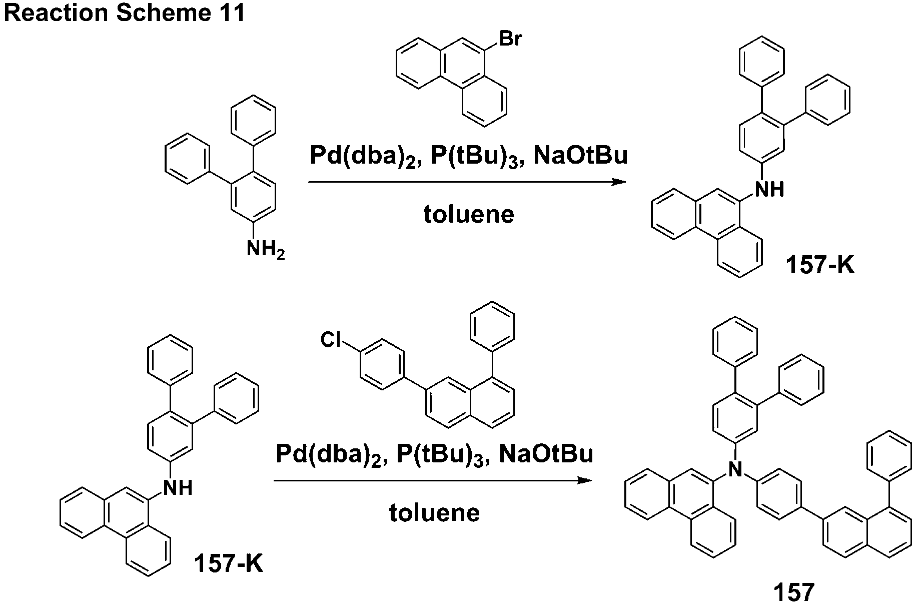

### Synthesis of Intermediate Compound 157-K

11.1 g of Intermediate Compound 157-K (yield of 65%) was obtained in substantially the same manner as synthesis of Intermediate Compound 6-A except that [1,1':2',1"-terphenyl]-4'-amine (10.0 g) was used in place of [1,1':3',1"-terphenyl]-5'-amine (10.0 g) and 9-bromophenanthrene (10.4 g) was used in place of 1-iodonaphthalene (10.3 g). A molecular weight of Intermediate Compound 157-K, as measured by FAB-MS, was 421.

### Synthesis of Compound 157

6.0 g of Compound 157 (yield of 73%) was obtained in substantially the same manner as synthesis of Compound 6 except that Intermediate Compound 157-K (5.0 g) was used in place of Intermediate Compound 6-A (5.0 g) and 7-(4-chlorophenyl)-1-phenylnaphthalene (3.8 g) was used in place of 7-(4-chlorophenyl)-1-phenylnaphthalene (4.3 g). A molecular weight of Compound 157, as measured by FAB-MS, was 699.

### (12) Synthesis of Monoamine Compound 166

Monoamine Compound 166 according to one or more embodiments may be synthesized according to, for example, steps in Reaction Scheme 12.

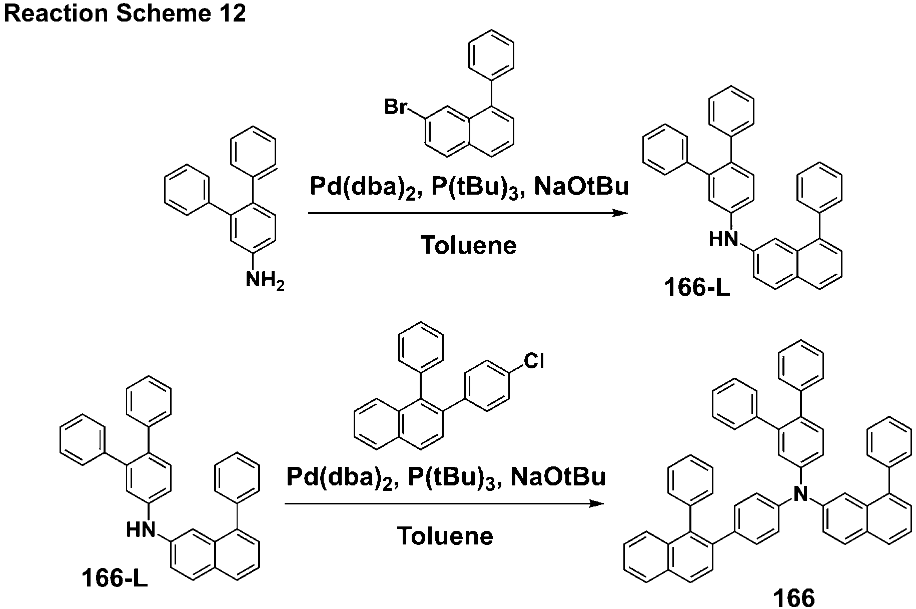

### Synthesis of Intermediate Compound 166-L

10.7 g of Intermediate Compound 166-L (yield of 59%) was obtained in substantially the same manner as synthesis of Intermediate Compound 6-A except that [1,1':2',1"-terphenyl]-4'-amine (10.0 g) was used in place of [1,1':3',1"-terphenyl]-5'-amine (10.0 g) and 7-bromo-1-phenylnaphthalene (11.5 g) was used in place of 1-iodonaphthalene (10.3 g). A molecular weight of Intermediate Compound 166-L, as measured by FAB-MS, was 447.

### Synthesis of Compound 166

6.4 g of Compound 166 (yield of 79%) was obtained in substantially the same manner as synthesis of Compound 6 except that Intermediate Compound 166-L (5.0 g) was used in place of Intermediate Compound 6-A (5.0 g) and 2-(4-chlorophenyl)-1-phenylnaphthalene (3.5 g) was used in place of 7-(4-chlorophenyl)-1-phenylnaphthalene (4.3 g). A molecular weight of Compound 166, as measured by FAB-MS, was 725.

### (13) Synthesis of Monoamine Compound 170

Monoamine Compound 170 according to one or more embodiments may be synthesized according to, for example, a step in Reaction Scheme 13.

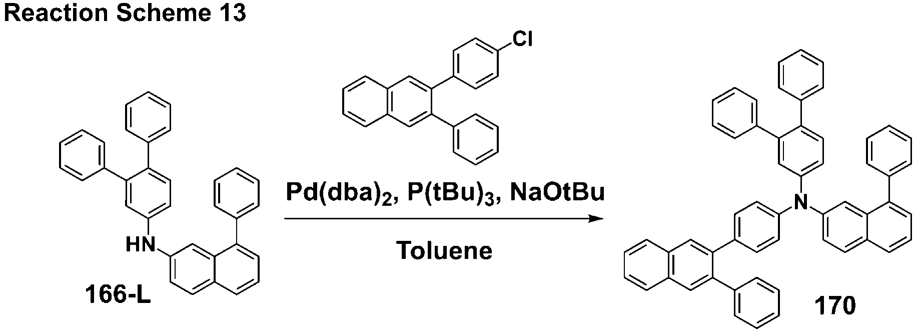

5.8 g of Compound 170 (yield of 72%) was obtained in substantially the same manner as synthesis of Compound 6 except that Intermediate Compound 166-L (5.0 g) was used in place of Intermediate Compound 6-A (5.0 g) and 2-(4-chlorophenyl)-3-phenylnaphthalene (3.5 g) was used in place of 7-(4-chlorophenyl)-1-phenylnaphthalene (4.3 g). A molecular weight of Compound 170, as measured by FAB-MS, was 725.

### 2. Manufacture and evaluation of light-emitting element

### (1) Manufacture of light-emitting element

Light-emitting elements respectively including the monoamine compounds according to Examples or Comparative Example Compounds in a hole transport layer were each manufactured by the following method. The light-emitting elements according to Examples 1 to 13 were manufactured respectively using Compounds 6, 9, 11, 15, 17, 68, 75, 83, 86, 141, 157, 166, and 170, which are each a monoamine compound according to an embodiment, as a material for a hole transport layer. The light-emitting elements according to Comparative Examples 1 to 17 were manufactured respectively using Comparative Example Compounds X-1 to X-17 as a material for a hole transport layer.

A glass substrate, on which an ITO having a thickness of about 150 nm was patterned as a first electrode, was subjected to ultrasonic cleaning for about 5 minutes each with isopropyl alcohol and ultrapure water. After the ultrasonic cleaning, the glass substrate was irradiated with UV for about 30 minutes, and subjected to ozone treatment. Thereafter, 2-TNATA was deposited to a thickness of about 60 nm to form a hole injection layer. Respective Example Compound or respective Comparative Example Compound was deposited to a thickness of about 30 nm on the hole injection layer to form a hole transport layer.

TBP and ADN were co-deposited to a thickness of about 25 nm on the hole transport layer to form an emission layer. TBP and ADN were co-deposited at a weight ratio of about 3:97. Thereafter, Alq₃, and LiF were sequentially deposited to a thickness of about 25 nm, and to a thickness of about 1 nm, respectively, to form an electron transport region. Next, Al was deposited to a thickness of about 100 nm to form a second electrode. The hole injection layer, the hole transport layer, the emission layer, the electron transport region, and the second electrode were each formed using a vacuum deposition device.

### Materials used in manufacture of light-emitting element

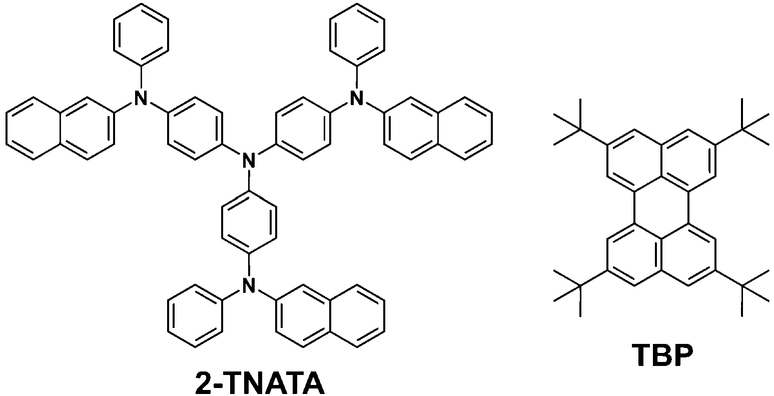

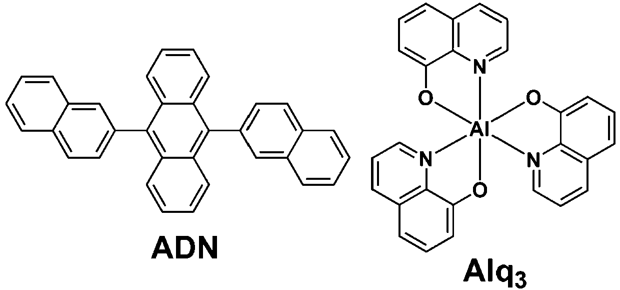

### Example Compounds

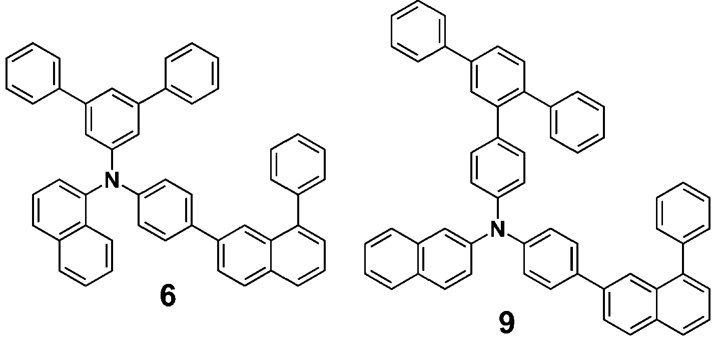

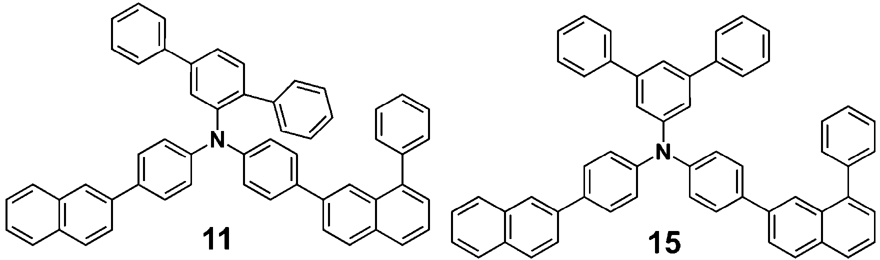

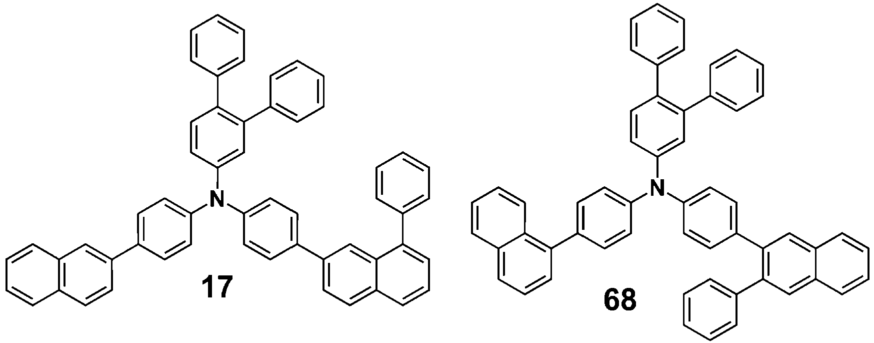

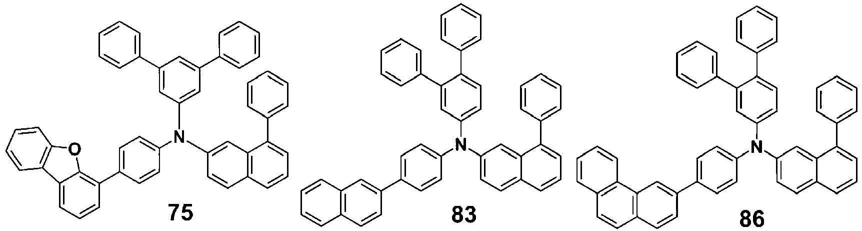

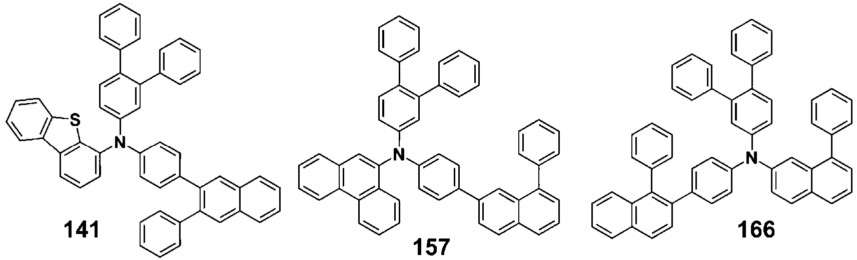

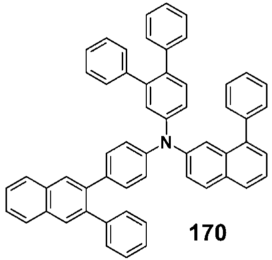

### Comparative Example Compounds

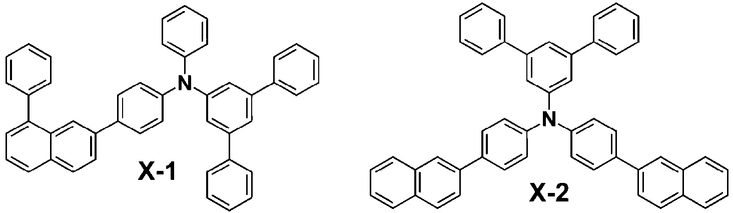

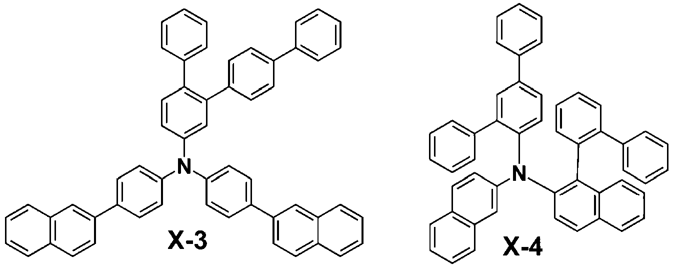

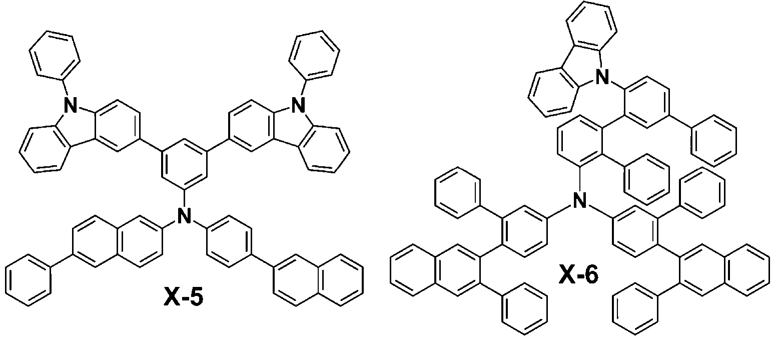

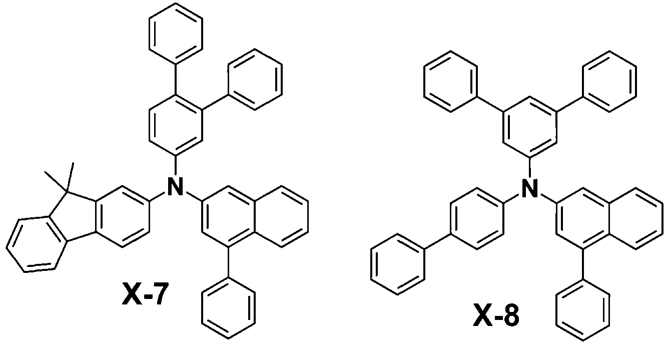

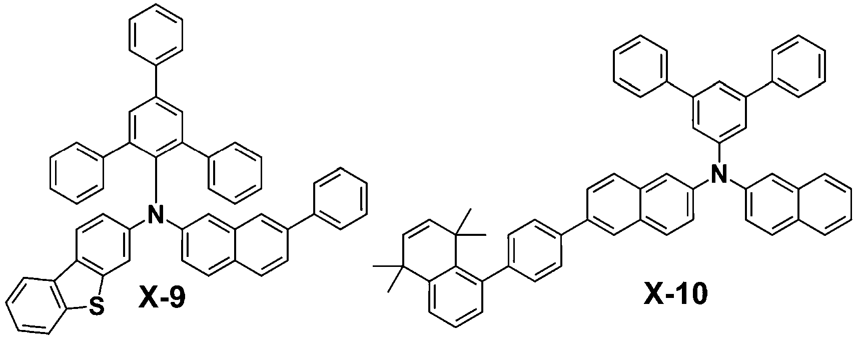

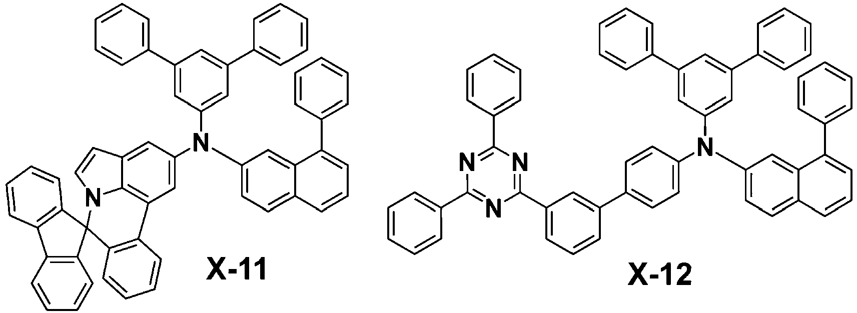

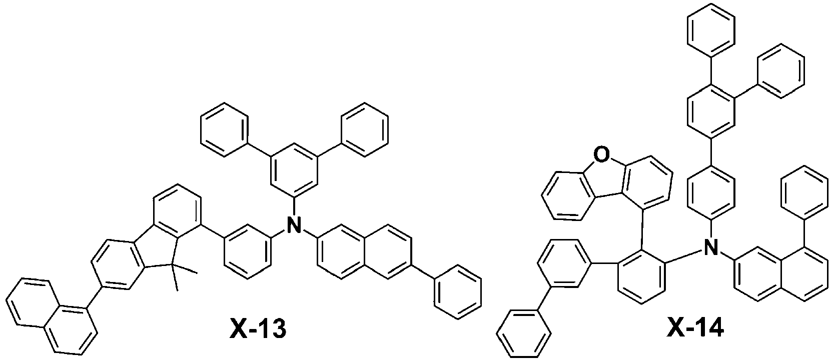

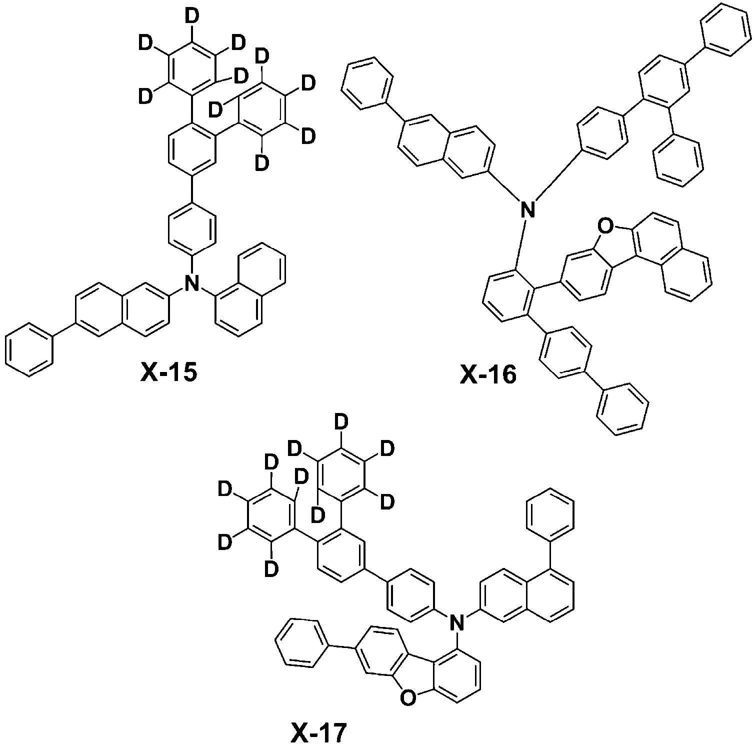

### (2) Evaluation of light-emitting elements

The light-emitting elements according to Examples and Comparative Examples were each evaluated, and the results are listed in Table 1. Light-emitting efficiency and lifespan at a current density of about 10 mA/cm² were evaluated using C9920-11, which is a luminance-orientation characteristic measurement device made by Hamamatsu Photonics Ltd. The lifespan (LT50) is obtained by measuring the time taken for the initial luminance to decrease from 100% to 50%. The relative light-emitting efficiency and lifespan values in Table 1 are relative values with respect to the values of Comparative Example 1 of 100%.

**Table 1**

| Element manufacturing examples | Hole transport layer | Luminous efficiency (@ 10mA/cm²) | Lifespan (LT50) |
|---|---|---|---|
| Example 1 | Compound 6 | 103% | 110% |
| Example 2 | Compound 9 | 102% | 110% |
| Example 3 | Compound 11 | 102% | 130% |
| Example 4 | Compound 15 | 104% | 130% |
| Example 5 | Compound 17 | 102% | 160% |
| Example 6 | Compound 68 | 105% | 140% |
| Example 7 | Compound 75 | 104% | 130% |
| Example 8 | Compound 83 | 103% | 150% |
| Example 9 | Compound 86 | 102% | 140% |
| Example 10 | Compound 141 | 105% | 120% |
| Example 11 | Compound 157 | 104% | 130% |
| Example 12 | Compound 166 | 104% | 120% |
| Example 13 | Compound 170 | 103% | 140% |
| Comparative Example 1 | Comparative Example Compound X-1 | 100% | 100% |
| Comparative Example 2 | Comparative Example Compound X-2 | 97% | 100% |
| Comparative Example 3 | Comparative Example Compound X-3 | 98% | 90% |
| Comparative Example 4 | Comparative Example Compound X-4 | 101% | 60% |
| Comparative Example 5 | Comparative Example Compound X-5 | 95% | 55% |
| Comparative Example 6 | Comparative Example Compound X-6 | 98% | 30% |
| Comparative Example 7 | Comparative Example Compound X-7 | 97% | 50% |
| Comparative Example 8 | Comparative Example Compound X-8 | 98% | 60% |
| Comparative Example 9 | Comparative Example Compound X-9 | 99% | 30% |
| Comparative Example 10 | Comparative Example Compound X-10 | 97% | 20% |
| Comparative Example 11 | Comparative Example Compound X-11 | 98% | 20% |
| Comparative Example 12 | Comparative Example Compound X-12 | 95% | 20% |
| Comparative Example 13 | Comparative Example Compound X-13 | 100% | 50% |
| Comparative Example 14 | Comparative Example Compound X-14 | 98% | 50% |
| Comparative Example 15 | Comparative Example Compound X-15 | 99% | 50% |
| Comparative Example 16 | Comparative Example Compound X-16 | 98% | 40% |
| Comparative Example 17 | Comparative Example Compound X-17 | 100% | 45% |

Referring to Table 1, it can be seen that, as compared to the light-emitting elements according to Comparative Examples 1 to 17, the light-emitting elements according to Examples 1 to 13 each exhibit higher light-emitting efficiency and longer lifespan. The light-emitting elements according to Examples 1 to 13 respectively include Compounds 6, 9, 11, 15, 17, 68, 75, 83, 86, 141, 157, 166, and 170, and Compounds 6, 9, 11, 15, 17, 68, 75, 83, 86, 141, 157, 166, and 170 are each a monoamine compound according to one or more embodiments of the present disclosure. Compounds 6, 9, 11, 15, 17, 68, 75, 83, 86, 141, 157, 166, and 170 each include first to third substituents, bonded to an amine group. The first substituent is a diphenylphenyl group, the second substituent is a phenylnaphthalenyl group, and the third substituent is an aryl group or a heteroaryl group. Therefore, it can be seen that the monoamine compound according to one or more embodiments improves efficiency and lifespan of the light-emitting element. It can be seen that the light-emitting element including the monoamine compound according to one or more embodiments exhibits high light-emitting efficiency and long lifespan characteristics.

The light-emitting element according to Comparative Example 1 includes Comparative Example Compound X-1, and Comparative Example Compound X-1 includes a diphenylphenyl group and a phenylnaphthalenyl group. However, in Comparative Example Compound X-1, the third substituent (that is Ar¹ in Formula 1) is an unsubstituted phenyl group having 6 ring-forming carbon atoms, and thus Comparative Example Compound X-1 differs from the monoamine compound according to an embodiment. Because Comparative Example Compound X-1 includes a phenyl group, the material stability thereof deteriorates, and the light-emitting element according to Comparative Example 1 exhibits relatively low light-emitting efficiency and short lifespan.

The light-emitting element according to Comparative Example 2 includes Comparative Example Compound X-2, and the light-emitting element according to Comparative Example 3 includes Comparative Example Compound X-3. Each of Comparative Example Compounds X-2 and X-3 includes a diphenylphenyl group. Each of Comparative Example Compounds X-2 and X-3 includes no phenylnaphthalenyl group, and includes an unsubstituted naphthalenyl group, and thus each of Comparative Example Compounds X-2 and X-3 differs from the monoamine compound according to an embodiment. Because each of Comparative Example Compounds X-2 and X-3 includes an unsubstituted naphthalenyl group, intermolecular stacking increases, and thus a hole transport balance decreases. Therefore, the light-emitting elements according to Comparative Examples 2 and 3 exhibit relatively low light-emitting efficiency and short lifespan.

The light-emitting element according to Comparative Example 4 includes Comparative Example Compound X-4, and Comparative Example Compound X-4 includes a diphenylphenyl group and a phenylnaphthalenyl group. However, in phenylnaphthalenyl group included in Comparative Example Compound X-4, the position of the phenyl group substituted with naphthalene is the position corresponding to R^{c} in Formula 1 above described, and thus Comparative Example Compound X-4 differs from the monoamine compound according to an embodiment. Therefore, Comparative Example Compound X-4 has decreased material stability due to steric hindrance in a molecule, and a deposition temperature increases during the manufacture of a light-emitting element. Therefore, the light-emitting element according to Comparative Example 4 exhibits relatively low light-emitting efficiency and short lifespan.

The light-emitting element according to Comparative Example 5 includes Comparative Example Compound X-5, and the light-emitting element according to Comparative Example 6 includes Comparative Example Compound X-6. Each of Comparative Example Compounds X-5 and X-6 includes a carbazole group, which is a nitrogen-containing heterocyclic group, and thus each of Comparative Example Compounds X-5 and X-6 differs from the monoamine compound according to an embodiment. Therefore, each of Comparative Example Compounds X-5 and X-6 has a disrupted charge transport balance, low thermal stability, and high deposition temperature. The light-emitting elements according to Comparative Examples 5 and 6 including hole transport layers, formed respectively using Comparative Example Compounds X-5 and X-6, have decreased stability of the hole transport layer. Therefore, the light-emitting elements according to Comparative Examples 5 and 6 each exhibit relatively low light-emitting efficiency and short lifespan.

The light-emitting element according to Comparative Example 7 includes Comparative Example Compound X-7, and the light-emitting element according to Comparative Example 13 includes Comparative Example Compound X-13. Each of Comparative Example Compounds X-7 and X-13 includes a fluorene group, and the fluorene group includes a *sp*³ carbon atom, and thus each of Comparative Example Compounds X-7 and X-13 differs from the monoamine compound according to an embodiment. Therefore, Comparative Example Compounds 7 and 13 each have low thermal stability, the light-emitting elements according to Comparative Examples 7 and 13, in which hole transport layers are formed respectively using Comparative Example Compounds X-7 and X-13, have reduced stability in the hole transport layer. Therefore, the light-emitting elements according to Comparative Examples 7 and 13 each exhibit relatively low light-emitting efficiency and short lifespan.

The light-emitting element according to Comparative Example 8 includes Comparative Example Compound X-8. In Comparative Example Compound X-8, a portion corresponding to Ar¹ in Formula 1 described above is an unsubstituted phenyl group or unsubstituted biphenyl group, and thus Comparative Example Compound X-8 differs from the monoamine compound according to an embodiment. The phenyl group is an aryl group having 6 ring-forming carbon atoms, and the biphenyl group is an aryl group in which a plurality of phenyl groups is connected by a direct linkage. Therefore, Comparative Example Compound X-8 has reduced hole transporting property and causes reduced efficiency. Therefore, the light-emitting element according to Comparative Example 8 exhibits relatively low light-emitting efficiency and short lifespan.

The light-emitting element according to Comparative Example 9 includes Comparative Example Compound X-9. Comparative Example Compound X-9 corresponds to a case where R^{a1} and R^{a5} are phenyl groups at the same time in Formula 1 above described, and thus Comparative Example Compound X-9 differs from the monoamine compound according to an embodiment. When R^{a1} and R^{a5} are phenyl groups at the same time, steric hindrance occurs, and Comparative Example Compound X-9 has reduced material stability due to the steric hindrance. Therefore, the light-emitting element according to Comparative Example 9 exhibits relatively low light-emitting efficiency and short lifespan.

The light-emitting element according to Comparative Example 10 includes Comparative Example Compound X-10. Comparative Example Compound X-10 includes a *sp*³ carbon atom, and thus Comparative Example Compound X-10 differs from the monoamine compound according to an embodiment. Therefore, Comparative Example Compound X-10 has low thermal stability, and the light-emitting element according to Comparative Examples 10, in which the hole transport layer is formed using Comparative Example Compound X-10, has reduced stability of the hole transport layer. Therefore, the light-emitting element according to Comparative Example 10 exhibits relatively low light-emitting efficiency and short lifespan.

The light-emitting element according to Comparative Example 11 includes Comparative Example Compound X-11, and the light-emitting element according to Comparative Example 12 includes Comparative Example Compound X-12. Each of Comparative Example Compounds X-11 and X-12 includes a nitrogen-containing heterocyclic group, and thus each of Comparative Example Compounds X-11 and X-12 differs from the monoamine compound according to an embodiment. Therefore, each of Comparative Example Compounds X-11 and X-12 has disrupted charge transport balance, and thus thermal stability is lowered and deposition temperature increases. The light-emitting elements according to Comparative Examples 11 and 12 in which hole transport layers are formed respectively using Comparative Example Compounds X-11 and X-12, have decreased stability in the hole transport layer. Therefore, the light-emitting elements according to Comparative Examples 11 and 12 each exhibit relatively low light-emitting efficiency and short lifespan.

The light-emitting element according to Comparative Example 14 includes Comparative Example Compound X-14. In Comparative Example Compound X-14, reference to Formula 1 above described, L¹ is a phenylene group (that is, L¹ is not a direct linkage) while R^{a2} and R^{a3} (or R^{a3} and R^{a4}) are each a phenyl group, and thus Comparative Example Compound X-14 differs from the monoamine compound according to an embodiment. Alternatively, in Comparative Example Compound X-14, a dibenzofuran group is bonded to an atom at ortho position with respect to N among ring-forming atoms of L² in Formula 1 above described, and thus Comparative Example Compound X-14 differs from the monoamine compound according to an embodiment. Therefore, in Comparative Example Compound X-14, steric hindrance occurs and material stability decreases. Therefore, the light-emitting element according to Comparative Example 14 exhibits relatively low light-emitting efficiency and short lifespan.

The light-emitting element according to Comparative Example 15 includes Comparative Example Compound X-15. In Comparative Example Compound X-15, reference to Formula 1 above described, L¹ is a phenylene group (that is, is not a direct linkage) while R^{a2} and R^{a3} (or R^{a3} and R^{a4}) are each a phenyl group, and thus Comparative Example Compound X-15 differs from the monoamine compound according to an embodiment. Because L¹ is a phenylene group, Comparative Example Compound X-15 includes a para-terphenyl moiety, and thus a deposition temperature increase. In addition, because Comparative Example Compound X-15 includes an unsubstituted naphthalenyl group, intermolecular stacking increases, and thus a hole transport balance decreases. Therefore, the light-emitting element according to Comparative Example 15 exhibits relatively low light-emitting efficiency and short lifespan.

The light-emitting element according to Comparative Example 16 includes Comparative Example Compound X-16, and Comparative Example Compound X-16 includes a diphenylphenyl group and a phenylnaphthalenyl group. However, in Comparative Example Compound X-16, a portion corresponding to Ar¹ in Formula 1 described above is a phenyl group, biphenyl group, or terphenyl group, and thus Comparative Example Compound X-16 differs from the monoamine compound according to an embodiment. The phenyl group is an aryl group having 6 ring-forming carbon atoms, and the biphenyl group and the terphenyl group are each an aryl group in which a plurality of phenyl groups is connected by a direct linkage. Therefore, Comparative Example Compound X-16 has reduced hole transporting property and causes a decrease in efficiency. Therefore, the light-emitting element according to Comparative Example 16 exhibits relatively low light-emitting efficiency and short lifespan.

The light-emitting element according to Comparative Example 17 includes Comparative Example Compound X-17, and Comparative Example Compound X-17 includes a diphenylphenyl group and a phenylnaphthalenyl group. However, in Comparative Example Compound X-17, in Formula 1 above described, L¹ is a phenylene group (that is, L¹ is not a direct linkage) while R^{a2} and R^{a3} (or R^{a3} and R^{a4}) are each a phenyl group, and thus Comparative Example Compound X-17 differs from the monoamine compound according to an embodiment. Therefore, in Comparative Example Compound X-17, steric hindrance occurs and material stability decreases. Therefore, the light-emitting element according to Comparative Example 17 exhibits relatively low light-emitting efficiency and short lifespan.

The electronic apparatus according to one or more embodiments includes a light-emitting element. In the light-emitting element according to one or more embodiments, the hole transport region includes the monoamine compound according to one or more embodiments. The monoamine compound according to one or more embodiments may include first to third substituents bonded to an amine group. The first substituent may be a diphenylphenyl group, the second substituent may be a phenylnaphthalenyl group, and the third substituent may be an aryl group or a heteroaryl group. In the third substituent, the aryl group may have 10 to 30 ring-forming carbon atoms. Therefore, the monoamine compound according to one or more embodiments may exhibit excellent or suitable material stability and excellent or suitable hole transporting property. The light-emitting element including the monoamine compound according to one or more embodiments may exhibit high light-emitting efficiency and long lifespan property. In one or more embodiments, the electronic apparatus including the light-emitting element may exhibit excellent or suitable reliability.

The light-emitting element according to one or more embodiments and the electronic apparatus including the same include the monoamine compound according to one or more embodiments, and thus high efficiency and long lifespan characteristics may be exhibited.

The monoamine compound according to one or more embodiments may contribute to improvements in high efficiency and long lifespan of the light-emitting element. For example, the monoamine compound may include first to third substituents bonded to an amine group: a diphenylphenyl group, a phenylnaphthalenyl group, and an aryl or heteroaryl group, where the aryl group may have 10 to 30 ring-forming carbon atoms. This specific molecular configuration enhances material stability and hole transport properties, which are critical for the performance of the light-emitting element. As demonstrated through the above analysis with various reference compounds, the monoamine compounds according to the disclosed embodiments avoid structural features-such as nitrogen-containing heterocycles, *sp*³ carbon atoms, and unsubstituted phenyl or biphenyl groups-that may degrade thermal stability, disrupt charge transport balance, or increase deposition temperature. By contrast, the disclosed monoamine compounds maintain a favorable molecular geometry and electronic structure that support efficient charge injection and transport. As a result, light-emitting elements incorporating these compounds exhibit superior luminous efficiency and extended operational lifespan, and the electronic apparatuses that include them demonstrate enhanced reliability and performance consistency over time.

As utilized herein, the terms "substantially," "about," or similar terms are used as terms of approximation and not as terms of degree, and are intended to account for the inherent deviations in measured or calculated values that would be recognized by those of ordinary skill in the art. "About" as used herein, is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" may mean within one or more standard deviations, or within ± 30%, 20%, 10%, or 5% of the stated value.

In the context of the present application and unless otherwise defined, the terms "use," "using," and "used" may be considered synonymous with the terms "utilize," "utilizing," and "utilized," respectively.

Any numerical range recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in this specification is intended to include all higher numerical limitations subsumed therein.

The light-emitting element, the display device, the electronic apparatus, the manufacturing apparatuses thereof, or any other relevant apparatuses/devices or components according to embodiments of the present disclosure described herein may be implemented utilizing any suitable hardware, firmware (e.g., an application-specific integrated circuit), software, or a combination of software, firmware, and hardware. For example, the various components of the device may be formed on one integrated circuit (IC) chip or on separate IC chips. Further, the various components of the device may be implemented on a flexible printed circuit film, a tape carrier package (TCP), a printed circuit board (PCB), or formed on one substrate. Further, the various components of the device may be a process or thread, running on one or more processors, in one or more computing devices, executing computer program instructions and interacting with other system components for performing the various functionalities described herein. The computer program instructions are stored in a memory which may be implemented in a computing device using a standard memory device, such as, for example, a random-access memory (RAM). The computer program instructions may also be stored in other non-transitory computer readable media such as, for example, a CD-ROM, flash drive, or the like. Also, a person of skill in the art should recognize that the functionality of various computing devices may be combined or integrated into a single computing device, or the functionality of a particular computing device may be distributed across one or more other computing devices without departing from the scope of the embodiments of the present disclosure.

In the present disclosure, each suitable feature of the various embodiments of the disclosure may be combined or combined with each other, partially or entirely, and may be technically interlocked and operated in various suitable ways, and each embodiment may be implemented independently of each other or in conjunction with each other in any suitable manner unless otherwise stated or implied.

Hitherto, although one or more embodiments of the present disclosure have been described with reference to example embodiments, those skilled in the art or having ordinary knowledge of the art will understand that one or more suitable modifications and changes may be made without departing from the technical idea or features of the present disclosure as described in the appended claims.

Accordingly, the technical scope of the present disclosure is not limited to what is set forth in the detailed description of the disclosure, but should be defined by the appended claims and equivalents thereof.

## Claims

1. A monoamine compound represented by Formula 1: 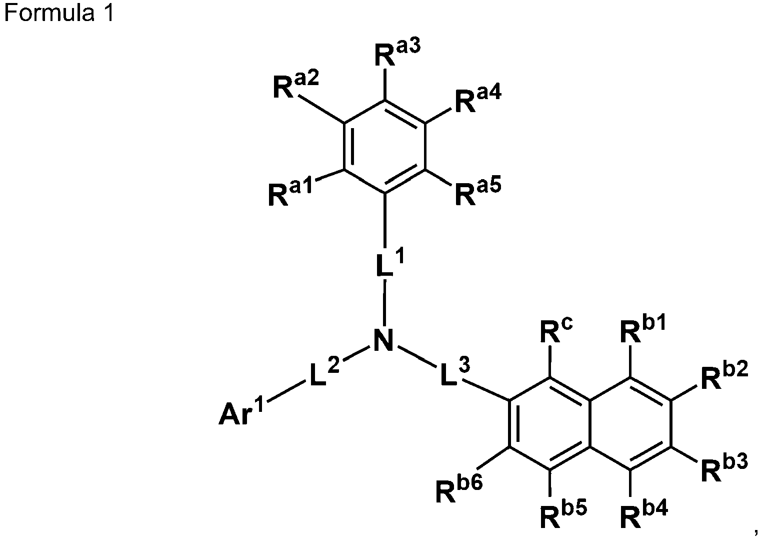 wherein, in Formula 1,
L¹ to L³ are each independently a direct linkage, a substituted or unsubstituted arylene group having 6 to 30 ring-forming carbon atoms, or a substituted or unsubstituted heteroarylene group having 5 to 30 ring-forming carbon atoms,
atoms at an ortho position with respect to N in L¹ to L³ are each independently bonded to hydrogen or deuterium,
Ar¹ is a substituted or unsubstituted aryl group having 10 to 30 ring-forming carbon atoms or a substituted or unsubstituted heteroaryl group having 5 to 30 ring-forming carbon atoms,
a case where Ar¹ is an aryl group in which a plurality of phenyl groups is connected by a direct linkage is excluded,
at least two selected from among R^{a1} to R^{a5} are each a substituted or unsubstituted phenyl group, and the rest are each independently hydrogen, deuterium, a halogen, a substituted or unsubstituted alkyl group having 1 to 15 carbon atoms, a substituted or unsubstituted aryl group having 6 to 30 ring-forming carbon atoms, or a substituted or unsubstituted heteroaryl group having 5 to 30 ring-forming carbon atoms,
a case where R^{a1} and R^{a5} are each a phenyl group at the same time is excluded,
R^{c} is hydrogen, deuterium, or fluorine,
at least one selected from among R^{b1} to R^{b6} is a substituted or unsubstituted phenyl group, and the rest are each independently hydrogen, deuterium, a halogen, a substituted or unsubstituted alkyl group having 1 to 15 carbon atoms, a substituted or unsubstituted aryl group having 6 to 30 ring-forming carbon atoms, or a substituted or unsubstituted heteroaryl group having 5 to 30 ring-forming carbon atoms,
when R^{a2} and R^{a3} are each a phenyl group or R^{a3} and R^{a4} are each a phenyl group, then L¹ is a direct linkage,
the monoamine compound comprises no nitrogen-containing heterocyclic group,
the monoamine compound comprises no cyclic group comprising a carbon atom which comprises *sp*³ hybrid orbitals, and
any hydrogen of the monoamine compound is optionally substituted with deuterium.

2. The monoamine compound of claim 1, wherein the monoamine compound represented by Formula 1 is represented by any one selected from among Formula 1-A1 to Formula 1-A4: 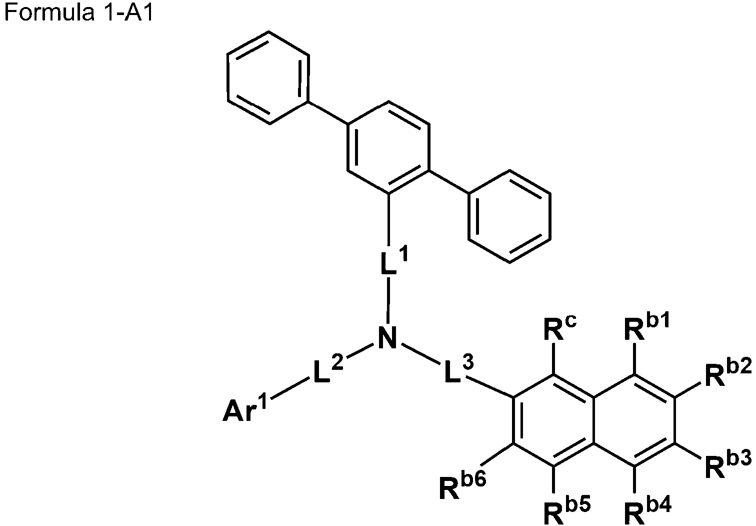 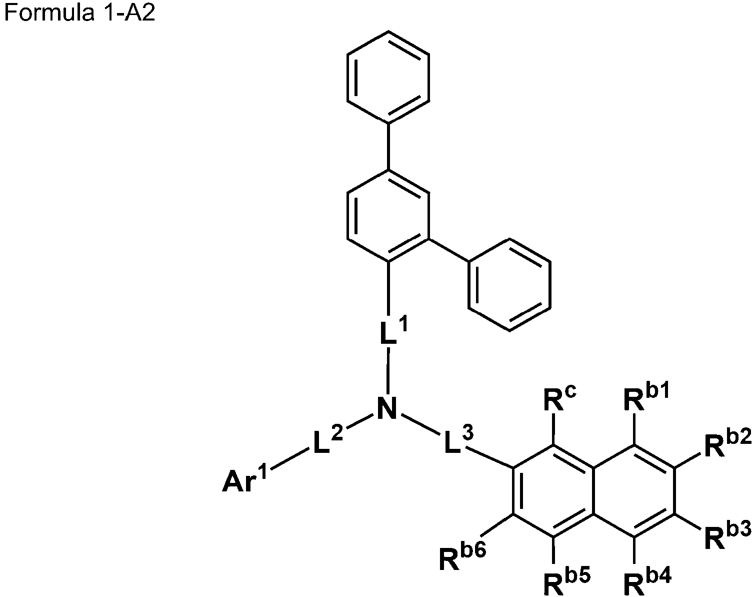 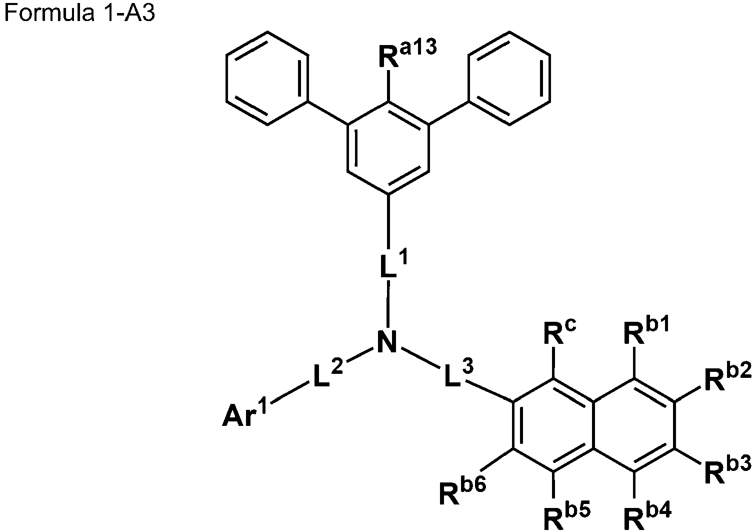 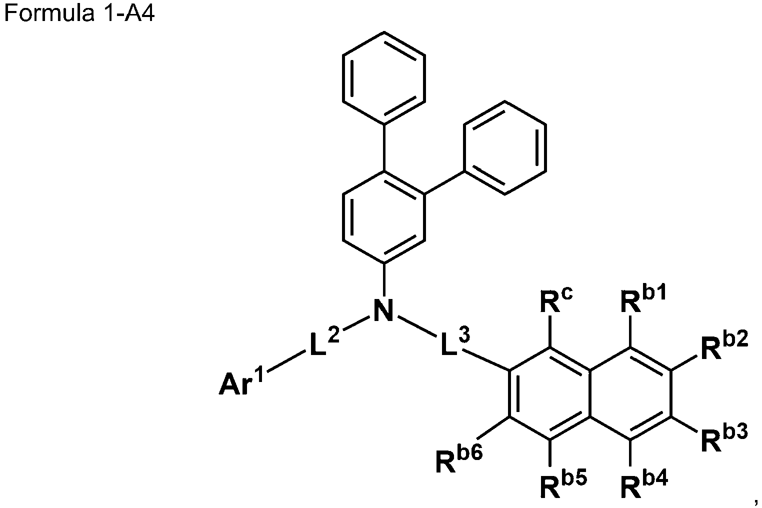 in Formula 1-A3,
R^{a13} being hydrogen or fluorine, and
in Formula 1-A1 to Formula 1-A4,
L¹ to L³, Ar¹, R^{c}, and R^{b1} to R^{b6} being the same as defined in Formula 1.

3. The monoamine compound of claim 2, wherein in Formula 1-A1 to Formula 1-A4:
at least one selected from among L¹ to L³ is a substituted or unsubstituted phenylene group, and
the rest are direct linkages.

4. The monoamine compound of claim 1, wherein the monoamine compound represented by Formula 1 is represented by any one selected from among Formula 1-B1 to Formula 1-B6: 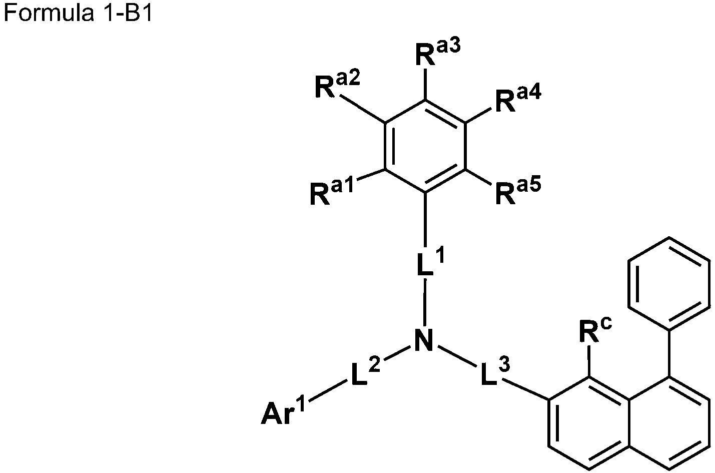 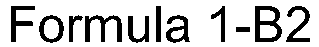 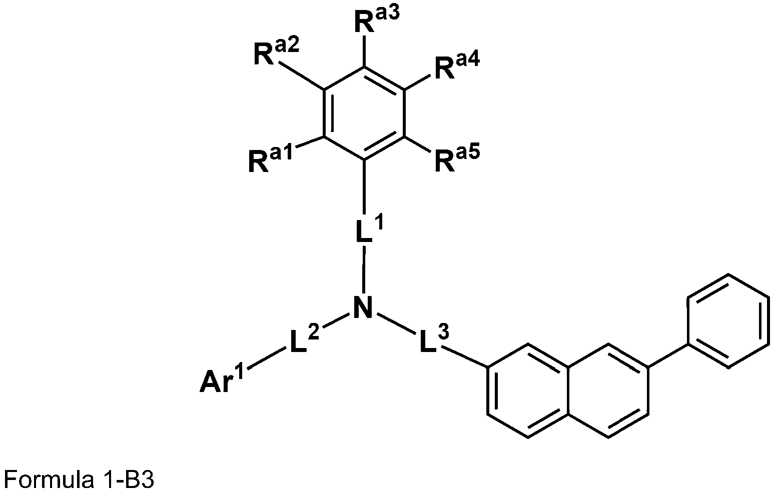 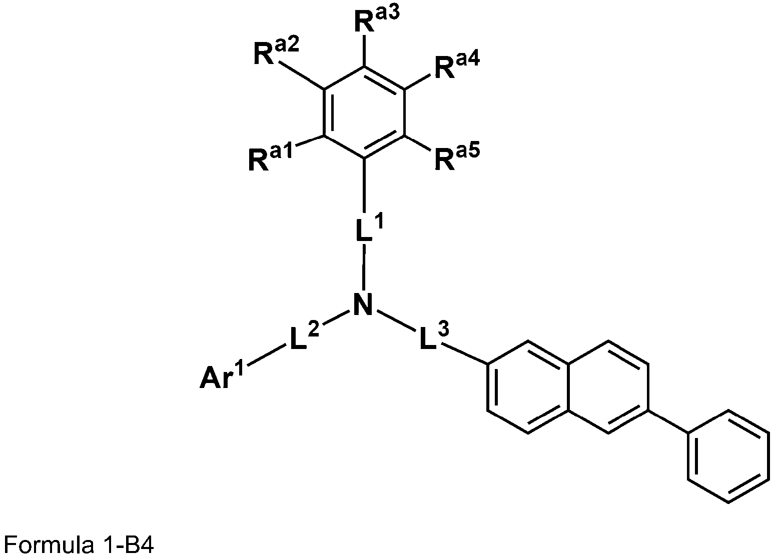 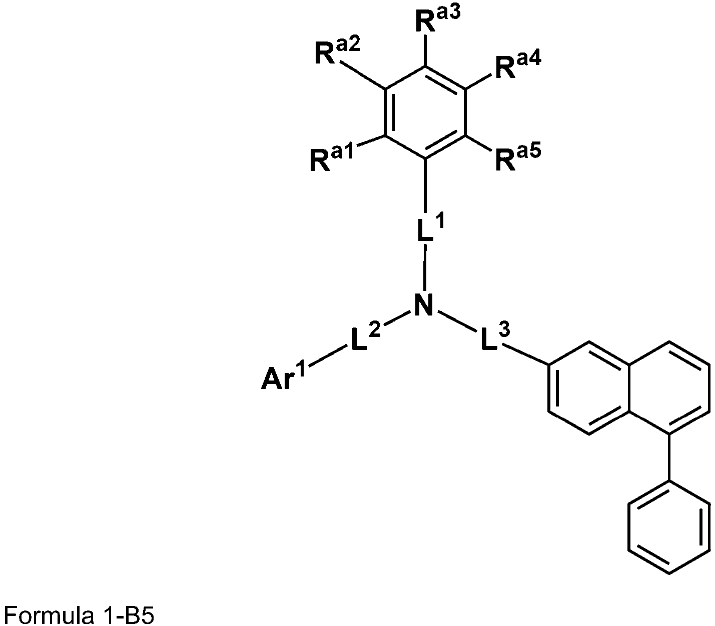 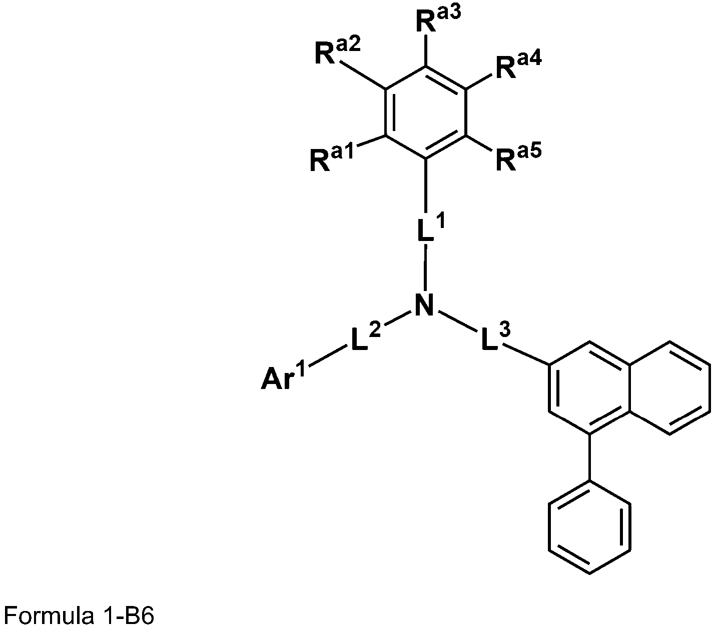 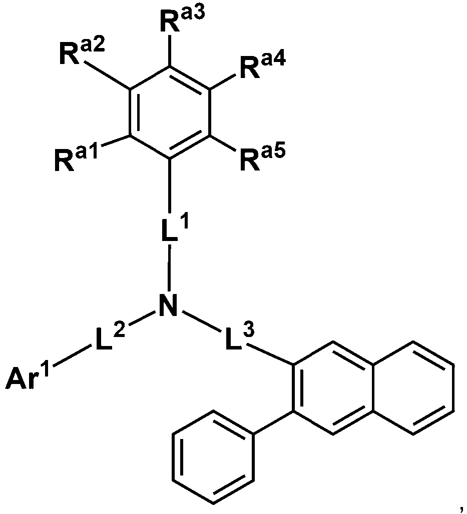 in Formula 1-B1 to Formula 1-B6,
L¹ to L³, Ar¹, R^{a1} to R^{a5}, and R^{c} being the same as defined in Formula 1.

5. The monoamine compound of any one of claims 1 to 4, wherein in Formula 1, Ar¹ is a substituted or unsubstituted naphthalene group, a substituted or unsubstituted phenanthrene group, a substituted or unsubstituted dibenzofuran group, a substituted or unsubstituted dibenzothiophene group, a substituted or unsubstituted benzonaphthofuran group, or a substituted or unsubstituted benzonaphthothiophene group.

6. The monoamine compound of any one of claims 1 to 4, wherein in Formula 1, Ar¹ is represented by any one selected from among AR-1 to AR-20: 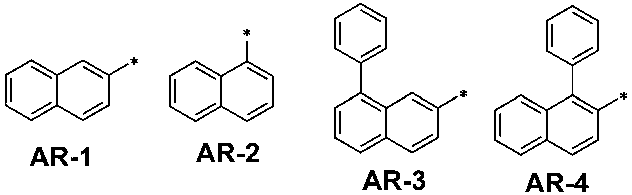 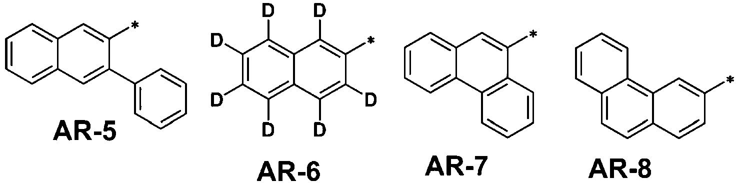 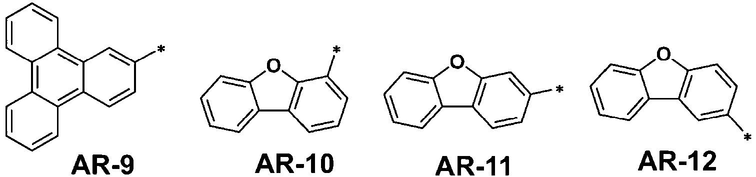 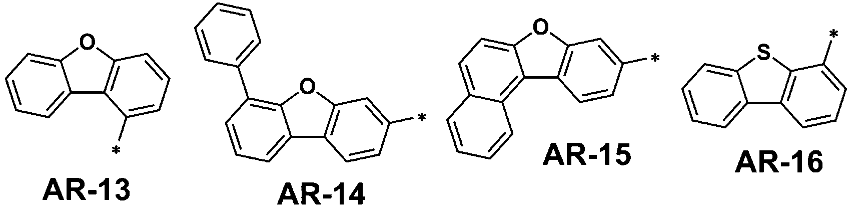 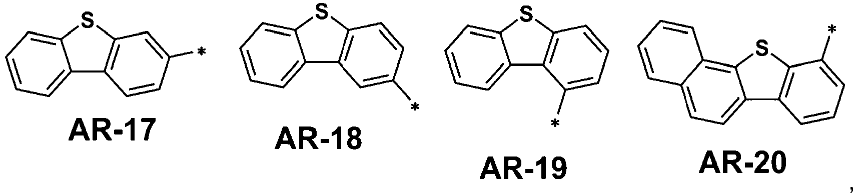 in AR-6, D being deuterium.

7. The monoamine compound of any one of claims 1 to 6, wherein in Formula 1, L¹ to L³ are each independently a direct linkage, or represented by L-1 or L-2:

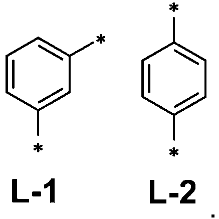

8. The monoamine compound of claim 1, wherein the monoamine compound represented by Formula 1 is any one selected from among compounds in Compound Group 1: 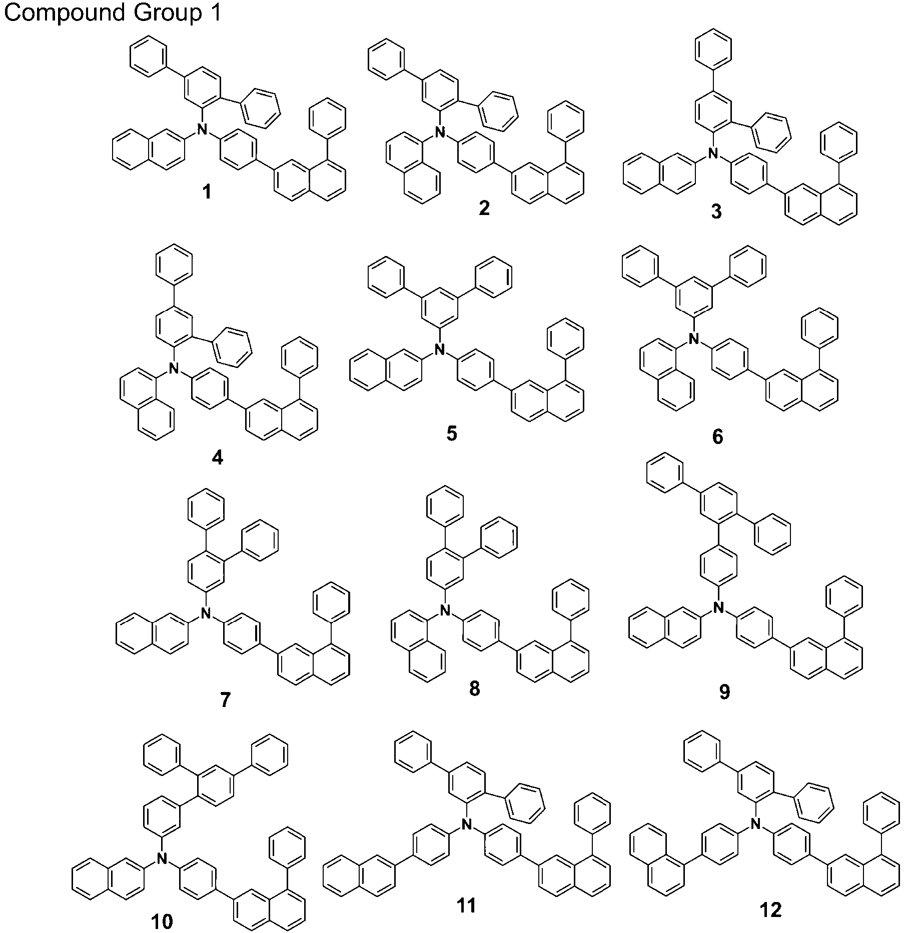 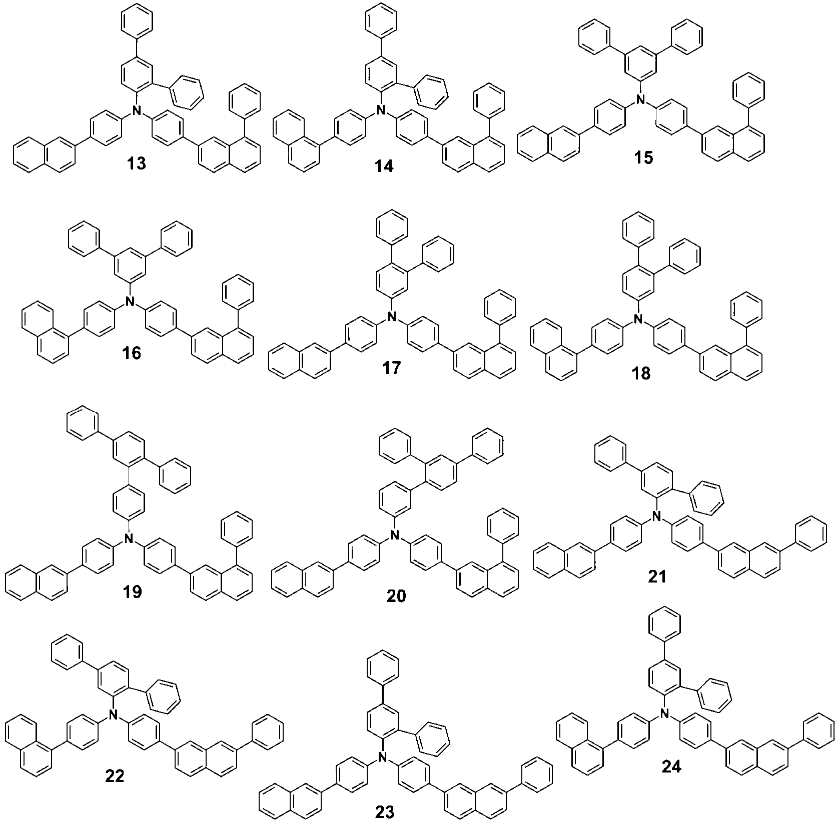 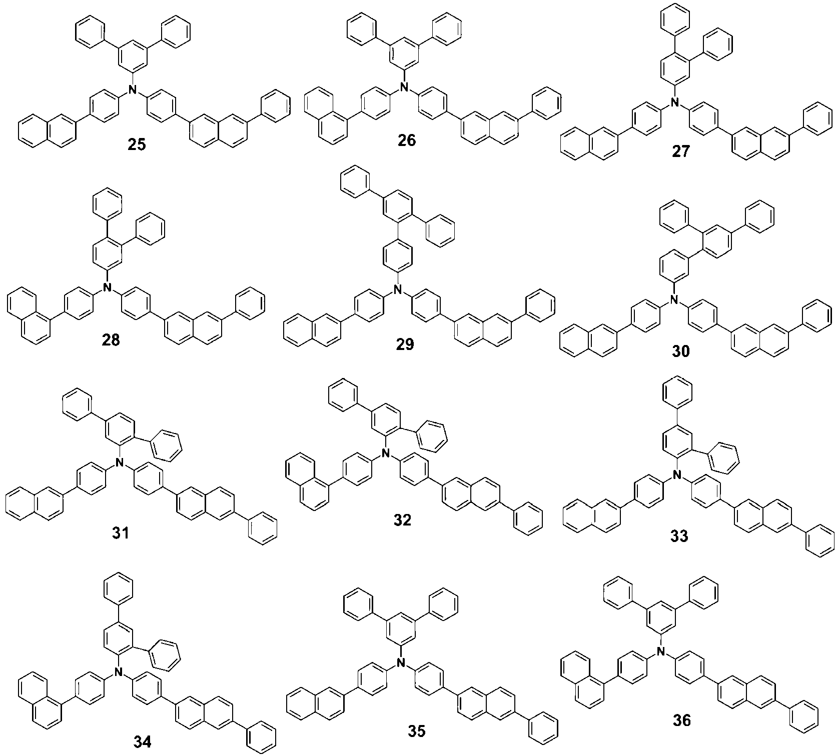 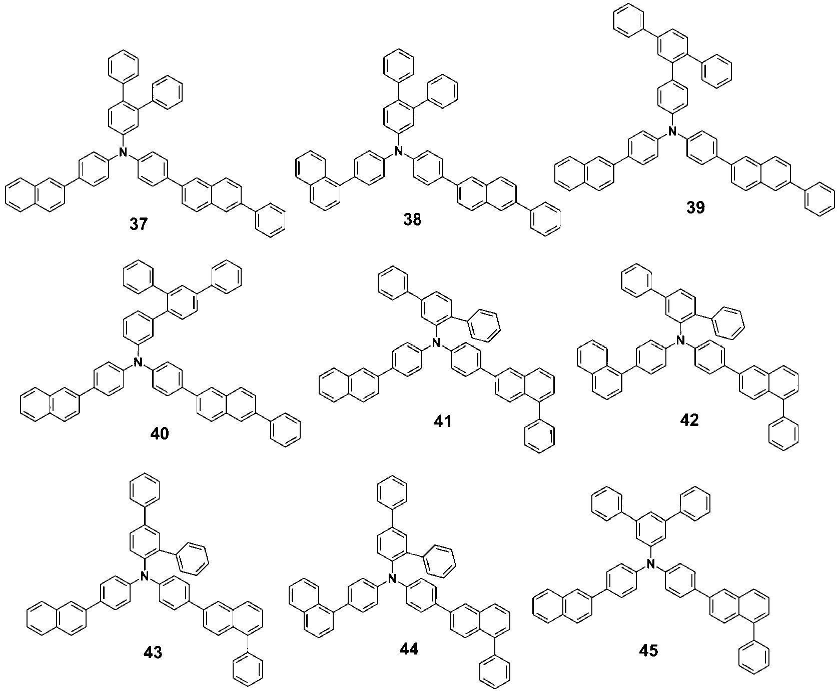 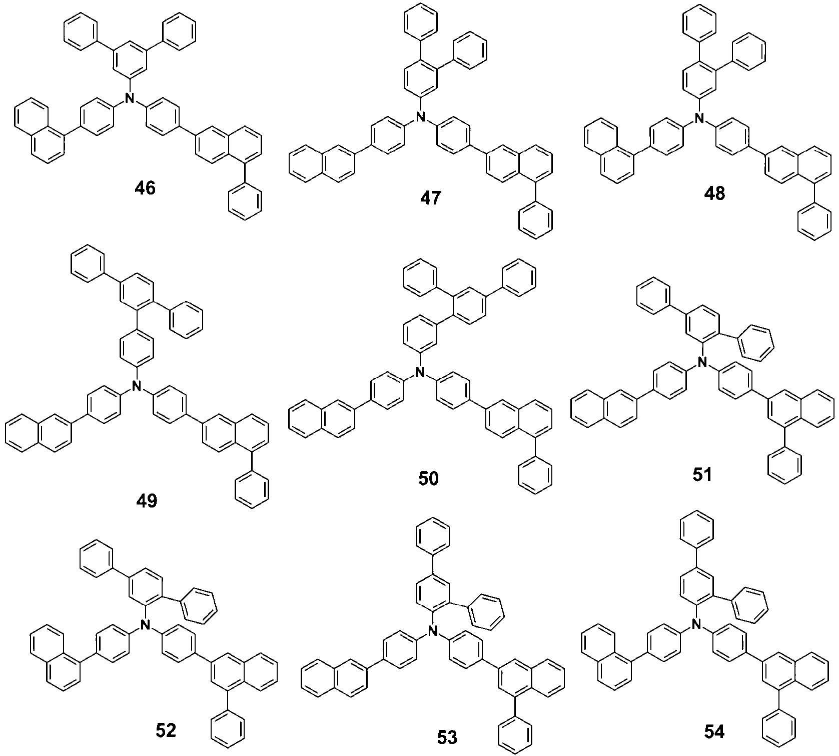 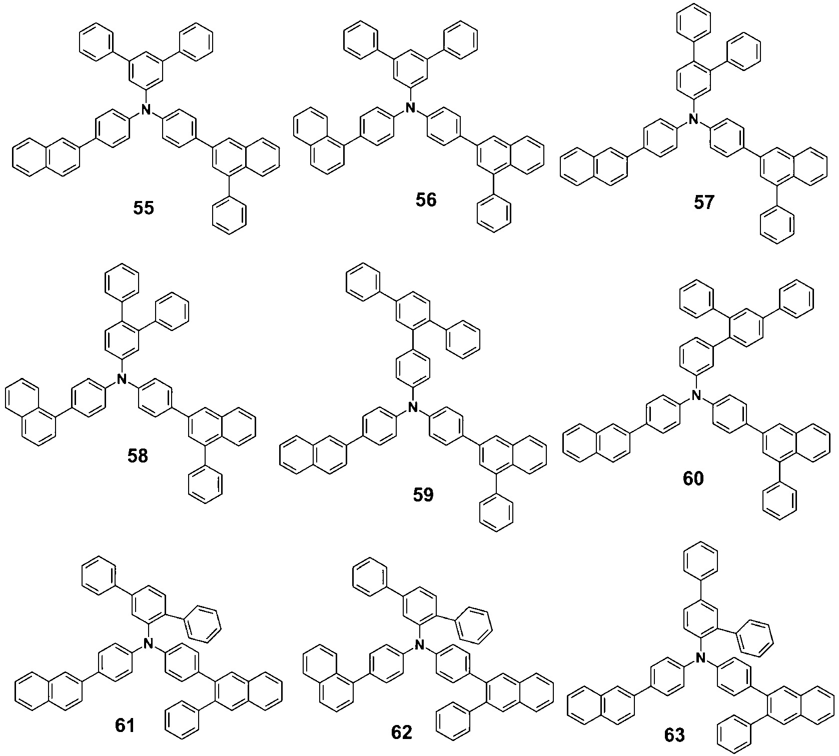 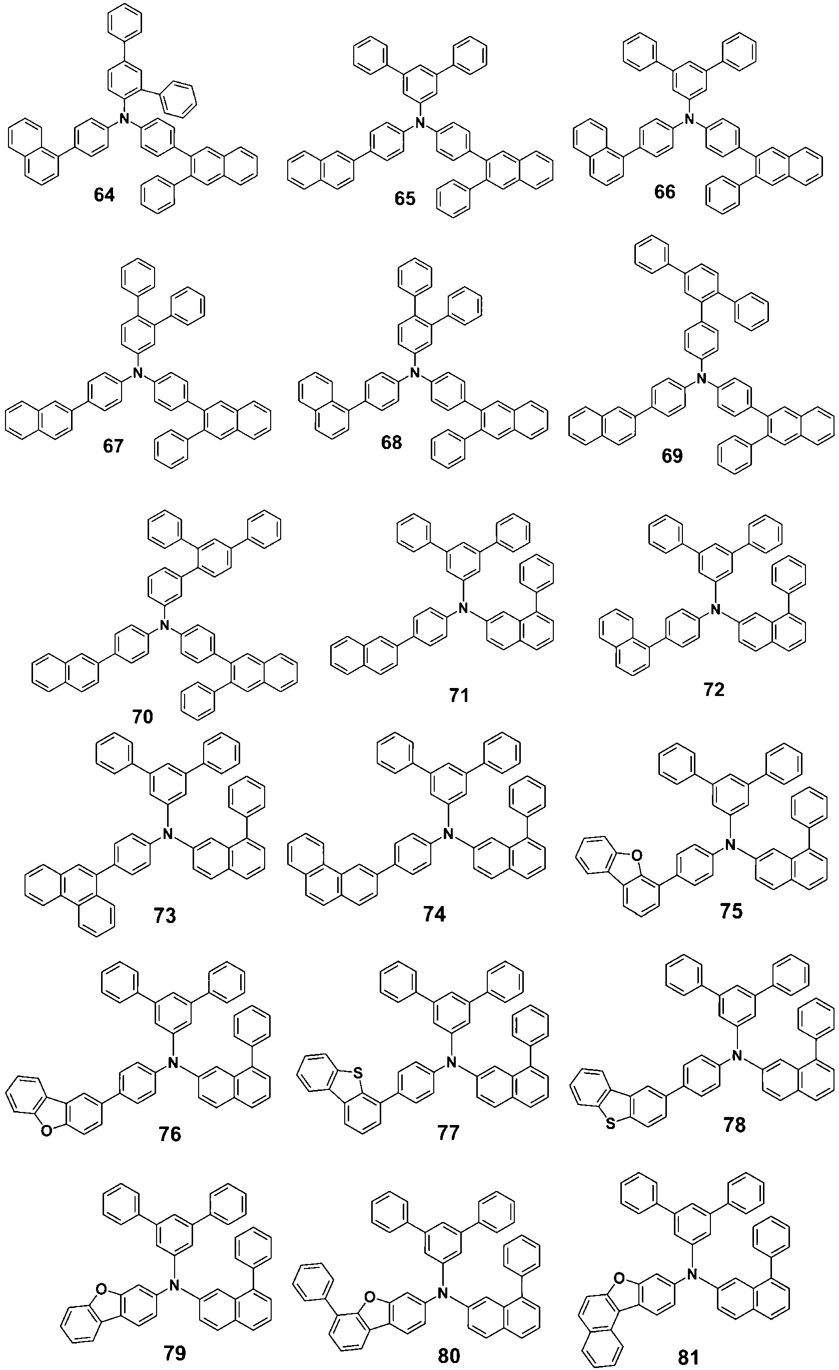 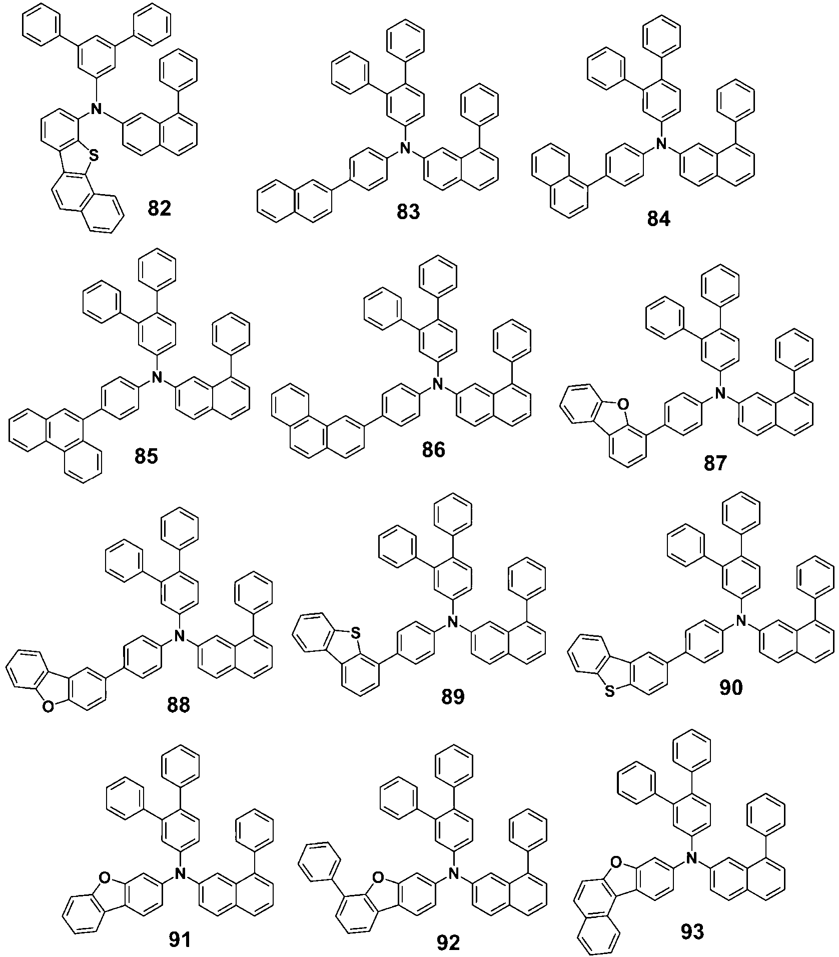 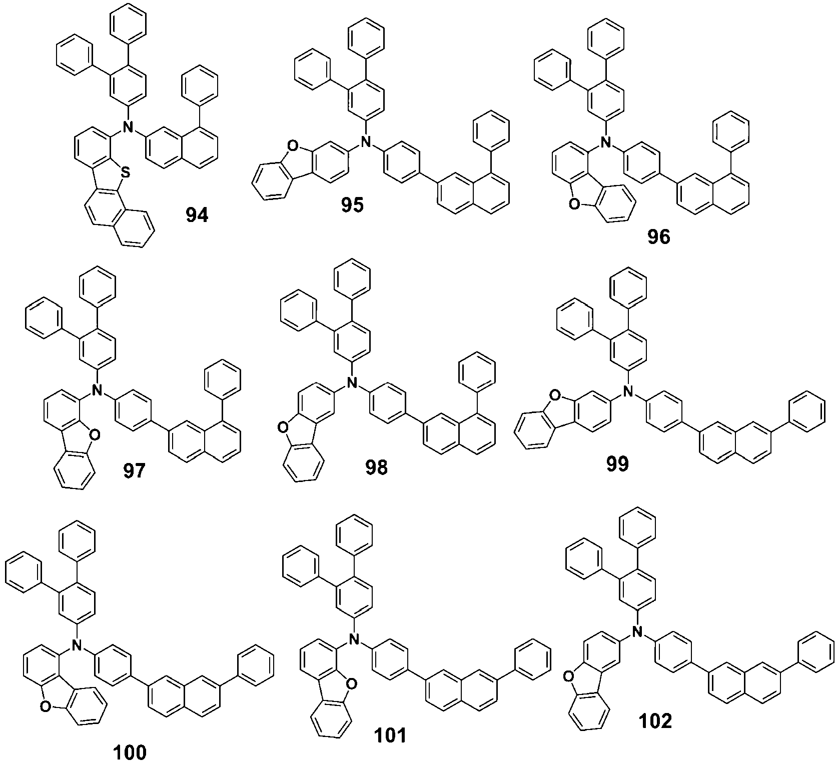 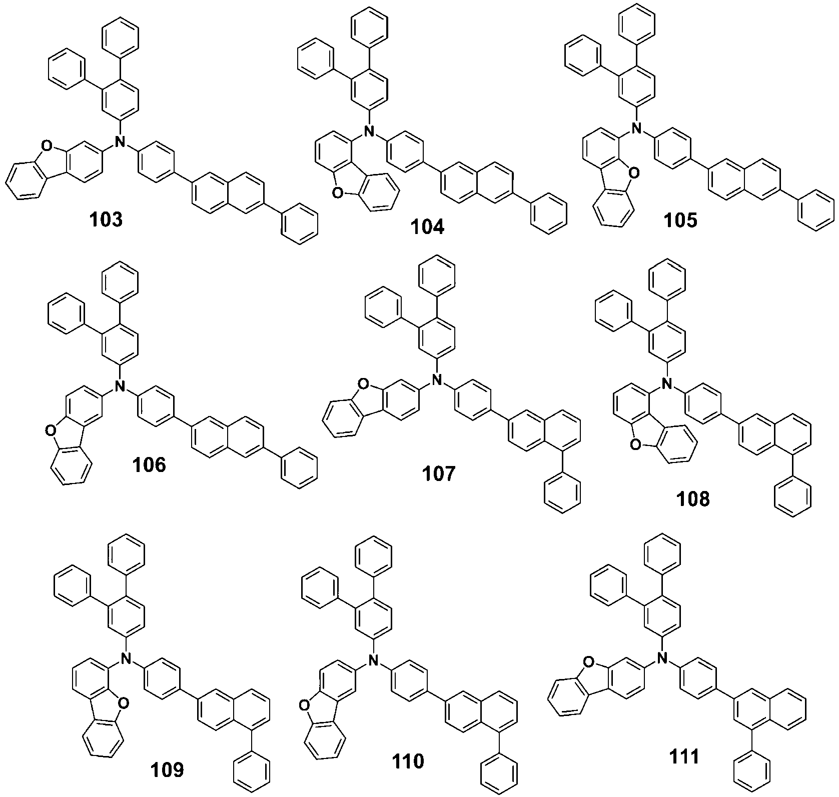 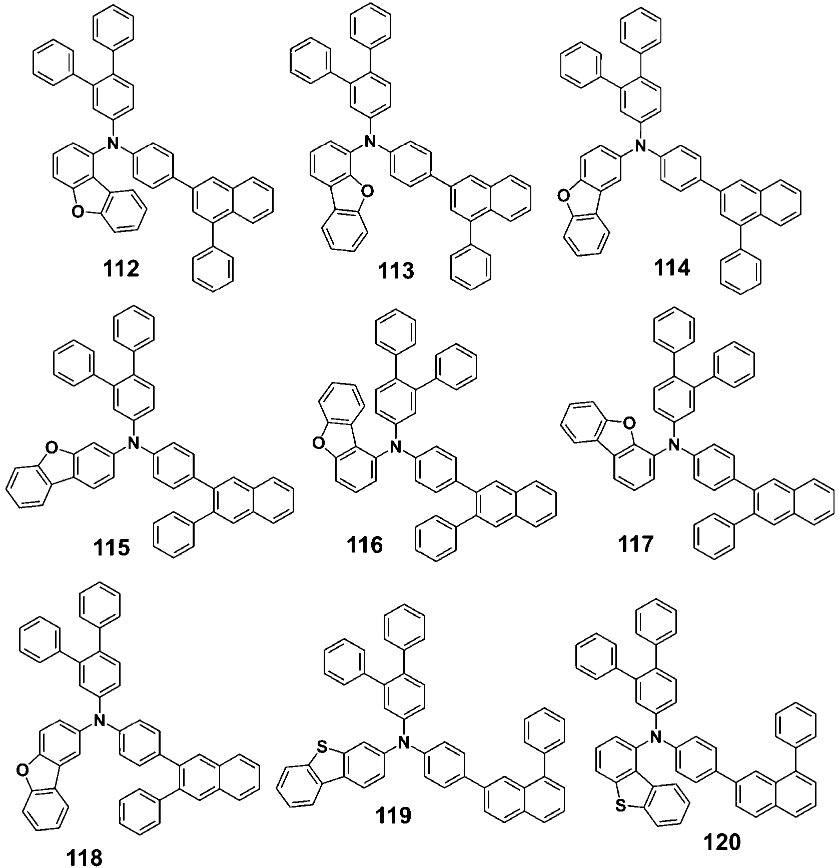 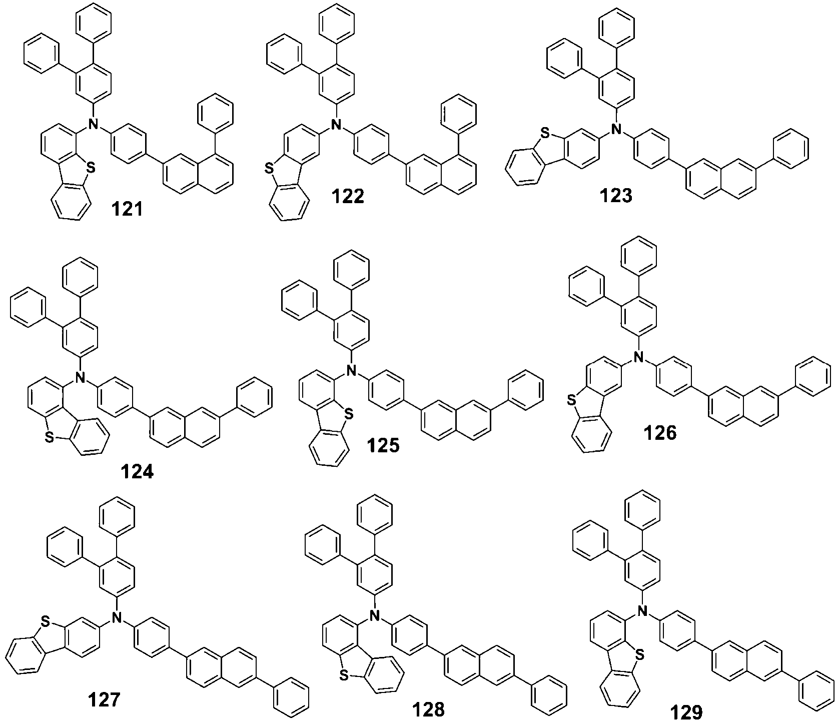 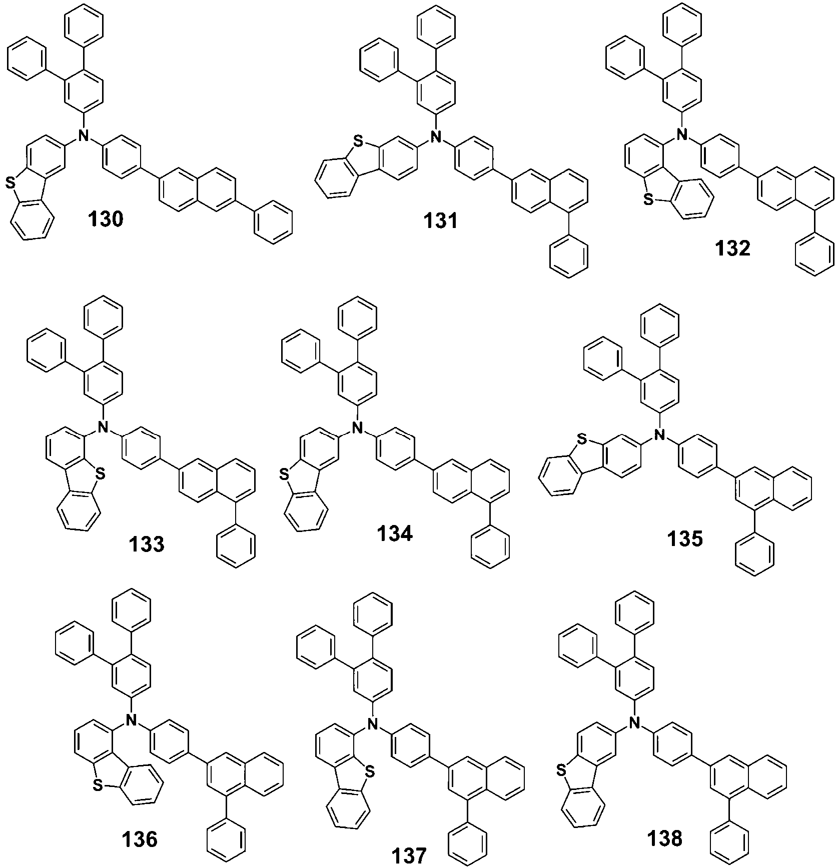 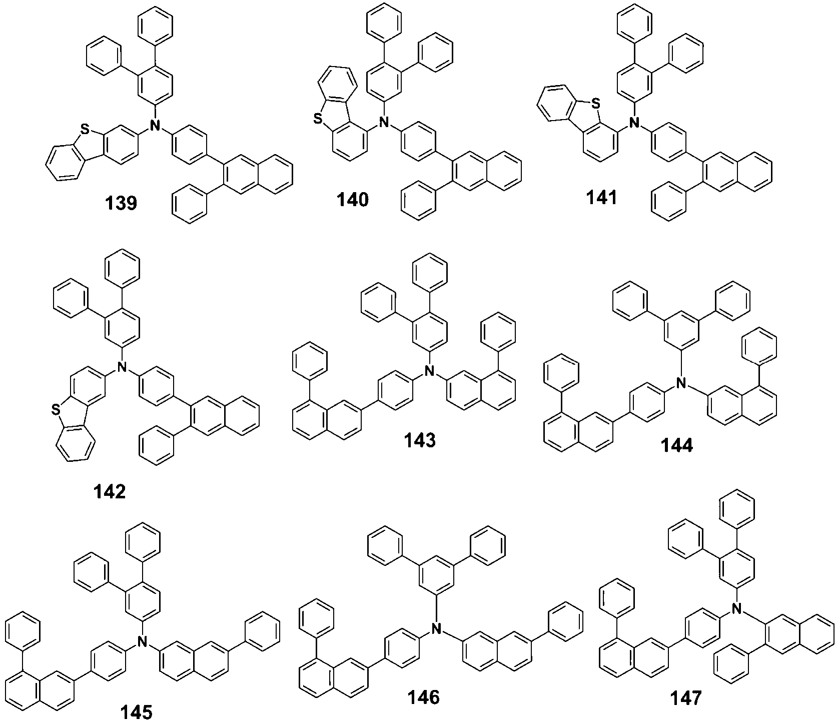 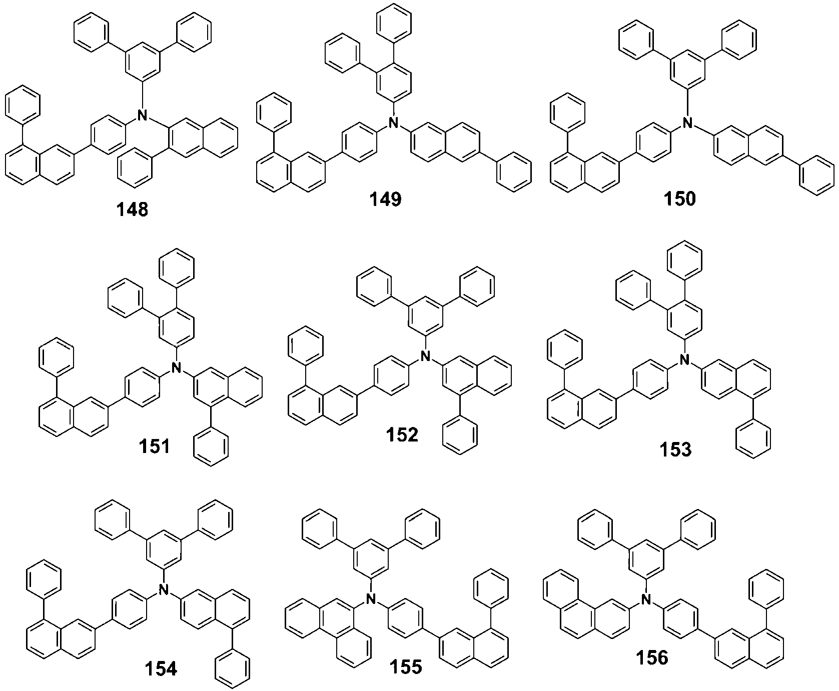 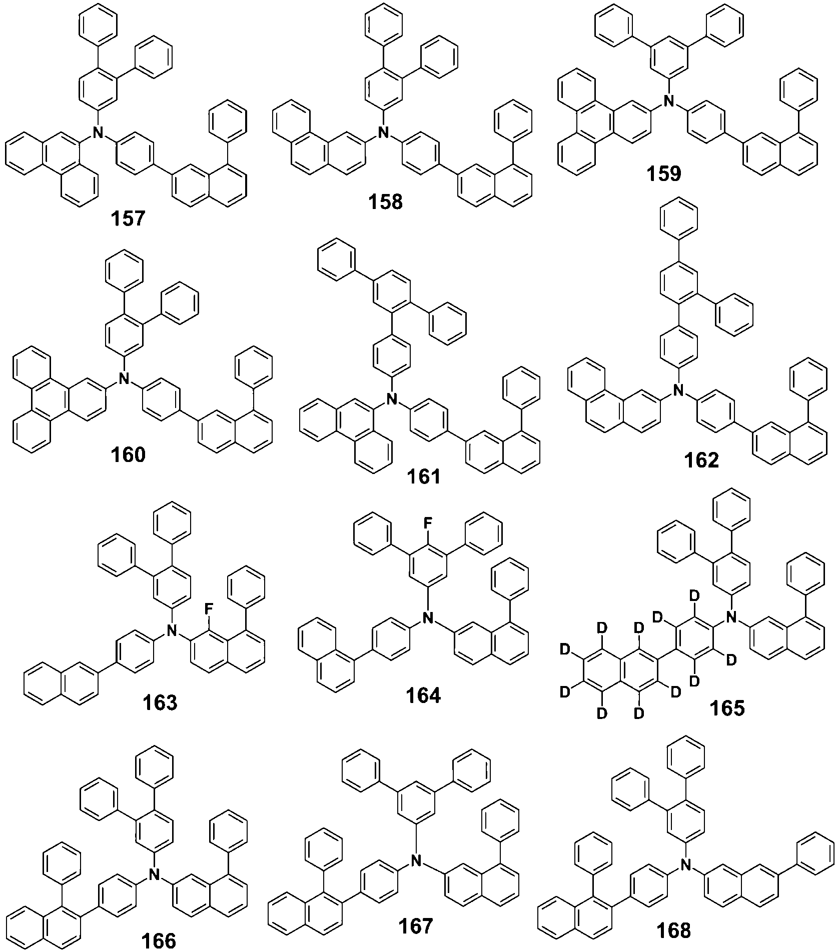 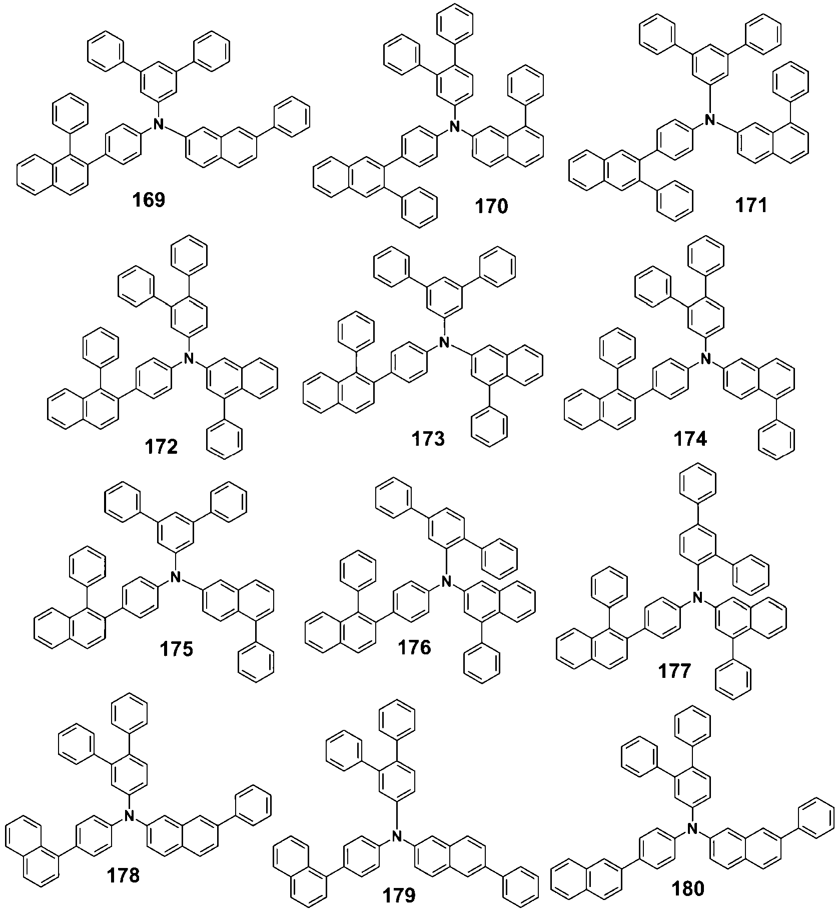 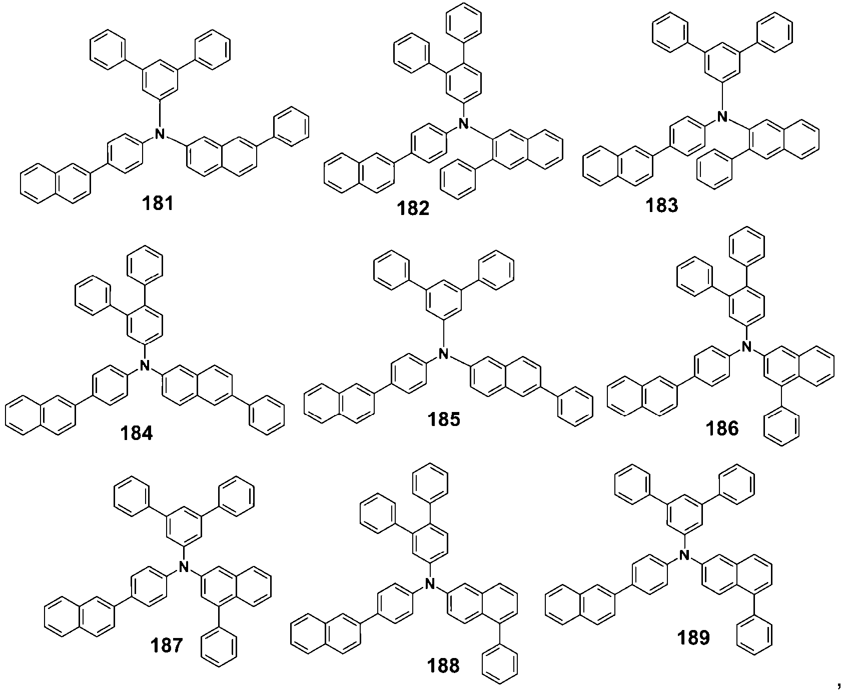 in Compound Group 1, D being deuterium.

9. A light-emitting element (ED) comprising:
a first electrode (EL1);
a hole transport region (HTR) on the first electrode (EL1) and comprising a monoamine compound according to any one of claims 1 to 8;
an emission layer (EML) on the hole transport region (HTR);
an electron transport region (ETR) on the emission layer (EML); and
a second electrode (EL2) on the electron transport region (ETR).

10. The light-emitting element of claim 9,
wherein
the hole transport region (HTR) comprises a hole injection layer (HIL), a hole transport layer (HTL) on the hole injection layer (HIL), and an electron blocking layer (EBL) on the hole transport layer (HTL), and
at least one of the hole injection layer (HIL), the hole transport layer (HTL), or the electron blocking layer (EBL) comprises the monoamine compound.

11. The light-emitting element of claim 9 or claim 10, wherein the emission layer comprises a compound represented by Formula E-1: 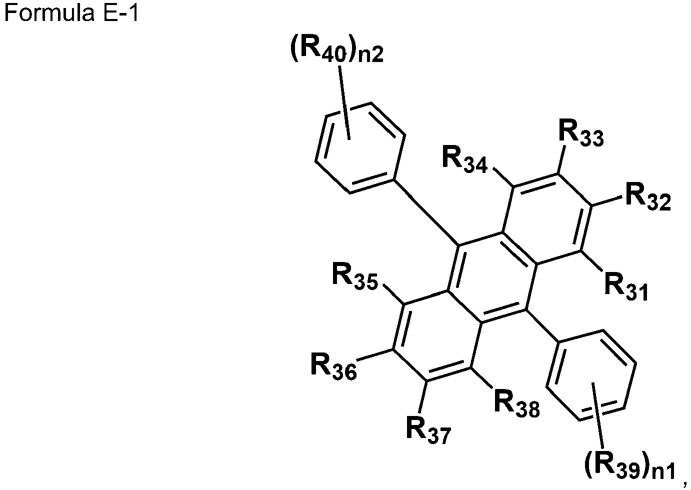 in Formula E-1,
n1 and n2 being each independently an integer of 0 to 5, and
R₃₁ to R₄₀ being each independently hydrogen, deuterium, a halogen, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms, and/or bonded to an adjacent group to form a ring.

12. An electronic apparatus comprising a display device,
the display device comprising:
a base layer;
a circuit layer on the base layer; and
a display element layer on the circuit layer and comprising a light-emitting element,
wherein the light-emitting element comprises:
a first electrode;
a hole transport region on the first electrode and comprising a monoamine compound according to any one of claims 1 to 8;
an emission layer on the hole transport region;
an electron transport region on the emission layer; and
a second electrode on the electron transport region.

13. The electronic apparatus of claim 12, wherein the display device further comprises at least one selected from among an light control layer and a color filter layer, where the light control layer comprises a quantum dot, and the color filter layer comprises a pigment and/or a dye.

14. The electronic apparatus of claim 12 or claim 13, wherein the electronic apparatus comprises any one selected from among a television, a monitor, an outdoor billboard, a personal computer, a laptop computer, a personal digital assistant, a vehicular display device, a game console, a portable electronic device, and a camera.
